(19) Europäisches Patentamt
European Patent Office
Office européen des brevets

(11) **EP 4 331 609 A1**

(12) **EUROPEAN PATENT APPLICATION**

(43) Date of publication:
**06.03.2024 Bulletin 2024/10**

(21) Application number: **22193399.7**

(22) Date of filing: **01.09.2022**

(51) International Patent Classification (IPC):
***A61K 47/59*** *(2017.01)* ***A61K 47/64*** *(2017.01)*
***A61K 47/68*** *(2017.01)* ***A61P 7/00*** *(2006.01)*
***A61P 35/00*** *(2006.01)*

(52) Cooperative Patent Classification (CPC):
**A61K 47/6849; A61K 47/595; A61K 47/6455; A61P 7/00; A61P 35/00; C12N 15/88;**
A61K 48/0041

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
- **Sapreme Technologies B.V.**
  **3721 MA Bilthoven (NL)**
- **Charité - Universitätsmedizin Berlin**
  **10117 Berlin (DE)**
- **Freie Universität Berlin**
  **14195 Berlin (DE)**
- **Universidade de Santiago de Compostela**
  **15782 Santiago de Compostela (ES)**

(72) Inventors:
- **POSTEL, Ruben**
  **3721 MA Bilthoven (NL)**
- **FUCHS, Hendrik**
  **10117 Berlin (DE)**
- **NAGEL, Gregor**
  **10117 Berlin (DE)**
- **WENG, Alexander**
  **14195 Berlin (DE)**
- **KOLSTER, Meike Karen**
  **14195 Berlin (DE)**
- **CORREA CHINEA, Juan Francisco**
  **15782 Santiago de Compostela (ES)**
- **FERNÁNDEZ MEGÍA, Eduardo Pedro**
  **15782 Santiago de Compostela (ES)**
- **LÓPEZ BLANCO, Roi**
  **15782 Santiago de Compostela (ES)**

(74) Representative: **Nederlandsch Octrooibureau**
**P.O. Box 29720**
**2502 LS The Hague (NL)**

Remarks:
The complete document including Reference Table(s) and the Sequence Listing(s) can be downloaded from the EPO website

# (54) METHODS AND COMPOSITIONS FOR ENHANCED POLYPLEX DELIVERY

(57) The invention lies in the field of delivery of nucleic acids into a cell. In particular, disclosed herein are compositions comprising at least two components being (i) a polyplex comprising a nucleic acid to be delivered into a cell; and (ii) a covalent conjugate of an endosomal-escape-enhancing saponin bound to a cell targeting ligand by a linker configured to release the saponin from the cell targeting ligand under conditions present in an endosome. Thanks to the combination of the at least two components (i) and (ii), the presented herein compositions and methods achieve enhanced cytosolic release of the nucleic acid specifically in the cells targeted by the cell targeting ligand bound to the saponin.

EP 4 331 609 A1

FIG. 8
A

A431 (EGFR high)
Relative cell viability (Untr. = 100%)
npSaporin (pM)
PAMAM G5 (PEG-N3)6.2
PAMAM G5 (PEG-N3)6.2
+ 4uM SO1861-EMCH
PAMAM G5 (PEG-N3)6.2
+ 73 nM Cetuximab-SO1861
(corresponding to 0.3 uM SO1861)

B

A2058 (EGFR negative)
Relative cell viability (Untr. = 100%)
npSaporin (pM)
PAMAM G5 (PEG-N3)6.2
PAMAM G5 (PEG-N3)6.2
+ 4uM SO1861-EMCH
PAMAM G5 (PEG-N3)6.2
+ 73 nM Cetuximab-SO1861
(corresponding to 0.3 uM SO1861)

FIG. 8
C

A431 (EGFR high)
Relative cell viability (Untr. = 100%)
pEGFP (pM)
PAMAM G5 (PEG-N3)6.2
PAMAM G5 (PEG-N3)6.2
+ 4uM SO1861-EMCH
PAMAM G5 (PEG-N3)6.2
+ 73 nM Cetuximab-SO1861
(corresponding to 0.3 uM SO1861)

D

A2058 (EGFR negative)
Relative cell viability (Untr. = 100%)
pEGFP (pM)
PAMAM G5 (PEG-N3)6.2
PAMAM G5 (PEG-N3)6.2
+ 4uM SO1861-EMCH
PAMAM G5 (PEG-N3)6.2
+ 73 nM Cetuximab-SO1861
(corresponding to 0.3 uM SO1861)

## Description

### FIELD

The invention lies in the field of delivery of nucleic acids into a cell. In particular, disclosed herein are compositions comprising at least two components being (i) a polyplex comprising a nucleic acid to be delivered into a cell; and (ii) a covalent conjugate of an endosomal-escape-enhancing saponin bound to a cell targeting ligand by a linker configured to release the saponin from the cell targeting ligand under conditions present in an endosome. Thanks to the combination of the at least two components (i) and (ii), the presented herein compositions and methods achieve enhanced cytosolic release of the nucleic acid specifically in the cells targeted by the cell targeting ligand bound to the saponin.

### BACKGROUND

Gene therapy is one of the most promising treatment options for future advanced therapies in a broad range of diseases. These include replacement of defective genes in inherited diseases (George et al., 2017), elimination of diseased cells such as cancer cells by suicide genes (Sama et al., 2018), gene repair or modification by CRISPR/Cas technology (Mali, et al. 2013), and gene control by micro ribonucleic acid (RNA) methodologies (Berkhout and Liu, 2014), for instance, to improve the body's response against acquired diseases. Currently, *in vivo* delivery of genes is predominantly accomplished via viral vectors (Lee et al., 2017), which still have major safety concerns. Importantly, their manufacturing process is complex and involves substantial production costs, especially on a large scale, which hinders their commercial implementation for the treatment of diseases with large patients' cohorts. Furthermore, viral gene carriers tend to induce adverse immune responses to viral structural proteins (Kwoh et al., 1999). Consequently, alternatives to viral delivery methods for gene therapeutics are intensively being sought for, but the existing approaches all face a major bottleneck caused by poor cytosolic and nucleosolic uptake rates of nucleic acids when delivered to or even into the cell (He et al., 2013).

Nucleic acids (NAs) are polymers of nucleotides - each nucleotide comprising a pentose sugar, a phosphate group and either purine or pyrimidine. The term "transfection" is generally understood as referring to the delivery into cells of an organism of a nucleic acid (Agarwal et al., 2012), for example ribonucleic acid (RNA) or deoxyribonucleic acid (DNA), or modified and/or synthetic equivalents thereof, typically carrying a "genetic information" such as a gene or a part thereof (e.g. an exon or a number thereof). To be able to carry such genetic information, gene therapeutics will typically involve relatively large genetic constructs encompassing more than thousand, frequently several thousands of consecutive nucleotides ("kilobases") or nucleotide pairs ("kbp" i.e. kilo base pairs). Naturally, transfection of such large and, furthermore, charged macromolecular compounds over the cellular membrane remains challenging.

To enable cellular delivery of large nucleic acids, typically, stable synthetic carriers with high-affinity to NAs are employed, such as cationic polymers as well as cationic lipids (He et al., 2013). Such cationic polymers have the ability to spontaneously form through the electrostatic condensation interpolyelectrolyte high-density complexes with NAs, termed polyplexes (Hess et al., 2017; Kabanov and Kabanov, 1995; Rumschöttel et al., 2016). As an illustration, polyplexes can be formed when DNA is mixed with a cationic polymer like poly(lysine) (PLL or polyK) or poly-D-lysine (PDL) (Wu and Wu, 1987; Vasiliu et al., 2017, Kwoh et al., 1999, Clifford et al., 2019), or another very commonly used poly(ethylenimine) (PEI) (Démoulins et al., 2016). These and many other cationic polymers are known to easily form polyplexes with NAs, notably including, to name a few more, polyamidoamine dendrimers (PAMAMs, Koping-Hoggard et al., 2004), chitosan (Koping-Hoggard et al., 2004, Puras et al., 2013), 2-dimethyl(aminoethyl) methacrylate (pDMAEM), and other natural or synthetic DNA-binding proteins or polypeptides (Tros de Ilarduya et al., 2010). Depending on the desired property or purpose for a given polyplex, like route of administration or half-life in plasma, there exist many different chemical modifications that have been tried and applied to various cationic polyplex-forming polymers. For example, instances of modified PLL particles have been reported for enhanced DNA delivery by e.g. addition of PEG groups (Kozielski et al., 2016, Ziady et al., 2003). Regardless of their chemical modification status, and because of their ability to condense and stabilise NA payloads in the form of a high-density polyplex, the cationic polymer carriers are frequently referred to as "scaffolds" or, simply, polyplexing agents.

The condensed nature of polyplexes leads to their easier cell internalization and enhanced protection from enzymatic degradation (Hess et al., 2017). Several mechanisms through which polyplexes interact with cells have been suggested. For example, it has been postulated that they may interact with the cell surface via electrostatic and non-specific interactions (Tros de Ilarduya et al., 2010). Despite the above, the therapeutic efficiency of polyplexes *in vivo* remains unsatisfactorily poor compared to viral vectors. Without wishing to be bound to any theory, it is possible that like with other non-viral approaches, this effect could potentially be related not to the inability of a polyplex to enter the cell *per se* but possibly to an insufficient endosomal escape after the engulfment by the cell.

Indeed, it has been estimated that the inability to escape the endosomal/lysosomal compartment within the cell remains an insurmountable hurdle against successful implementation of gene therapeutics in clinical settings. This

perhaps is best reflected by the fact that more than 98% of genetic material that enters the cell was estimated to be transported and degraded in the lysosomes (Gilleron et al., 2013). Due to this effect, only limited and typically insufficient amounts of nucleic acid-based therapeutics manage to successfully enter the cytosol.

In line with the above, polyplexes were suggested to be transported into cells via endocytosis or endocytosis-like mechanisms and, once inside, when endosome pH decreases from pH 7 to 5.5, it was hypothesised that a part of the polyplex-bound nucleic acid dissociates from the polyplex and egresses into the cytosol (Tros de Ilarduya et al., 2010). For example, poly-lysine (poly-K) scaffolds were hypothesised to possess a very weak transfection efficiency because of their inefficient release from the endosomes (Zhang et al., 2010). Furthermore, as poly-K scaffolds do not possess a hydrophobic domain, they are inherently incapable of fusing with or destabilizing the endosome (Tros de Ilarduya et al., 2010).

In addition to the challenges related to insufficient cytosolic and nucleosolic uptake of gene therapeutics, in particular those of substantial sizes above one or more kilobases, successful gene therapy *in vivo* further frequently requires targeted delivery into specific tissue in which the gene therapy is supposed to take effect. These and other challenges are hereby addressed, as explained in the continuation.

## SUMMARY

In general, to address the drawbacks of the prior art, presented herein are novel pharmaceutical compositions comprising:

(i) a polyplex comprising at least one nucleic acid and a polymeric scaffold; and

(ii) a targeted saponin conjugate comprising a saponin and a first targeting ligand recognised by a first endocytic receptor, wherein the saponin is covalently bound to the first targeting ligand by a linker adapted to cleave and release the saponin from the first targeting ligand under conditions present in an endosome or a lysosome; wherein the saponin is a triterpenoid 12,13-dehydrooleanane-type saponin that in an unreacted state comprises an aldehyde group at position C-23 of the saponin's aglycone core structure.

Without wishing to be bound by any theory, the inventors hypothesised that specific targeting of the 12,13-dehydrooleanane type triterpenoid saponin will stimulate efficient exiting of the therapeutic nucleic acid only from polyplexes that entered the endosomes of the ligand-targeted cells (in a very much desired for therapeutics but not fully understood phenomenon termed *endosomal escape).*

In other words, disclosed herein are compositions comprising at least two components being (i) a polyplex comprising a nucleic acid to be delivered into a cell; and (ii) a covalent conjugate of an endosomal escape enhancing (EEE) saponin bound to a cell targeting ligand by a linker configured to release the saponin from the cell targeting ligand under conditions present in an endosome only in the target cell population, thus limiting the availability of the gene therapeutic to this particular cell population.

Thanks to the combination of the at least two components (i) and (ii), the presented herein compositions and methods are configured to achieve enhanced cytosolic release of the therapeutic nucleic acid for gene therapy, specifically in the cells targeted by the cell targeting ligand bound to the EEE saponin, while at the same time avoiding potential issues related to size-exclusion or competition between target-specific and non-specific mechanisms via which the target cell absorbs both components of the presented herein compositions.

Thanks to e.g. acid sensitive linker, it is hypothesised that the EEE saponin will only be released in the endosomes of the targeted cells. This ensures that the EEE saponin will only lead to enhanced cytosolic release of the gene-carrying therapeutic specifically in the cells wherein the non-specifically internalized polyplex and the EEE saponin coincided.

It is hypothesised that, thanks to the intracellular dynamics of the endosomal/lysosomal compartments, which undergo constant fusion and budding processes, the chances of the components (i) and (ii) will be sufficient to coincide within said compartment of the ligand-targeted cells, even if both components (i) and (ii) are internalised by the same cell via different mechanisms, into initially separate vesicles formed at distinct zones of the cell's membrane. Such configuration has the advantages that the competition between components (i) and (ii) for the cellular entry points is minimised, while at the same time ensuring cell-specific action of the therapeutic versus non-targeted tissue types to which the targeted saponin conjugate did not bind.

The presented herein co-administration experiments with the components (i) and (ii) confirm the above assumptions, showing marked increase in receptor-targeted selectivity of the treatment. Furthermore, they also indicate that, in addition to this selectivity, the targeted saponin conjugates have the advantage of being less toxic to cells than free non-targeted saponin, such as Sapofectosid that needs to be used at about 10-fold higher amounts as compared to its targeted form to achieve similar effects on transfection efficiency.

This particular advantage demonstrates that co-administration of polyplexes with targeted saponin conjugates

has a promising potential for conducting gene therapy not only ex *vivo* but notably also *in vivo* using systemic administration. The latter is further supported by promising stability results and low haemolytic activity of the disclosed herein polyplexes and polymeric scaffold as investigated in human blood.

In sum, the disclosed herein compositions, methods, uses, and novel polymeric scaffolds suitable to be used in any of these compositions, methods, or uses, address the need of solving a longstanding problem of cytoplasmic and nucleic delivery of gene therapeutics, in particular in vivo and by minimizing the treatment risks through circumventing viral vector-mediated gene therapy.

The disclosed herein compositions, methods, uses, and novel polymeric scaffolds address this need by exhibiting the highly desired property of enhancing delivery of nucleic acids polyplexes specifically into target cells, and thus have the potential of increasing the efficacy of targeted gene therapeutic treatment in patients.

Furthermore, they are compatible with personalized gene therapies, e.g. by advanced CRISPR/Cas technology and thanks to the simplicity of the 2-component design, they have the potential of being easily produced and thus of reducing the costs of gene therapy for making it available to a broad patient base.

The innovative concepts presented herein will be described with respect to particular embodiments that should be regarded as descriptive and not limiting beyond of what is described in the claims. These embodiments as described herein can operate in combination and cooperation, unless specified otherwise.

It is one of several objectives of embodiments of present disclosure to provide a solution to the problem of insufficient delivery of nucleic acids into appropriate compartments of their target cells and of the related therewith non-specificity encountered when administering nucleic acid-based therapeutics to human patients.

It is a further one of several objectives of the embodiments to provide a solution to the problem of insufficient safety characteristics of current nucleic-acid-based drugs, when administered to human patients in need thereof, in particular at side-effect inducing excessive doses.

It is yet a further one of several objectives of embodiments of the current invention to provide a solution to the problem of current nucleic acid-based therapies being less efficacious than desired, when administered to human patients in need thereof, due to not being sufficiently capable to reach and/or enter into to the diseased target cells with little to no off-target activity on non-diseased cells, when administered to human patients in need thereof.

At least one of the above objectives is achieved by providing any of the composition, method, polymeric scaffold, or use as described in continuation. In particularly advantageous instances, different particular embodiments are provided, comprising advantageous components such as the ones selected from preferred sub-types of endosomal-escape-enhancing saponins, different therapeutic nucleic acids, and advantageous ligands or combinations thereof for targeting said saponins and nucleic acids to cells of interest, as well as covalent linkers for connecting the ligands with the saponins and nucleic acids and configured for being cleavable under conditions present in human endosomes and or lysosomes.

These and other aspects of the disclosure are presented in detail in continuation.

## DEFINITIONS

As used herein, the term "polyplex" has its regular established in the art meaning and refers to a complex between a nucleic acid and cationic, likely polymeric, carrier having a sufficient degree of affinity to the nucleic acid and capable of binding to said nucleic acid primarily by electrostatic interactions. Typical examples of such cationic carriers include e.g. cationic polymers.

As used herein, such cationic carrier capable of forming a polyplex with a nucleic acid will further be termed as a "polymeric scaffold". Examples of such polymeric scaffolds include, but are not limited to compounds comprising or consisting of cationic polymers such as polyamidoamine dendrimer, further referred to as PAMAM, or polypeptides with a net cationic charge, for example comprising sufficient number of cationic amino acids such as lysine or e.g. arginine. An example of a polypeptide with a net cationic charge is a peptide comprising or consisting of a strip of lysines, which can be referred to as poly-lysine, PLL, poly-K, polyK, poly-Lys etc. Other examples of polymeric scaffolds include but are not limited to poly-D-lysine (PDL), poly(ethylenimine)(PEI), chitosan, 2-dimethyl(aminoethyl) methacrylate (pDMAEM), and any synthetic and/or modified forms thereof, such as any of these and other polymeric scaffolds conjugated with chemical modifications, including but not limited to polyethylene glycol (PEG), i.e. pegylated (also spelled as PEGylated).

As used herein, typical polyplexes may comprise smaller nucleic acids (e.g. siRNA, microRNA, or generally understood oligonucleotides) but preferentially will comprise much larger nucleic acids (e.g., lager than 150 bp, preferably larger than 0.5 kb, more preferably larger than 1 kb, most preferably larger than 2 kb, e.g. 100 times larger than a typical 20-25 bp siRNA). While the sizes of the present polyplexes are not invariant, preferably they will fall within the range of 50-250 nm, more preferably within the range of 100-200 nm.

As used herein, the terms "nucleic acid" and "polynucleotide" are synonymous to one another and are to be construed as encompassing any polymeric molecule made of units, wherein a unit comprises at least a nucleobase (or simply "base" e.g. being a canonical nucleobase like adenine (A), cytosine (C), guanine (G), thymine (T), or uracil (U),

or any known non-canonical, modified, or synthetic nucleobase like 5-methylcytosine, 5-hydroxymethylcytosine, xanthine, hypoxanthine, 7-methylguanine; 5,6-dihydrouracil etc.) or a functional equivalent thereof, which renders said polymeric molecule capable of engaging in hydrogen bond-based nucleobase pairing (such as Watson-Crick base pairing) under appropriate hybridisation conditions with naturally-occurring nucleic acids such as deoxyribonucleic acid (DNA) or ribonucleic acid (RNA), which naturally-occurring nucleic acids are to be understood being polymeric molecules made of units being nucleotides, whereby each nucleotide consists of a pentose sugar, a phosphate group and one of the nucleobases.

Hence, from a chemistry perspective, the term nucleic acid under the present definition can be construed as encompassing polymeric molecules that chemically are DNA or RNA, as well as polymeric molecules that are nucleic acid analogues, also known as xeno nucleic acids (XNA) or artificial nucleic acids, which are polymeric molecules wherein one or more (or all) of the units are modified nucleotides or are functional equivalents of nucleotides. Nucleic acid analogues are well known in the art and due to various properties, such as improved specificity and/or affinity, higher binding strength to their target and/or increased stability in vivo, they are extensively used in research and medicine. Typical examples of nucleic acid analogues include but are not limited to locked nucleic acid (LNA) (that is also known as bridged nucleic acid (BNA)), phosphorodiamidate morpholino oligomer (PMO also known as Morpholino), peptide nucleic acid (PNA), glycol nucleic acid (GNA), threose nucleic acid (TNA), hexitol nucleic acid (HNA), 2'-deoxy-2'-fluoroarabinonucleic acid (FANA or FNA), 2'-deoxy-2'-fluororibonucleic acid (2'-F RNA or FRNA); altritol nucleic acids (ANA), cyclohexene nucleic acids (CeNA) etc.

In line with the above, in some instance, the nucleic acid of the present disclosure may be modified. For example, the nucleic acid may be modified on its backbone. Examples of modifications that can be performed on the backbone of a nucleic acid include, but are not limited to, phosphorothioate (PS), boranophosphate, phosphonoacatate (PACE), morpholine, peptide nucleic acid backbone modification (PNA), and amid-linked bases. The nucleic acid may also be modified on the sugar moiety and/or on the base moiety. Examples of modifications that can be performed on the sugar and/or the base moieties include, but are not limited to, locked nucleic acid (LNA), phosphoramidate (NP), 2'F-RNA, 2'-0 methoxyethyl (2'MOE), 2'O-methyl (2'OMe), 2'-O-fluoro (2'-F) 5-bromouracil, 5-iodouracil, 5-methylcytosine, ethylene bridged nucleic acids (ENA), diaminopurine, 2-thiouracil, 4-thiouracil, pseudouracil, hypoxantine, 2-aminoadenine, 6-methyl or other alkyl derivates of adenine and guanine, 2-propyl and other derivative of adenine and guanine, 6-azo-uracil, 8-halo, 8-amino, 8-thiol, 8-hydroxyk and other 8-substituted adenines and guanines, constrained ethyl sugar moiety (cET), ribofuranosyl, 2'-0,4'-C-methylene and 2'-0,4'-C-ethylene bicyclic nucleotide analogues, acyclic nucleotides (UNA and PNA), and dihydrouridine modification. Other modifications that may be performed on nucleic acids are, but are not limited to, modifications that include deoxyribonucleotide bases incorporated in a ribonucleotide sequence. The incorporations may be limited to the overhang structure in the canonical siRNA architecture or may be distributed in the sequence. Modifications to RNA molecules include, but are not limited to blunt-ended siRNA, 25-27mer siRNA, single strand siRNA, short hairpin siRNA, dumbbell siRNA, asymetric siRNA, short interspaced siRNA, hybrid between siRNA and antisense oligonucleotides (ASO). Other analogue nucleic acids may be contemplated include those with non-ribose backbones. In addition, mixtures of naturally occurring nucleic acids, analogues, and both may be made. Nucleic acids include but are not limited to DNA, RNA and hybrids where the nucleic acid contains any combination of deoxyribo- and ribo-nucleotides, and any combination of bases, including uracil, adenine, thymine, cytosine, guanine, inosine, xathanine hypoxathanine, isocytosine, isoguanine, 5-methylcytidine, pseudouridine etc. Modified 5' cap structures such as 3'-O-Me-m7G(5')ppp(5')G (anti-reverse cap analogue), may also be used for increased translation of mRNA. Nucleic acids include DNA in any form, RNA in any form, including triplex, duplex or single-stranded, antisense, siRNA, ribozymes, deoxyribozymes, polynucleotides, oligonucleotides, chimeras, and derivatives thereof.

In accordance with the cannon, length of a nucleic acid is expressed herein as the number of units from which a single strand of a nucleic acid is build. Because each unit corresponds to exactly one nucleobase capable of engaging in one base pairing event, the length is frequently expressed in so called "base pairs" or "bp" regardless of whether the nucleic acid in question is a single stranded (ss) or double stranded (ds) nucleic acid. In naturally-occurring nucleic acids, the length expressed in bp usually corresponds to the length expressed in nucleotides (nt).. For example, a single stranded nucleic acid made of 1000 nucleotides (or a double stranded nucleic acid made of two complementary strands each of which is made of 1000 nucleotides) can also be described as having a length of 1000 base pairs or 1000 bp, which length can also be expressed as 1000 nt or as 1 kilobase that is abbreviated to 1 kb. 2 kilobases or 2 kb are equal to the length of 2000 base pair which equates 2000 nucleotides of a single stranded RNA or DNA. To avoid confusion however, in view of the fact the nucleic acids as defined herein may comprise or consist of units not only chemically being nucleotides but also being functional equivalents thereof, the length of nucleic acids will preferentially be expressed herein in "bp" or "kb" rather than in the equally common in the art denotation "nt".

In certain embodiments, the nucleic acid can be an oligonucleotide (or simply an oligo) defined as nucleic acid being no longer than 150 bp, i.e. in accordance with the above provided definition, being any polymeric molecule comprising not more than 150 units (i.e. comprising maximally 150 units), wherein each unit comprises a nucleobase or a functional equivalent thereof, which renders said oligonucleotide capable of engaging in hydrogen bond-based

nucleobase pairing under appropriate hybridisation conditions with DNA or RNA. Within the ambit of said definition, it will immediately be appreciated that the disclosed herein oligonucleotides can comprise or consist of units not only being nucleotides but also being synthetic equivalents thereof. In other words, from a chemistry perspective, as used herein the term oligonucleotide will be construed as possibly comprising or consisting of RNA, DNA, or a nucleic acid analogue such as but not limited to LNA (BNA), PMO (Morpholino), PNA, GNA, TNA, HNA, FANA, FRNA, ANA, CeNA and/or the like.

In a particularly advantageous embodiment, the nucleic acid will be larger than 150 bp, preferably larger than 500 bp (i.e. 0.5 kb or 0.5 kbp), more preferably larger than 1000 bp (i.e. 1 kb or 1 kbp).

In preferred implementations, the nucleic acid is a circular, preferably double stranded DNA (dsDNA), such as a plasmid, mini-circle DNA, or other circular vector DNA. In some instances, the circular ds DNA such as plasmid or other circular vector DNA comprises a sequence that encodes for a therapeutic molecule such a therapeutic protein. For example, the sequence may encode for a component involved in gene editing such as a component of the clustered regularly interspaced short palindromic repeats (CRISPRs)/Cas gene system. Alternatively, the sequence may encode for a therapeutic RNA, which can be defined as an RNA molecule capable of exerting a therapeutic effect in a mammalian cell. Therapeutic RNAs include antisense RNAs, siRNAs, short hairpin RNAs, and enzymatic RNAs. The sequence may include nucleic acids intended to form triplex molecules, protein binding nucleic acids, ribozymes, deoxyribozymes, or small nucleotide molecules. In advantageous embodiments, the sequence can encode for a therapeutic peptide or a therapeutic protein, including cytotoxic proteins such as toxins (e.g. saporin or dianthin) or prodrugs; ribozymes; antisense or the complement thereof; or other such molecules.

In another embodiment, the nucleic acid may be used to effect gene therapy (also referred to as "genetic therapy"), for example by serving as a replacement or enhancement for a defective gene or to compensate for lack of a particular gene product, e.g. by encoding a therapeutic product. Alternatively, the nucleic acid may also inhibit expression of an endogenous gene or may encode all or a portion of a translation product, or may function by recombining with DNA already present in a cell, thereby replacing a defective portion of a gene. It further may also encode a portion of a protein and exert its effect by virtue of co-suppression of a gene product.

The term "saponin" has its regular established meaning and refers herein to a group of amphipathic glycosides which comprise one or more hydrophilic saccharide chains combined with a lipophilic aglycone core which is a sapogenin. The saponin may be naturally occurring or synthetic (i.e. non-naturally occurring). The term "saponin" includes naturally-occurring saponins, functional derivatives of naturally-occurring saponins as well as saponins synthesized *de novo* through chemical and/or biotechnological synthesis routes. Saponin according to the conjugate of the invention has a triterpene backbone, which is a pentacyclic C30 terpene skeleton, also referred to as sapogenin or aglycone. Within the conjugate of the invention saponin is not considered an effector molecule nor an effector moiety in the conjugates according to the invention. In the context of the conjugate of the invention, the term saponin refers to those saponins which exert an endosomal/lysosomal escape enhancing activity, when present in the endosome and/or lysosome of a mammalian cell such as a human cell, towards an effector moiety, here the nucleic acid, comprised by the polyplex of the invention and present in said endosome/lysosome together with the saponin.

As used herein, the term "saponin derivative" (also known as "modified saponin") shall be understood as referring to a compound corresponding to a naturally-occurring saponin that has been derivatised on the aldehyde group of the aglycone core; or on the carboxyl group of a saccharide chain or on an acetoxy group of a saccharide chain. Typically, the saponin derivative does not have a natural counterpart, i.e. the saponin derivative is not produced naturally by e.g. plants or trees. The term "saponin derivative" includes derivatives obtained by derivatisation of naturally-occurring saponins as well as derivatives synthesized *de novo* through chemical and/or biotechnological synthesis routes resulting in a compound corresponding to a naturally-occurring saponin which has been derivatised by one or more chemical modifications. A saponin derivative in the context of the invention should be understood as a saponin functional derivative. "Functional" in the context of the saponin derivative is understood as the capacity or activity of the saponin or the saponin derivative to enhance the endosomal escape of an effector molecule which is contacted with a cell together with the saponin or the saponin derivative.

The term "aglycone core structure" shall be understood as referring to the aglycone core of a saponin without the carbohydrate antennae or saccharide chains (glycans) bound thereto. For example, quillaic acid is the aglycone core structure for SO1861, QS-7 and QS21. Typically, the glycans of a saponin are mono-saccharides or oligo-saccharides, such as linear or branched glycans.

The term "saccharide chain" has its regular scientific meaning and refers to any of a glycan, a carbohydrate antenna, a single saccharide moiety (mono-saccharide) or a chain comprising multiple saccharide moieties (oligosaccharide, polysaccharide). The saccharide chain can consist of only saccharide moieties or may also comprise further moieties such as any one of 4E-Methoxycinnamic acid, 4Z-Methoxycinnamic acid, and 5-O-[5-O-Ara/Api-3,5-dihydroxy-6-methyl-octanoyl]-3,5-dihydroxy-6-methyl-octanoic acid), such as for example present in QS-21.

The term "Api/Xyl-" or "Api- or Xyl-" in the context of the name of a saccharide chain has its regular scientific meaning and here refers to the saccharide chain either comprising an apiose (Api) moiety, or comprising a xylose (Xyl) moiety.

As used herein, the term "an endocytic receptor" is to be understood as any one of cell surface molecules, likely receptors or transporters that are accessible to their specific ligands from the external side or surface of cell membrane (also known as plasmalemma) and capable of undergoing internalisation via endocytic pathway e.g., upon external stimulation, such as ligand binding to the receptor. In some embodiments, an endocytic receptor can be internalized by clathrin-mediated endocytosis, but can also be internalized by a clathrin-independent pathway, such as, for example, phagocytosis, macropinocytosis, caveolae- and raft-mediated uptake or constitutive clathrin-independent endocytosis. In some embodiments, the endocytic receptor comprises an intracellular domain, a transmembrane domain, and/or (e.g., and) an extracellular domain, which may optionally further comprise a ligand-binding domain. In some embodiments, the endocytic receptor becomes internalized by the cell after ligand binding. In some embodiments, a ligand may be a specific-cell-targeting agent, for example a natural ligand (or a synthetic fragment thereof) or an antibody or a binding fragment thereof.

As used herein, the term "ligand" is to be understood as any molecule that binds to or can be recognised by a receptor. Typical ligand can be an antibody, a binding fragment of an antibody, simply fragment of an antibody. Alternatively, a typical ligand can also be a protein, a peptide, a polysugar, a glycoprotein, or a fragment of any one thereof which fragment is capable of being recognised by an endocytic receptor.

As used herein, the term "antibody or a binding fragment thereof" refers to a polypeptide that includes at least one immunoglobulin variable domain or at least one antigenic determinant, e.g., paratope that specifically binds to an antigen. In some embodiments, an antibody is a full-length antibody. In some embodiments, an antibody is a chimeric antibody. In some embodiments, an antibody is a humanized antibody. However, in some embodiments, an antibody is a Fab fragment, a F(ab') fragment, a F(ab')2 fragment, a Fv fragment or a scFv fragment. In some embodiments, an antibody is a nanobody derived from a camelid antibody or a nanobody derived from shark antibody. In some embodiments, an antibody is a diabody. In some embodiments, an antibody comprises a framework having a human germline sequence. In another embodiment, an antibody comprises a heavy chain constant domain selected from the group consisting of IgG, IgG1, IgG2, IgG2A, IgG2B, IgG2C, IgG3, IgG4, IgAI, IgA2, IgD, IgM, and IgE constant domains. In some embodiments, an antibody comprises a heavy (H) chain variable region (abbreviated herein as VH), and/or (e.g., and) a light (L) chain variable region (abbreviated herein as VL). In some embodiments, an antibody comprises a constant domain, e.g., an Fc region. An immunoglobulin constant domain refers to a heavy or light chain constant domain. Human IgG heavy chain and light chain constant domain amino acid sequences and their functional variations are known. With respect to the heavy chain, in some embodiments, the heavy chain of an antibody described herein can be an alpha (a), delta (D), epsilon (e), gamma (g) or mu (m) heavy chain. In some embodiments, the heavy chain of an antibody described herein can comprise a human alpha (a), delta (D), epsilon (e), gamma (g) or mu (m) heavy chain. In a particular embodiment, an antibody described herein comprises a human gamma 1 CHI, CH2, and/or (e.g., and) CH3 domain. In some embodiments, the amino acid sequence of the VH domain comprises the amino acid sequence of a human gamma (g) heavy chain constant region, such as any known in the art. Non-limiting examples of human constant region sequences have been described in the art, e.g., see U.S. Pat. No. 5,693,780 and Kabat E A et al, (1991) supra. In some embodiments, the VH domain comprises an amino acid sequence that is at least 70%, 75%, 80%, 85%, 90%, 95%, 98%, or at least 99% identical to any of the variable chain constant regions provided herein. In some embodiments, an antibody is modified, e.g., modified via glycosylation, phosphorylation, sumoylation, and/or (e.g., and) methylation. In some embodiments, an antibody is a glycosylated antibody, which is conjugated to one or more sugar or carbohydrate molecules. In some embodiments, the one or more sugar or carbohydrate molecule are conjugated to the antibody via N-glycosylation, O-glycosylation, C-glycosylation, glypiation (GPI anchor attachment), and/or (e.g., and) phosphoglycosylation. In some embodiments, the one or more sugar or carbohydrate molecule are monosaccharides, disaccharides, oligosaccharides, or glycans. In some embodiments, the one or more sugar or carbohydrate molecule is a branched oligosaccharide or a branched glycan. In some embodiments, the one or more sugar or carbohydrate molecule includes a mannose unit, a glucose unit, an N-acetylglucosamine unit, an N-acetylgalactosamine unit, a galactose unit, a fucose unit, or a phospholipid unit. In some embodiments, an antibody is a construct that comprises a polypeptide comprising one or more antigen binding fragments of the disclosure linked to a linker polypeptide or an immunoglobulin constant domain. Linker polypeptides comprise two or more amino acid residues joined by peptide bonds and are used to link one or more antigen binding portions. Examples of linker polypeptides have been reported (see e.g., Holliger, P, et al. (1993) Proc. Natl. Acad. Sci. USA 90:6444-6448; Poljak, R. J., et al. (1994) Structure 2:1121-1123). Still further, an antibody may be part of a larger immunoadhesion molecule, formed by covalent or noncovalent association of the antibody or antibody portion with one or more other proteins or peptides. Examples of such immunoadhesion molecules include use of the streptavidin core region to make a tetrameric scFv molecule (Kipriyanov, S. M., et al. (1995) Human Antibodies and Hybridomas 6:93-101) and use of a cysteine residue, a marker peptide and a C-terminal polyhistidine tag to make bivalent and biotinylated scFv molecules (Kipriyanov, S. M., et al. (1994) Mol. Immunol. 31:1047-1058).

The term "single domain antibody", or "sdAb", in short, or 'nanobody', has its regular scientific meaning and here refers to an antibody fragment consisting of a single monomeric variable antibody domain, unless referred to as more than one monomeric variable antibody domain such as for example in the context of a bivalent sdAb, which

comprises two of such monomeric variable antibody domains in tandem. A bivalent nanobody is a molecule comprising two single domain antibodies targeting epitopes on molecules present at the extracellular side of a cell, such as epitopes on the extracellular domain of a cell surface molecule that is present on the cell. Preferably the cell-surface molecule is a cell-surface receptor. A bivalent nanobody is also named a bivalent single domain antibody. Preferably the two different single domain antibodies are directly covalently bound or covalently bound through an intermediate molecule that is covalently bound to the two different single domain antibodies. Preferably the intermediate molecule of the bivalent nanobody has a molecular weight of less than 10,000 Dalton, more preferably less than 5000 Dalton, even more preferably less than 2000 Dalton, most preferably less than 1500 Dalton.

As used herein, the term "covalently linked" refers to a characteristic of two or more molecules being linked together via at least one covalent bond, i.e. directly, or via a chain of covalent bonds, i.e. via a linker comprising at least one or more atoms.

As used herein, the term "conjugate" is to be construed as a combination of two or more different molecules that have been and are covalently bound. For example, different molecules forming a conjugate as disclosed herein may include one or more saponin molecules and one or more ligands that bind to an endocytic receptor, preferably wherein the ligand is an antibody or a binding fragment thereof, such as an IgG, a monoclonal antibody (mAb), a VHH domain or another nanobody type, a bivalent nanobody molecule comprising two single domain antibodies, etc. In some aspects, the disclosed herein conjugates may be made by covalently linking different molecules via one or more intermediate molecules such as linkers, such as for example via linking to a central or further linker. In a conjugate, not all of the two or more, such as three, different molecules need to be directly covalently bound to each other. Different molecules in the conjugate may also be covalently bound by being both covalently bound to the same intermediate molecule such as a linker or each by being covalently bound to an intermediate molecule such as a further linker or a central linker wherein these two intermediate molecules such as two (different) linkers, are covalently bound to each other. According to this definition even more intermediate molecules, such as linkers, may be present between the two different molecules in the conjugate as long as there is a chain of covalently bound atoms in between.

As used herein, the terms "administering" or "administration" means to provide a substance to a subject in a manner that is physiologically and/or (e.g., and) pharmacologically useful (e.g., to treat a condition in the subject).

As used herein, the term "approximately" or "about," as applied to one or more values of interest, refers to a value that is similar to a stated reference value. In certain embodiments, the term "approximately" or "about" refers to a range of values that fall within 15%, 14%, 13%, 12%, 11%, 10%, 9%, 8%, 7%, 6%, 5%, 4%, 3%, 2%, 1%, or less in either direction (greater than or less than) of the stated reference value unless otherwise stated or otherwise evident from the context (except where such number would exceed 100% of a possible value).

The terms first, second, third and the like in the description and in the claims, are used for distinguishing between for example similar elements, compositions, constituents in a composition, or separate method steps, and not necessarily for describing a sequential or chronological order. The terms are interchangeable under appropriate circumstances and the embodiments of the invention can operate in other sequences than described or illustrated herein, unless specified otherwise.

The embodiments as described herein can operate in combination and cooperation, unless specified otherwise. Furthermore, the various embodiments, although referred to as "preferred" or "e.g." or "for example" or "in particular" and the like are to be construed as exemplary manners in which the disclosed herein concepts may be implemented rather than as limiting.

The term "comprising", used in the claims, should not be interpreted as being restricted to for example the elements or the method steps or the constituents of a compositions listed thereafter; it does not exclude other elements or method steps or constituents in a certain composition. It needs to be interpreted as specifying the presence of the stated features, integers, (method) steps or components as referred to, but does not preclude the presence or addition of one or more other features, integers, steps or components, or groups thereof. Thus, the scope of the expression "a method comprising steps A and B" should not be limited to a method consisting only of steps A and B, rather with respect to the present invention, the only enumerated steps of the method are A and B, and further the claim should be interpreted as including equivalents of those method steps. Thus, the scope of the expression "a composition comprising components A and B" should not be limited to a composition consisting only of components A and B, rather with respect to the present invention, the only enumerated components of the composition are A and B, and further the claim should be interpreted as including equivalents of those components.

In addition, reference to an element or a component by the indefinite article "a" or "an" does not exclude the possibility that more than one of the element or component are present, unless the context clearly requires that there is one and only one of the elements or components. The indefinite article "a" or "an" thus usually means "at least one".

The term "Saponinum album" has its normal meaning and here refers to a mixture of saponins produced by Merck KGaA (Darmstadt, Germany) containing saponins from *Gypsophila paniculata* and *Gypsophila arostii,* containing SA1657 and mainly SA1641.

The term "Quillaja saponin" has its normal meaning and here refers to the saponin fraction of Quillaja saponaria

and thus the source for all other QS saponins, mainly containing QS-18 and QS-21.

"QS-21" or "QS21" has its regular scientific meaning and here refers to a mixture of QS-21 A-apio (-63%), QS-21 A-xylo (-32%), QS-21 B-apio (-3.3%), and QS-21 B-xylo (-1.7%).

Similarly, "QS-21A" has its regular scientific meaning and here refers to a mixture of QS-21 A-apio (-65%) and QS-21 A-xylo (-35%).

Similarly, "QS-21B" has its regular scientific meaning and here refers to a mixture of QS-21 B-apio (-65%) and QS-21 B-xylo (-35%).

The term "Quil-A" refers to a commercially available semi-purified extract from Quillaja saponaria and contains variable quantities of more than 50 distinct saponins, many of which incorporate the triterpene-trisaccharide substructure Gal-(1→2)-[Xyl-(1→3)]-GlcA- at the C-3beta-OH group found in QS-7, QS-17, QS-18, and QS-21. The saponins found in Quil-A are listed in van Setten (1995), Table *2 [*Dirk C. van Setten, Gerrit van de Werken, Gijsbert Zomer and Gideon F. A. Kersten, Glycosyl Compositions and Structural Characteristics of the Potential Immuno-adjuvant Active Saponins in the Quillaja saponaria Molina Extract Quil A, RAPID COMMUNICATIONS IN MASS SPECTROMETRY, VOL. 9,660-666 (1995)]. Quil-A and also Quillaja saponin are fractions of saponins from Quillaja saponaria and both contain a large variety of different saponins with largely overlapping content. The two fractions differ in their specific composition as the two fractions are gained by different purification procedures.

The term "QS1861" and the term "QS1862" refer to QS-7 and QS-7 api. QS1861 has a molecular mass of 1861 Dalton, QS1862 has a molecular mass of 1862 Dalton. QS1862 is described in Fleck et *al.* (2019) in Table 1, row no. 28 *[*Juliane Deise Fleck, Andresa Heemann Betti, Francini Pereira da Silva, Eduardo Artur Troian, Cristina Olivaro, Fernando Ferreira and Simone Gasparin Verza, Saponins from Quillaja saponaria and Quillaja brasiliensis: Particular Chemical Characteristics and Biological Activities, Molecules 2019, 24, 171; doi:10.3390/molecules24010171*].* The described structure is the api-variant QS1862 of QS-7. The molecular mass is 1862 Dalton as this mass is the formal mass including proton at the glucuronic acid. At neutral pH, the molecule is deprotonated. When measuring in mass spectrometry in negative ion mode, the measured mass is 1861 Dalton.

The terms "SO1861" and "SO1862" refer to the same saponin of *Saponaria officinalis,* though in deprotonated form or api form, respectively. The molecular mass is 1862 Dalton as this mass is the formal mass including a proton at the glucuronic acid. At neutral pH, the molecule is deprotonated. When measuring the mass using mass spectrometry in negative ion mode, the measured mass is 1861 Dalton.

## BRIEF DESCRIPTION OF FIGURES

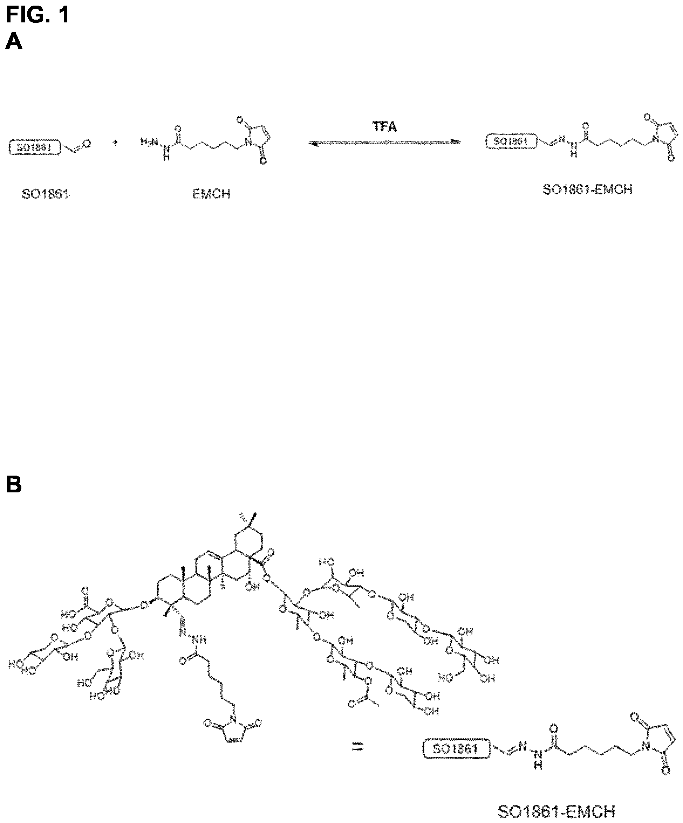

**Figure 1:** (A) Reaction scheme for pH-cleavable derivative of SO1861, further termed "SO1861-EMCH", (B) Skeletal structural formula of derivatized SO1861-EMCH and simplified schematic representations thereof;

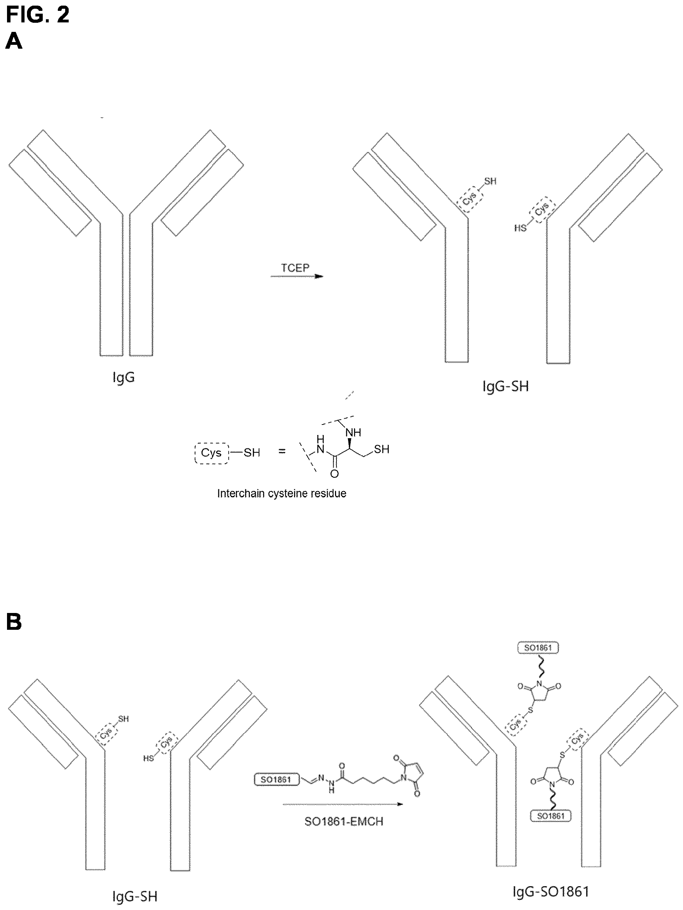

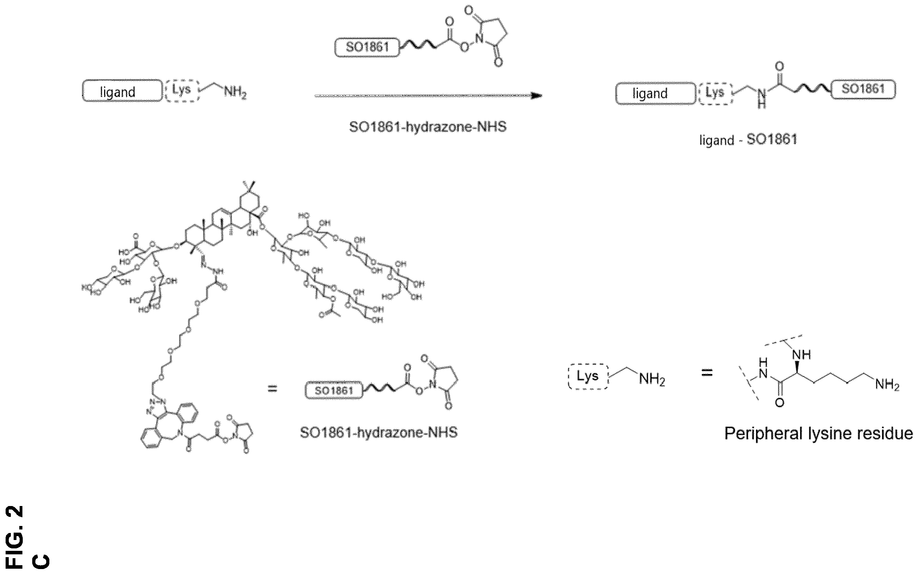

**Figure 2:** (A-B) Exemplary synthesis of monoclonal antibody-targeted SO1861 conjugates (e.g. with Cetuximab or Panitumumab, symbolically represented as an IgG scheme) yielding an intact mAb conjugated with SO1861 via interchain cysteines and then reassembled together by various forces as known in the art, wherein pane (A) shows generation of the mAb-SH intermediate (IgG-SH), while pane (B) shows synthesis of mAb-SO1861 (mAb is schematically represented by a general and simplified IgG and the final construct is marked as IgG-SO1861; merely for the purposes of clarity of schematic representation, the space between the heavy chains was enlarged); (C) Exemplary scheme of conjugation of SO1861-hydrazone-NHS to a proteinaceous ligand using peripheral lysine residues as exposed in said ligand;

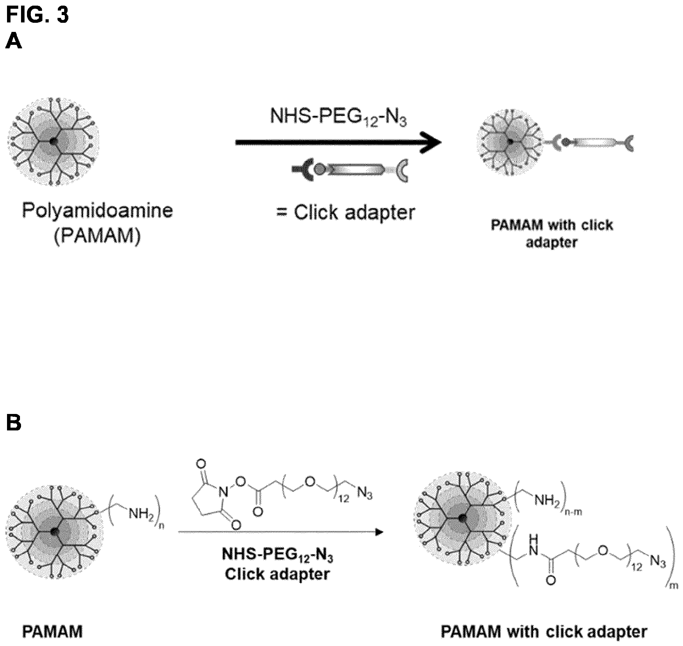

**Figure 3:** Reaction scheme of PAMAM dendrimer with a PEG adapter ending with a click-chemistry reactive azide group that can be used for conjugation with a targeting ligand. Such PEG adapter has the additional advantage of being potentially usable as a "click adapter" for conjugation of a targeting ligand or another molecule. The PEG adapter is shown using the example of NHS-$PEG_{12}$ adapter ending with the click-chemistry reactive azide but other designs of PEG adapters are also possible. (A) schematic representation, and (B) detailed representation when the click adapter moiety is considered;

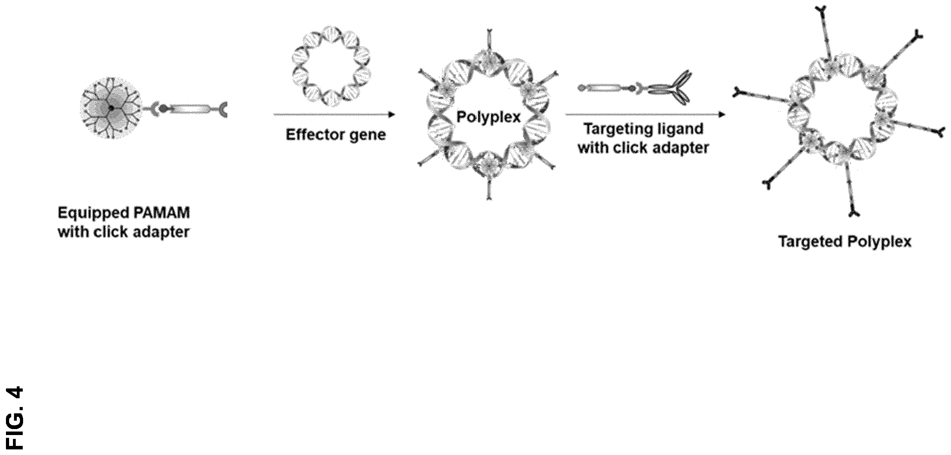

**Figure 4:** Schematic representation of a possible strategy for preparing an embodiment of a targeted polyplex assembled with an effector gene (schematically represented by a circular double stranded nucleic acid) and a polyplexing agent based on a pegylated PAMAM scaffold, which polyplexing agent is further conjugated with a targeting ligand by means of a click-chemical reaction of two compatible click adapters. The conjugation with the ligand can either be performed before assembling of the polyplex by mixing the effector gene with the thus obtained ligand-targeted polymeric scaffold (not shown); or after mixing the effector gene with the non-targeted polymeric

scaffold (as shown herein);

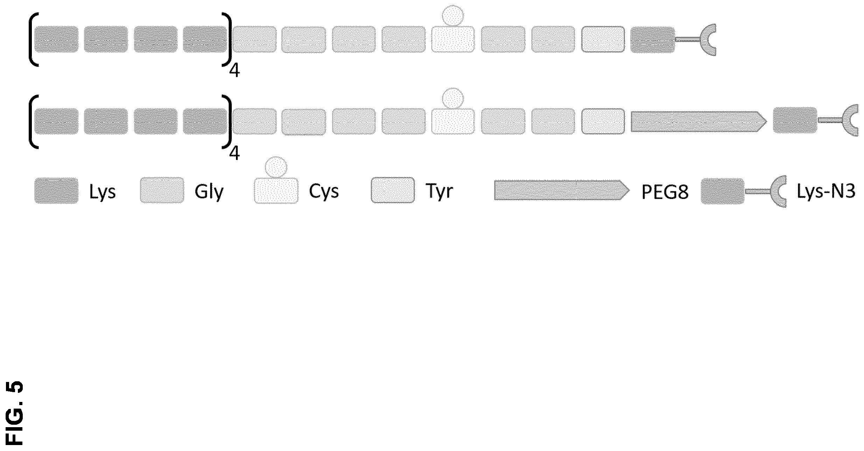

**Figure 5:** Schematic representation of one possible optimized peptide scaffold design with consecutive poly-lysine region at the N-terminal of the peptide and a C-terminal click-chemistry reactive azide group for conjugation of a targeting ligand (in this example introduced as part of a C-terminal azidolysine). C-terminal amidation of the peptide scaffold increases stability and prolongs its shelf life. The poly-lysine region and the C-terminal click-chemistry reactive azide group are separated by a spacer sequence primarily comprising glycine residues and (in present design) also a single cysteine and a single tyrosine that can be used for further various conjugation reactions;

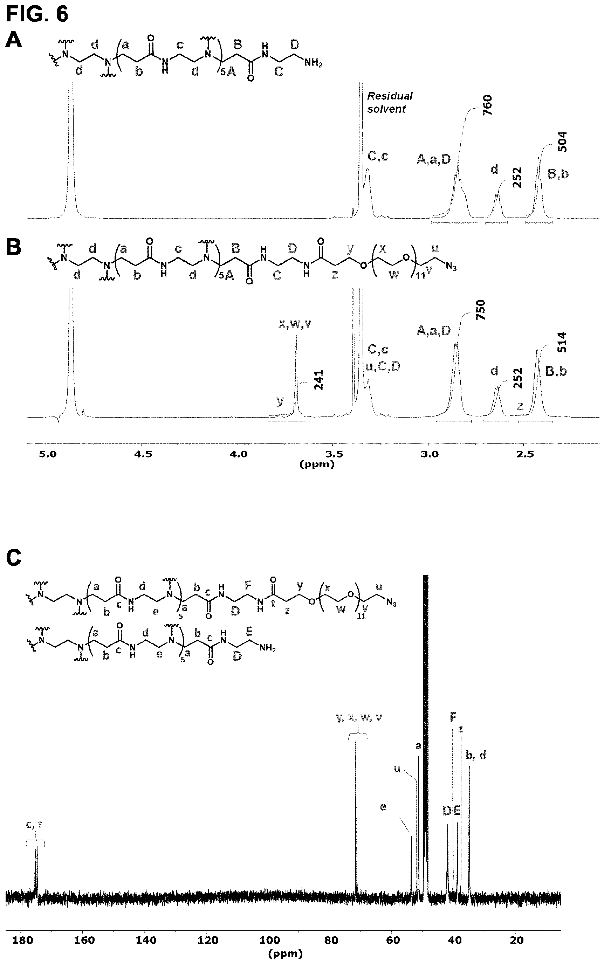

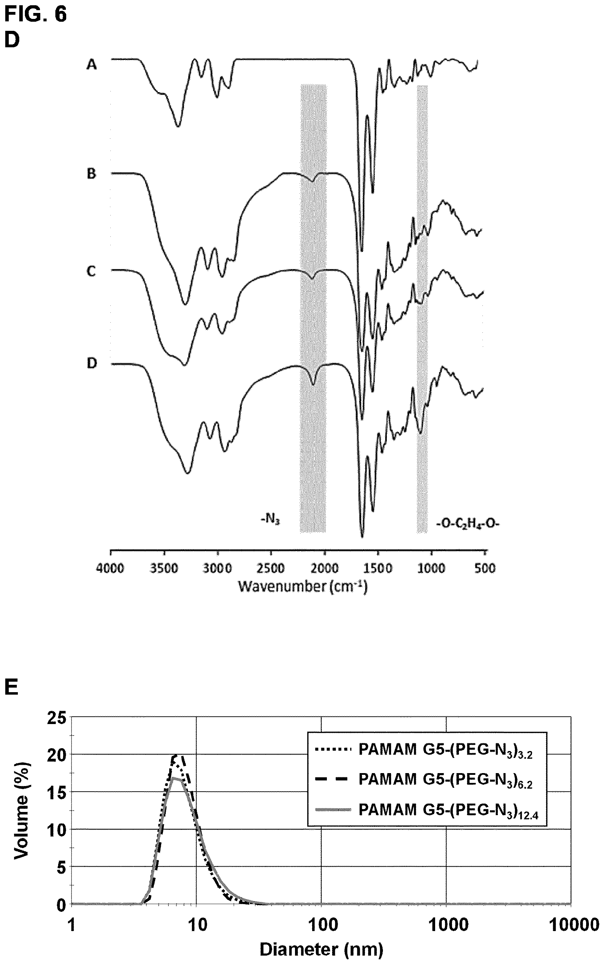

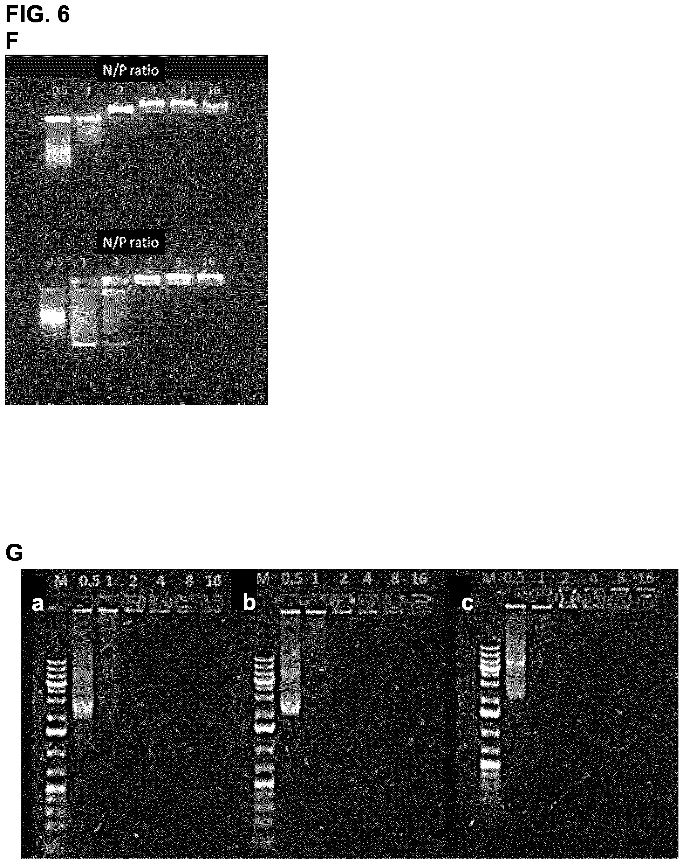

**Figure 6:** (A) $^{1}H$ NMR spectra ($CD_3OD$, 500 MHz) of commercial PAMAM G5 and (B) G5-(PEG-$N_3$)$_5$; (C) 13C NMR spectra ($CD_3OD$, 125 MHz) of G5-(PEG-$N_3$)$_6$; (D) IR spectra (KBr) of (a) PAMAM-G5, (b) PAMAM-G5-(PEG-$N_3$)$_{3.2}$, (c) PAMAM-G5-(PEG-$N_3$)$_{6.2}$, and (d) PAMAM-G5-(PEG-$N_3$)$_{12.4}$; (E) DLS size distribution of PAMAM-G5-(PEG-$N_3$) with PEGylation degrees 3.2, 6.2, and 12.4; (F) gel retardation of pUC19 DNA by complexation with highly PEGylated PAMAM conjugates and at different N/P ratios. Top image: complexes with PAMAM(-PEG$_{12}$-azide)$_{60}$; bottom: PAMAM(-PEG$_{12}$-azide)$_{80}$; (G) complexation efficiency of different PAMAM scaffolds as a function of N/P ratio used for polyplex formation. No free DNA was detected in gel retardation assay for all PAMAM scaffolds studied. (a) PAMAM-G5-(PEG$N_3$)$_{3.2}$, (b) PAMAM-G5-(PEG$N_3$)$_{6.2}$, (c) PAMAM-G5-(PEG$N_3$)$_{12.4}$;

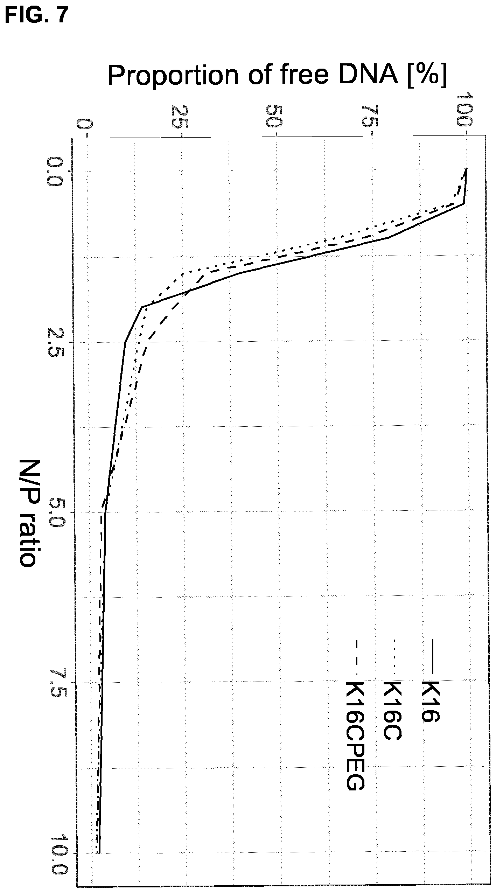

**Figure 7:** Complexation efficiency of peptide scaffold designs as shown in Figure 5 and as a function of N/P ratio used for polyplex formation. $\leq$ 2.5% free DNA was detected for the studied peptide scaffolds at N/P 10. Data is expressed as mean of triplicates. K16 = $K_{16}$, K16C = $K_{16}G_4$CGGYK($N_3$), K16CPEG = $K_{16}G_4$CGGY-PEG8-K($N_3$);

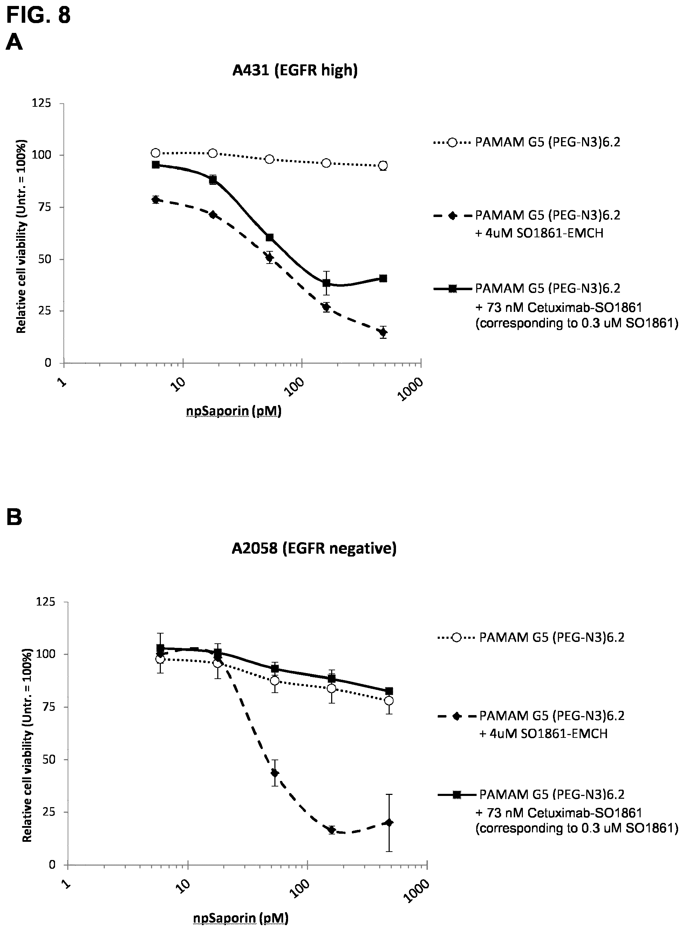

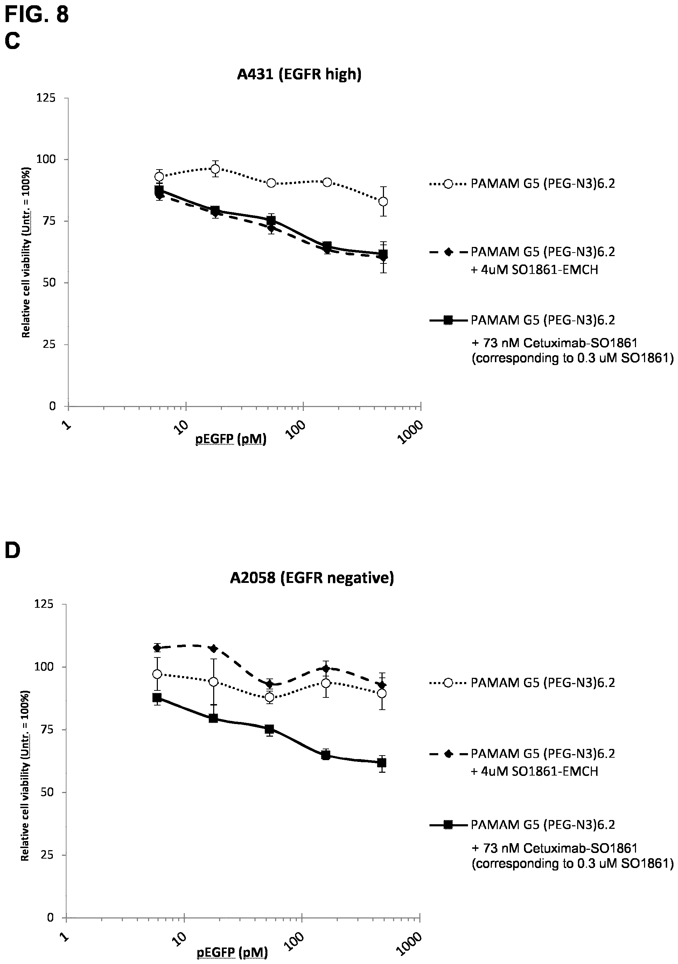

**Figure 8:** Transfection of a nanoplasmid encoding for saporin (npSaporin) or an EGFP-encoding plasmid (pEGFP-N3) that were polyplexed with pegylated PAMAM G5 into two different cell lines differing in EGFR-expression following co-administration with either Cetuximab-targeted SO1861 or non-targeted SO1861-EMCH ('SO1861-EMCH'); (A) npSaporin plasmid transfection to EGFR+ A431 cells; (B) npSaporin plasmid transfection to EGFR- A2058 cells; (C) pEGFP-N3 plasmid transfection to EGFR+ A431 cells; (D) pEGFP-N3 plasmid transfection to EGFR- A2058 cells; The cells were transfected for 72 h using different concentrations of the plasmids (pM) at N/P ratio of 8;

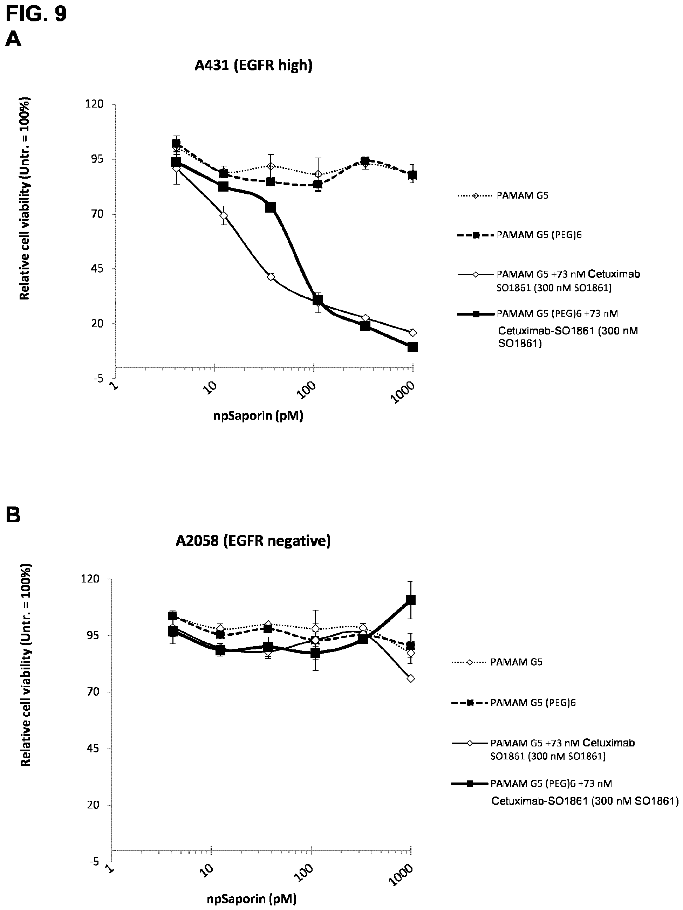

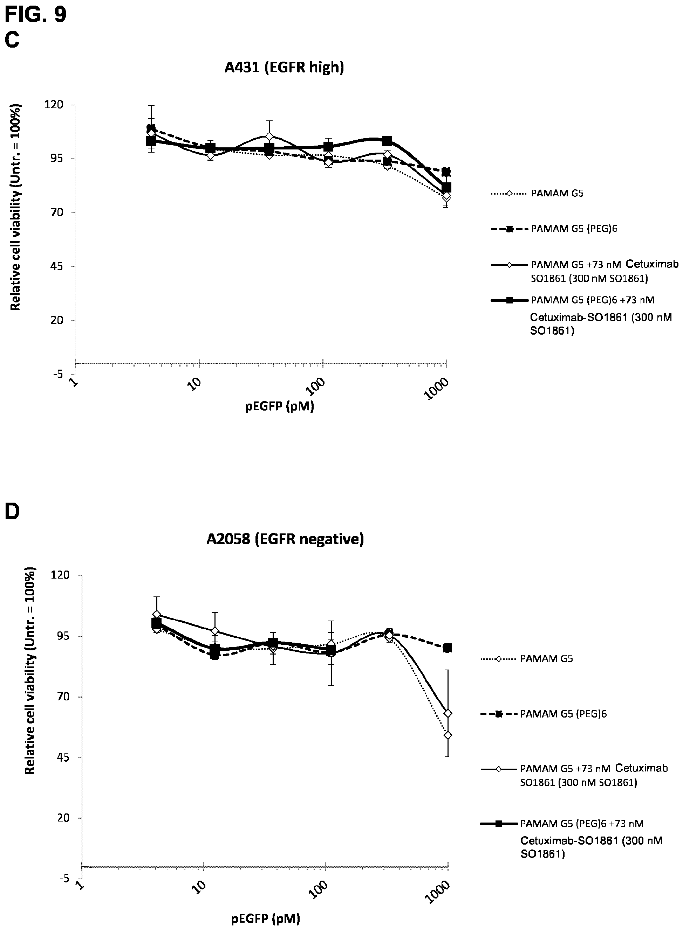

**Figure 9:** Transfection of a nanoplasmid encoding for saporin (npSaporin) or a EGFP-encoding plasmid that were polyplexed with pegylated or unpegylated PAMAM G5 into two different cell lines differing in EGFR-expression following co-administration with Cetuximab-targeted SO1861 or with free SO1861-EMCH; (A) npSaporin plasmid transfection to EGFR+ A431 cells; (B) npSaporin plasmid transfection EGFR- to A2058 cells; (C) pEGFP plasmid transfection to EGFR+ A431 cells; (D) pEGFP-N3 plasmid transfection to EGFR- A2058 cells; The cells were transfected for 72 h using different concentrations of the plasmids (pM) at N/P ratio of 8;

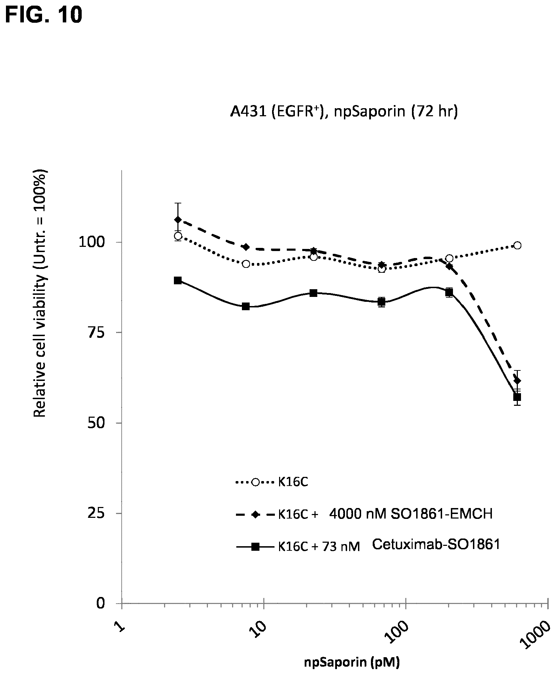

**Figure 10:** Transfection of nanoplasmid encoding for saporin (npSaporin) polyplexed with K16C peptide scaffold (N/P ratio 10), into A431 EGFR+ cells alone or with either Cetuximab-targeted SO1861 or non-targeted SO1861-EMCH;

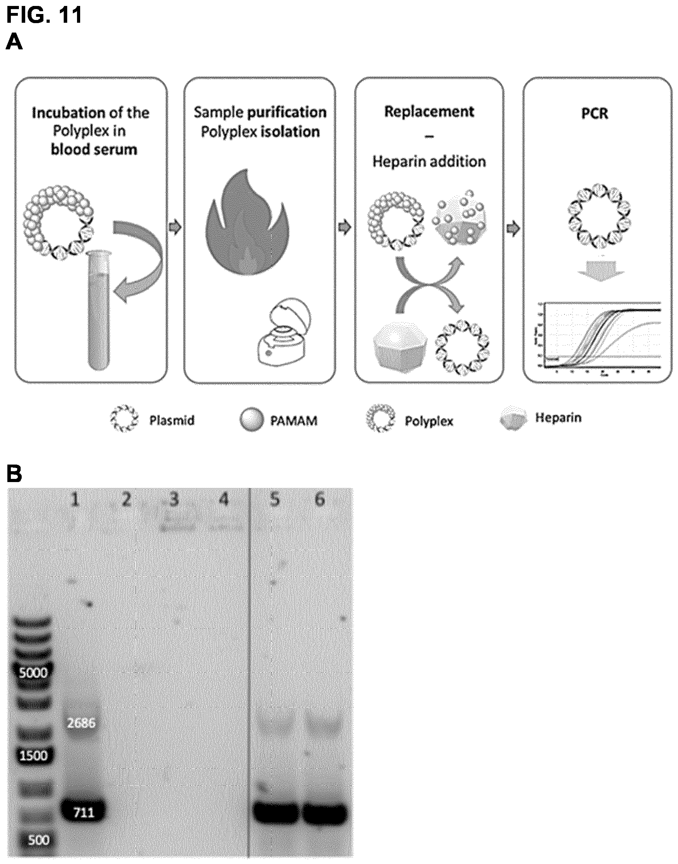

**Figure 11:** (A) Experimental setup of the assay for assessing stability of polyplexes in blood. (B) gel electrophoresis of samples treated according to the scheme shown in A: (1.) PCR positive control, (2.) negative control (DNA-free sample), (3.) peptide-based polyplex ($K_{16}$C = $K_{16}G_4$CGGYK($N_3$)) without heparin, (4.) polyplex prepared with PAMAM G5 without heparin, (5.) $K_{16}$C polyplex after release through heparin addition, (6.) PAMAM G5 polyplexes after heparin addition. White numbers on the DNA ladder indicate base pairs;

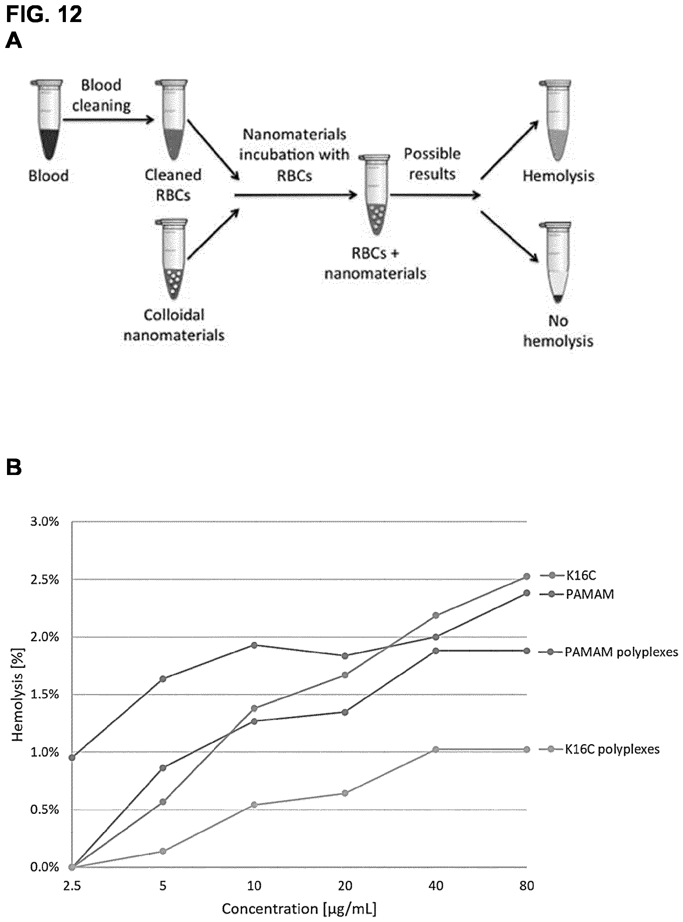

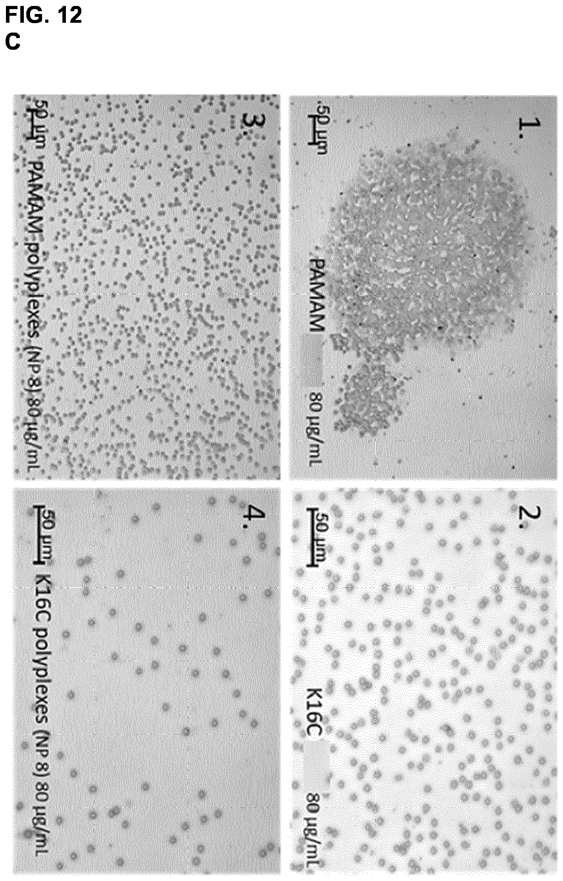

**Figure 12:** (A) Procedure of the hemolysis assay and possible results. The term "Nanomaterials" refers to the different PAMAM- or peptide-based scaffolds; (B) hemolytic activity of scaffolds and polyplexes at different concentrations. For better comparison, the concentrations are referred to the scaffold molecules PAMAM and K16C and not to the polyplexes; (C) morphology of RBCs treated with either of (1.) PAMAM, (2.) $K_{16}$C = $K_{16}G_4$CGGYK($N_3$), (3.) polyplexes prepared with PAMAM, (4.) polyplexes prepared with $K_{16}$C (as indicated) at a final test concentration of 80 $\mu$g/mL.

## DETAILED DESCRIPTION

In a first general aspect, disclosed herein are improved biologically active compositions, preferably pharmaceutical compositions for target-cell specific treatment with polyplexed nucleic acids. The target cell-specific component

of the disclosed compositions comprises a covalent conjugate of a ligand recognised by a first endocytic receptor and of an endocytic-escape-enhancing (EEE) saponin from the triterpenoid 12,13-dehydrooleanane saponin type that in an unreacted state comprises an aldehyde group at position C-23 of the saponin's aglycone core structure. The non-specific component of the disclosed compositions comprises a polyplex of a nucleic acid that will be specifically released into the cytosol of the target cell only in the presence of the EEE saponin brought in by the target cell-specific component. The disclosed herein compositions possess the particular advantage of exhibiting the highly desired property of enhanced and effective delivery of therapeutic nucleic acids from the polyplex, preferentially only within the target cells.

The innovative concepts presented herein will be described with respect to particular embodiments or aspects of the disclosure, that should be regarded as descriptive and not limiting beyond of what is described in the claims. The particular aspects as described herein can operate in combination and cooperation, unless specified otherwise. While the invention has been described with reference to these embodiments, it is contemplated that alternatives, modifications, permutations and equivalents thereof will become apparent to one having ordinary skill in the art upon reading the specification and upon study of the drawings and graphs. The invention is not limited in any way to the illustrated embodiments. Changes can be made without departing from the scope which is defined by the appended claims.

It is one of several objectives of embodiments of present disclosure to provide a solution to the problem of inefficient delivery encountered when administering nucleic acid-based therapeutics to human patients and in need of such therapeutics. It is a further one of several objectives of the embodiments to provide a solution to the problem of current nucleic acid-based therapies being less efficacious than desired, when administered to human patients in need thereof, due to not being sufficiently capable to reach and/or enter into to the target cell, e.g. a diseased cell, with little to no off-target activity on non-targeted cells, e.g. non-diseased cells, when administered to human patients in need thereof.

Without wishing to be bound by any theory, the disclosed herein novel pharmaceutical compositions were conceived based on the observation that a specific group of triterpenoid 12,13-dehydrooleanane-type saponins appears to exhibit potent endosomal-escape enhancing properties for nucleic acid-based therapeutics.

Endocytic pathways are complex and not fully understood. Nowadays, it is hypothesized that they involve stable compartments connected by vesicular traffic. A compartment is a complex, multifunctional membrane organelle that is specialized for a particular set of essential functions for the cell. Vesicles are considered to be transient organelles, simpler in composition, and are defined as membrane-enclosed containers that form *de novo* by budding from a pre-existing compartment. In contrast to compartments, vesicles can undergo maturation, which is a physiologically irreversible series of biochemical changes. Early endosomes and late endosomes represent stable compartments in the endocytic pathway while primary endocytic vesicles, phagosomes, multivesicular bodies (also called endosome carrier vesicles), secretory granules, and even lysosomes represent vesicles.

An endocytic vesicle, which arises at the plasma membrane, most prominently from clathrin-coated pits, first fuses with the early endosome, which is a major sorting compartment of approximately pH 6.5. A large part of the internalized cargo and membranes are recycled back to the plasma membrane through recycling vesicles (recycling pathway). Components that should be degraded are transported to the acidic late endosome (pH lower than 6) via multivesicular bodies. Lysosomes are vesicles that can store mature lysosomal enzymes and deliver them to a late endosomal compartment when needed. The resulting organelle is called the hybrid organelle or endolysosome. Lysosomes bud off the hybrid organelle in a process referred to as lysosome reformation. Late endosomes, lysosomes, and hybrid organelles are extremely dynamic organelles, and distinction between them is often difficult. Degradation of the endocytosed molecules occurs inside the endolysosomes.

*Endosomal escape* is the active or passive release of a substance from the inner lumen of any kind of compartment or vesicle from the endocytic pathway, preferably from clathrin-mediated endocytosis, or recycling pathway, into the cytosol. Endosomal escape thus includes but is not limited to release from endosomes, endolysosomes or lysosomes, including their intermediate and hybrid organelles. After entering the cytosol, said substance might move to other cell units such as the nucleus.

As will be demonstrated be the presented herein data, the inclusion of a triterpenoid 12,13-dehydrooleanane-type saponin comprising an aldehyde group at position C-23 of the saponin's aglycone core structure in the therapeutic compositions of the disclosure appeared to stimulate efficient exiting of the therapeutic nucleic acids' from polyplexes once present in the endosomes of the cells to which these saponins were delivered by means of a specific ligand and in which these saponins were released in the form comprising the aldehyde group at position C-23 thanks to appropriately designed linkers.

In line with these promising observations and findings, in a first general aspect, the invention provides a pharmaceutical composition comprising

a polyplex comprising at least one nucleic acid and a polymeric scaffold; and

a targeted saponin conjugate comprising a saponin and a first targeting ligand recognised by a first endocytic

receptor, wherein the saponin is covalently bound to the first targeting ligand by a linker adapted to cleave and release the saponin from the first targeting ligand under conditions present in an endosome or a lysosome; and

wherein the saponin is a triterpenoid 12,13-dehydrooleanane-type saponin that in an unreacted state comprises an aldehyde group at position C-23 of the saponin's aglycone core structure.

The above-referred to group of saponins possess triterpene 12,13-dehydrooleanane-type backbone core structure (also referred to as sapogenin or aglycone), usually shown as a pentacyclic C30 terpene skeleton, and further comprise an aldehyde group at position C-23 in their native (i.e. non-modified or non-reacted) state. Examples of such known saponins are shown in Table 1 and in Scheme Q

**TABLE 1. Saponins displaying (late) endosomal/lysosomal escape enhancing activity with an aglycone core of the 12,13-dehydrooleanane type[4)]**

| Saponin Name | Aglycone core with an aldehyde group at the C-23 position | Carbohydrate substituent at the C-3beta-OH group | Carbohydrate substituent at the C-28-OH group |
|---|---|---|---|
| NP-017777 | Gypsogenin | Gal-(1→2)-[Xyl-(1→73)]-GlcA- | Xyl-(1→4)-Rha-(1→2)-[R-(→4)]-Fuc- (R = 4E-Methoxycinnamic acid) |
| NP-017778 | Gypsogenin | Gal-(1→2)-[Xyl-(1→3)]-GlcA- | Xyl-(1→4)-Rha-(1→2)-[R-(→4)]-Fuc- (R = 4Z-Methoxycinnamic acid) |
| NP-017774 | Gypsogenin | Gal-(1→2)-[Xyl-(1→3)]-GlcA- | Xyl-(1→4)-[Gal-(1→3)]-Rha -(1→2)-4-OAc-Fuc- |
| NP-018110[c], NP-017772[d] | Gypsogenin | Gal-(1→2)-[Xyl-(1→3)]-GlcA- | Xyl-(1→4)-[Glc-(1→3)]-Rha -(1→2)-3,4-di-OAc-Fuc- |
| NP-018109 | Gypsogenin | Gal-(1→2)-[Xyl-(1→3)]-GlcA- | Xyl-(1→4)-[Glc-(1→3)]-Rha -(1→2)-[R-(→4)]-3-OAc-Fuc- (R = 4E-Methoxycinnamic acid) |
| NP-017888 | Gypsogenin | Gal-(1→2)-[Xyl-(1→3)]-GlcA- | Glc-(1→3)-Xyl-(1→4)-[Glc-(1→3)]-Rha-(1→2)-4-OAc-Fuc- |
| NP-017889 | Gypsogenin | Gal-(1→2)-[Xyl-(1→3)]-GlcA- | Glc-(1→3)-Xyl-(1→4)-Rha-(1→2)-4-OAc-Fuc- |
| NP-018108 | Gypsogenin | Gal-(1→2)-[Xyl-(1→3)]-GlcA- | Ara/Xyl-(1→3)-Ara/ Xyl-(1→4)-Rha/Fuc-(1→2)-[4-OAc-Rha/ Fuc-(1→4)]-Rha/Fuc- |
| SA1641[a], AE X55[b] | Gypsogenin | Gal-(1→2)-[Xyl-(1→3)]-GlcA- | Xyl-(1→3)-Xyl-(1→4)-Rha-(1→2)-[Qui- (1→4)]-Fuc- |
| S01658 | Gypsogenin | Gal-(1→2)-[Xyl-(1→3)]-GlcA- | Glc-(1→3)-[Xyl-(1→3)-Xyl-(1→4)]-Rha- (1→2)-Fuc- |
| gypsoside A[6)] | Gypsogenin | Gal-(1→4)-Glc (1→4)-[Ara-(1→3)]-GlcA- | Xyl-(1→3)-Fuc-(1→4)-[Xyl-(1→3)-Xyl-(1→3)]-Rha- |
| phytolaccagenin | Gypsogenin | absent | absent |
| | | | |

(continued)

| TABLE 1. Saponins displaying (late) endosomal/lysosomal escape enhancing activity with an aglycone core of the 12,13-dehydrooleanane type[4)] | | | |
|---|---|---|---|
| **Saponin Name** | **Aglycone core with an aldehyde group at the C-23 position** | **Carbohydrate substituent at the C-3beta-OH group** | **Carbohydrate substituent at the C-28-OH group** |
| Gypsophila saponin 1 (Gyp1) | Quillaic acid | Gal-(1→2)-[Xyl-(1→3)]-GlcA- | Glc-(1→3)-[Xyl-(1→4)]-Rha -(1→2)-Fuc- |
| NP-017674 | Quillaic acid | Gal-(1→2)-[Xyl-(1→3)]-GlcA- | Api-(1→3)-Xyl-(1→4)-[Glc-(1→3)]-Rha- (1→2)-Fuc- |
| NP-017810 | Quillaic acid | Gal-(1→2)-[Xyl-(1→3)]-GlcA- | Xyl-(1→4)-[Gal-(1→3)]-Rha -(1→2)-Fuc- |
| AG1 | Quillaic acid | Gal-(1→2)-[Xyl-(1→3)]-GlcA- | Xyl-(1→4)-[Glc-(1→3)]-Rha -(1→2)-Fuc- |
| NP-003881 | Quillaic acid | Gal-(1→2)-[Xyl-(1→3)]-GlcA- | Ara/Xyl-(1→4)-Rha/ Fuc-(1→4)-[Glc/Gal-(1→2)]-Fuc- |
| NP-017676 | Quillaic acid | Gal-(1→2)-[Xyl-(1→3)]-GlcA- | Api-(1→3)-Xyl-(1→4)-[Glc-(1→3)]-Rha-(1→2)-[R-(→4)]-Fuc-(R = 5-O-[5-O-Ara/Api-3,5-dihydroxy-6-methyl-octanoyl]-3,5-dihydroxy-6-methyl- octanoic acid) |
| NP-017677 | Quillaic acid | Gal-(1→2)-[Xyl-(1→3)]-GlcA- | Api-(1→3)-Xyl-(1→4)-Rha-(1→2)-[R-(→4)]-Fuc-(R = 5-O-[5-O-Ara/Api-3,5-dihydroxy-6-methyl-octanoyl]-3,5-dihydroxy-6-methyl- octanoic acid) |
| NP-017706 | Quillaic acid | Gal-(1→2)-[Xyl-(1→3)]-GlcA- | Api-(1→3)-Xyl-(1→4)-Rha-(1→2)-[Rha- (1→3)]-4-OAc-Fuc- |
| NP-017705 | Quillaic acid | Gal-(1→2)-[Xyl-(1→3)]-GlcA- | Api-(1→3)-Xyl-(1→4)-[Glc-(1→3)]-Rha-(1→2)-[Rha-(1→3)]-4-OAc-Fuc- |
| NP-017773 | Quillaic acid | Gal-(1→2)-[Xyl-(1→3)]-GlcA- | 6-OAc-Glc-(1→3)-Xyl-(1→4)-Rha-(1→2)-[3-OAc-Rha-(1→3)]-Fuc- |
| NP-017775 | Quillaic acid | Gal-(1→2)-[Xyl-(1→3)]-GlcA- | Glc-(1→3)-Xyl-(1→4)-Rha-(1→2)-[3-OAc-Rha-(1→3)]-Fuc- |
| SA1657 | Quillaic acid | Gal-(1→2)-[Xyl-(1--73)]-GlcA- | Xyl-(1→3)-Xyl-(1→4)-Rha-(1→2)-[Qui- (1→4)]-Fuc- |

(continued)

| TABLE 1. Saponins displaying (late) endosomal/lysosomal escape enhancing activity with an aglycone core of the 12,13-dehydrooleanane type[4)] | | | | |
|---|---|---|---|---|
| **Saponin Name** | **Aglycone core with an aldehyde group at the C-23 position** | **Carbohydrate substituent at the C-3beta-OH group** | **Carbohydrate substituent at the C-28-OH group** | |
| AG2 | Quillaic acid | Gal-(1→2)-[Xyl-(1→3)]-GlcA- | Glc-(1→3)-[Xyl-(1→4)]-Rha -(1→2)-[Qui- (1→4)]-Fuc- | |
| GE1741 | Quillaic acid | Gal-(1→2)-[Xyl-(1→3)]-GlcA- | Xyl-(1→3)-Xyl-(1→4)-Rha-( 1→2)-[3,4-di-OAc-Qui-(1→4)]-Fuc- | |
| S01542 | Quillaic acid | Gal-(1→2)-[Xyl-(1→3)]-GlcA- | Glc-(1→3)-[Xyl-(1→4)]-Rha -(1→2)-Fuc- | |
| S01584 | Quillaic acid | Gal-(1→2)-[Xyl-(1→3)]-GlcA- | 6-OAc-Glc-(1→3)-[Xyl-(1→4)]-Rha -(1→2)-Fuc- | |
| S01674 | Quillaic acid | Gal-(1→2)-[Xyl-(1→3)]-GlcA- | Glc-(1→3)-[Xyl-(1→3)-Xyl-( 1→4)]-Rha- (1→2)-Fuc- | |
| SO1700[3)] | Quillaic acid | Gal-(1→2)-[Xyl-(1→3)]-GlcA- | Xyl-(1→4)-Rha-(1→2)-[Xyl-(1→3)-4-OAc-Qui-(1→4)]-Fuc- | |
| Saponarioside B[1)] | Quillaic acid | Gal-(1→2)-[Xyl-(1→3)]-GlcA- | Xyl-(1→3)-Xyl-(1→4)-Rha-( 1→2)-[4-OAc-Qui-(1→4)]-Fuc- | |
| SO1730[3)] | Quillaic acid | Gal-(1→2)-[Xyl-(1→3)]-GlcA- | Glc-(1→3)-Xyl-(1→4)-Rha-(1→2)-[-4-OAc-Qui-(1→4)]-Fuc- | |
| SO1772[3)] | Quillaic acid | Gal-(1→2)-[Xyl-(1→3)]-GlcA- | 6-OAc-Glc-(1→3)-[Xyl-(1→4)]-Rha -(1→2)-[4-OAc-Qui-(1→4)]-Fuc-- | |
| SO1832[1)] (protonated S01831) = Saponarioside A | Quillaic acid | Gal-(1→2)-[Xyl-(1→3)]-GlcA- | Xyl-(1→3)-Xyl-(1→4)-Rha-( 1→2)-[Xyl-(1→3)-4-OAc-Qui-(1→4)]-Fuc- | |
| S01861 (deprotonated S01862) | Quillaic acid | | Gal-(1→2)-[Xyl-(1→3)]-Glc A- | Glc-(1→3)-Xyl-(1→4)-Rha -(1→2)-[Xyl-(1→3)-4-OAc-Qui-(1→4)]-Fuc- |
| S01862 (protonated SO1861), also referred to as *Sapofectosid*[5)] | Quillaic acid | | Gal-(1→2)-[Xyl-(1→3)]-Glc A- | Glc-(1→3)-Xyl-(1→4)-Rha -(1→2)-[Xyl-(1→3)-4-OAc-Qui-(1→4)]-Fuc- |
| SO1904[3)] | Quillaic acid | | Gal-(1→2)-[Xyl-(1→3)]-Glc A- | 6-OAc-Glc-(1→3)-[Xyl-(1→4)]-Rh a-(1→2)-[Xyl-(1→3)-4-OAc-Qui-(1→4)]-Fuc- |

(continued)

| TABLE 1. Saponins displaying (late) endosomal/lysosomal escape enhancing activity with an aglycone core of the 12,13-dehydrooleanane type[4)] | | | |
|---|---|---|---|
| **Saponin Name** | **Aglycone core with an aldehyde group at the C-23 position** | **Carbohydrate substituent at the C-3beta-OH group** | **Carbohydrate substituent at the C-28-OH group** |
| QS-7 (also referred to as QS1861) | Quillaic acid | Gal-(1→2)-[Xyl-(1→3)]-GlcA- | Api/Xyl-(1→3)-Xyl-(1→4)-[Glc-(1→3)]-Rha-(1→2)-[Rha-(1→3)]-4OAc-Fuc- |
| QS-7 api (also referred to as QS1862) | Quillaic acid | Gal-(1→2)-[Xyl-(1→3)]-GlcA- | Api-(1→3)-Xyl-(1→4)-[Glc-(1→3)]-Rha-(1→2)-[Rha-(1→3)]-4OAc-Fuc- |
| QS-17 | Quillaic acid | Gal-(1→2)-[Xyl-(1→3)]-GlcA- | Api/Xyl-(1→3)-Xyl-(1→4)-[Glc-(1→3)]-Rha-(1→2)-[R-(→4)]-Fuc-(R = 5-O-[5-O-Rha-(1→2)-Ara/Api-3,5-dihydroxy-6-methyl-octanoyl]-3,5-dihydroxy-6-methyl-octanoic acid) |
| QS-18 | Quillaic acid | Gal-(1→2)-[Xyl-(1→3)]-GlcA- | Api/Xyl-(1→3)-Xyl-(1→4)-[Glc-(1→3)]-Rha-(1→2)-[R-(→4)]-Fuc-(R = 5-O-[5-O-Ara/Api-3,5-dihydroxy-6-methyl-octanoyl]-3,5-dihydroxy-6-methyl-octanoic acid) |
| QS-21 A-apio | Quillaic acid | Gal-(1→2)-[Xyl-(1→3)]-GlcA- | Api-(1→3)-Xyl-(1→4)-Rha-(1→2)-[R-(→4)]-Fuc-(R = 5-O-[5-O-Ara/Api-3,5-dihydroxy-6-methyl-octanoyl]-3,5-dihydroxy-6-methyl-octanoic acid) |
| QS-21 A-xylo | Quillaic acid | Gal-(1→2)-[Xyl-(1→3)]-GlcA- | Xyl-(1→3)-Xyl-(1→4)-Rha-(1→2)-[R-(→4)]-Fuc-(R = 5-O-[5-O-Ara/Api-3,5-dihydroxy-6-methyl-octanoyl]-3,5-dihydroxy-6-methyl-octanoic acid) |
| QS-21 B-apio | Quillaic acid | Gal-(1→2)-[Xyl-(1→3)]-GlcA- | Api-(1→73)-Xyl-(1→4)-Rha-(1→2)-[R-(→3)]-Fuc-(R = 5-O-[5-O-Ara/Api-3,5-dihydroxy-6-methyl-octanoyl]-3,5-dihydroxy-6-methyl-octanoic acid) |

(continued)

| TABLE 1. Saponins displaying (late) endosomal/lysosomal escape enhancing activity with an aglycone core of the 12,13-dehydrooleanane type[4)] | | | | |
|---|---|---|---|---|
| **Saponin Name** | **Aglycone core with an aldehyde group at the C-23 position** | **Carbohydrate substituent at the C-3beta-OH group** | **Carbohydrate substituent at the C-28-OH group** | |
| QS-21 B-xylo | Quillaic acid | | Gal-(1→2)-[Xyl-(1→3)]-Glc A- | Xyl-(1→3)-Xyl-(1→4)-Rha -(1→2)-[R-(→3)]-Fuc-(R = 5-O-[5-O-Ara/Api-3,5-dihydroxy-6-methyl-octanoyl]-3,5-dihydroxy-6-methyl-octanoic acid) |
| QS-21 | | Quillaic acid | Gal-(1→2)-[Xyl-(1→3)]-Glc A- | Combination of the carbohydrate chains depicted for QS-21 A-apio, A-xylo, B-apio, B-xylo, for this position at the aglycone (see also the structure depicted as (Scheme Q)) |
| Agrostemmoside E (AG1856, AG2.8)[2)] | | Quillaic acid | Gal-(1→2)-[Xyl-(1→3)]-Glc A- | [4,6-di-OAc-Glc-(1→3)]-[Xyl-(1→4)]-R ha- (1→2)-[3,4-di-OAc-Qui-(1→4)]-Fuc- |

a, b: Different names refer to different isolates of the same structure

c, d: Different names refer to different isolates of the same structure

1) Jia et al., Major Triterpenoid Saponins from Saponaria officinalis, J. Nat. Prod. 1998, 61, 11, 1368-1373, Publication Date: September 19, 1998, https://doi.org/10.1021/np980167u

2) The structure of Agrostemmoside E (also referred to as AG1856 or AG2.8) is given in Fig. 4 of J. Clochard et al, A new acetylated triterpene saponin from Agrostemma githago L. modulates gene delivery efficiently and shows a high cellular tolerance, International Journal of Pharmaceutics, Volume 589, 15 November 2020, 119822.

3) Structures of SO1700, SO1730, SO1772, SO1904 are given in Moniuszko-Szajwaj et al., Highly Polar Triterpenoid Saponins from the Roots of Saponaria officinalis L., Helv. Chim. Acta, V99, pp. 347 - 354, 2016 (doi.org/10.1002/hlca. 201500224).

4) See for example:

- thesis by Dr Stefan Böttger (2013): Untersuchungen zur synergistischen Zytotoxizität zwischen Saponinen und Ribosomen inaktivierenden Proteinen Typ I, and
- Sama et al., Structure-Activity Relationship of Transfection-Modulating Saponins - A Pursuit for the Optimal Gene Trafficker, Planta Med. Volume 85, pp. 513-518, 2019 (doi:10.1055/a-0863-4795) and
- Fuchs et al., Glycosylated Triterpenoids as Endosomal Escape Enhancers in Targeted Tumor Therapies, Biomedicine, Volume 5, issue 14, 2017 (doi:10.3390/biomedicines5020014).

5) Sama et al., Sapofectosid - Ensuring non-toxic and effective DNA and RNA delivery, International Journal of Pharmaceutics, Volume 534, Issues 1-2, 20 December 2017, Pages 195-205 (dx.doi.org/10.1016/j.ijpharm. 2017.10.016) & Moniuszko-Szajwaj et al., Highly Polar Triterpenoid Saponins from the Roots of Saponaria officinalis L., Helv. Chim. Acta, V99, pp. 347 - 354, 2016 (doi.org/10.1002/hlca.201500224).

6) See for example: doi:10.1016/s0040-4039(01)90658-6, Tetrahedron Letters No. 8, pp. 477-482, 1963 and pubchem.ncbi.nlm.nih.gov/compound/Gipsoside

QS21(4 isomers: Api/Xyl (2:1))

QS21 A api

QS21 A xyl

QS21 B api

QS21 B xyl

**(Scheme Q)**

A notable feature of these saponins is the aldehyde group at position C-23 of the saponin's aglycone core structure. Without wishing to be bound by any theory, it was observed that presence of said aldehyde group in the

aglycone core structure of the saponin (here, also referred to as 'aglycone') is particularly beneficial for the capacity of the saponin to stimulate and/or potentiate the endosomal escape of the therapeutic nucleic acids comprised by the polyplex of the invention or by the cell-surface molecule targeted polyplex of the invention.

Consequently, in possible advantageous embodiments, the targeted saponin conjugate is prepared such that the aldehyde group at position C-23 of the saponin's aglycone core structure is present or restored under acidic conditions present in mammalian endosomes and/or lysosomes.

Advantageously, the saponin can be covalently bound to the ligand by an acid-sensitive linker through the position C-23 of the saponin's aglycone core structure and wherein the acid-sensitive linker is adapted to cleave and release the saponin from the ligand such that the aldehyde group at position C-23 of the saponin's aglycone core structure is restored under acidic conditions present in mammalian endosomes and/or lysosomes.

Hence, in a preferred embodiment, a pharmaceutical composition is provided, wherein the linker is an acid-sensitive linker or is a linker configured to be enzymatically cleaved by an enzyme present in the endosome or the lysosome, preferably wherein the linker is an acid-sensitive linker adapted to cleave and release the saponin from the first targeting ligand under acidic conditions present in the endosome or the lysosome, i.e. in particular conditions present in mammalian endosomes and/or lysosomes, defined as conditions of pH < 6, preferably pH < 5.5, more preferably pH < 5; even more preferably pH < 4.5, most preferably pH < 4.

For the endosomal escape enhancing properties, it appears to be particularly beneficial that the aldehyde group at position C-23 of the saponin's aglycone core structure is restored to a free aldehyde group once inside of the endosome, and hence any chemical modifications that uncap or restore the free aldehyde functional group at the position at position C-23 of the saponin's aglycone core structure under acidic conditions present in endosomes and/or lysosomes of human cells can in principle be applied to the disclosed herein suitable saponins shown in their native form in Table 1.

For example, endosomal-escape-enhancing properties of such saponins are still also very pronounced when the aldehyde group is e.g. substituted by a maleimide-comprising moiety attached at said position C-23 with a cleavable covalent bond that cleaves off under acidic conditions present in endosomes and/or lysosomes of human cells, whereby said aldehyde group at position C-23 of the saponin's aglycone core structure is restored upon said cleavage under acidic conditions present in endosomes and/or lysosomes of human cells.

In advantageous embodiments, such cleavable covalent bond can be selected from a hydrazone bond, or an imine bond.

For example, in further possible embodiments, the maleimide-comprising moiety can be a part of a molecule comprising or consisting of N-ε-mateimidocaproic acid hydrazide that is attached at position C-23 of the saponin's aglycone core structure upon forming a hydrazone bond (further referred to as EMCH).

Hence, in a possible embodiment, a composition is disclosed, wherein the aldehyde group at position C-23 of the saponin's aglycone core structure is either a free aldehyde group, or is an aldehyde group substituted by a maleimide-comprising moiety attached at said position C-23 with a cleavable covalent bond that cleaves off under acidic conditions present in endosomes and/or lysosomes of human cells, wherein said aldehyde group at position C-23 of the saponin's aglycone core structure is restored upon said cleavage under acidic conditions present in endosomes and/or lysosomes of human cells; preferably wherein the cleavable covalent bond is selected from a hydrazone bond, or an imine bond.

Advantageously, the polymeric scaffold can be based on any one of the known dendriplexes, which are easy to modify, can be selected to exhibit hydrodynamic diameters in the suitable nanometer range, and are already used for gene delivery and gene transfection purposes (Dufes et al., 2005). Dendrimers can provide hyperbranched structures comprising layers of monomers radiating from a central core (Kodama et al., 2014), which makes them particularly suitable for being fine-tuned by chemical modifications.

Hence, in a further preferred embodiment a pharmaceutical composition is provided, wherein the polymeric scaffold is or comprises polyamidoamine dendrimer, further referred to as PAMAM.

Preferably the PAMAM can comprise ethylenediamine core and/or is at least generation 3 PAMAM or higher, preferably is generation 5 PAMAM.

Possibly and preferably, the PAMAM may comprise at least one pegylated group, preferably comprising at least one PEG adapter. Preferably, the PEG adapter may comprise 5-15 PEG units, more preferably being 6-12 PEG units. Even more preferably, the pegylation degree of the PAMAM is comprised between on average 1.5-20 equivalents of PEG adapter per PAMAM core, preferably being on average 2-16 equivalents of PEG adapter per PAMAM core, even more preferably being on average 3-13 equivalents of PEG adapter per PAMAM core, most preferably being on average selected from any one of 3.2, 6.2, and 12.4 PEG adapter equivalents per PAMAM core.

Alternatively, the polymeric scaffold is or comprises a polypeptide, for example comprising sufficient amount of lysines and/or arginines to provide the required for nucleic acid binding net positive charge. Peptides containing repeating lysine or arginine motifs are known to complex genetic material and have certain advantages. For example, they are usually biocompatible and fully degradable but have limited binding sites. However, poly-lysine peptides are also known to lack the so called "proton sponge" ability (Vasiliu et al., 2017), which makes them inferior in gene delivery as compared to other polymeric scaffolds, e.g. based on PEI, due to not being able to destabilise endosomes. However,

as in the present context, the EEE-property is conferred by the targeted saponin conjugate and cell-target specificity if of importance, this particular feature of polylysine molecular scaffolds may contribute to avoiding off-target effects in cells where the effector therapeutic nucleic acid should not be released. Consequently, polymeric scaffolds comprising or consisting of a polypeptide comprising lysines can be particularly advantageous.

In line with this, in an alternative possible embodiment, a pharmaceutical composition is provided, wherein the polymeric scaffold is or comprises a polypeptide comprising between 5 to 25 lysines and preferably also at least one cysteine, more preferably being exactly one cysteine.

Preferably, such polypeptide comprises exactly one cysteine flanked by a string of 1-7 glycines from each side, preferably 1-5 glycines from each side, more preferably 2-4 glycines, even more preferably wherein the exactly one cysteine is flanked by 3-7 glycines from N-terminal side, preferably being 4 glycines; and by 1-4 glycines from C-terminal side, preferably being 2 glycines.

Optionally, the polypeptide may further comprise at least one C-terminal tyrosine.

As another option, the polypeptide may be terminated with a residue comprising an azide group, preferably being azidolysine, preferably 6-azido-L-lysine, which is beneficial for various ligation purposes using e.g. click-chemistry.

In a particularly advantageous embodiment, the polypeptide is represented by a general formula of, from N-terminus to C-terminus: 2-25 Lys; 3-7 Gly; 1 Cys; 2-4 Gly, and possibly 1 or more Tyr; preferably wherein the polypeptide is represented by an amino acid sequence given by SEQ ID NO. 1: KKKKKKKKKKKKKKKKGGGGCGGY or SEQ ID NO. 2: KKKKKKKKKKKKKKKKGGGGCGGYK* wherein K* is azidolysine, preferably 6-azido-L-lysine.

Naturally, other beneficial parameters can be envisaged when describing polyplexes, which include but are not limited to:

- N/P ratio, which enables calculation of the minimum ratio required to complex 100% of the nucleic acid and which will naturally differ depending on the polymeric scaffold used;
- charge/molar ratio, which is also scaffold-dependent and can be determined by e.g. gel electrophoresis shift;
- hydrodynamic diameter when in a solution, measurable via dynamic light scattering (DLS); and
- size and shape of the complexes in dried/frozen state that can be determined using high-resolution microscopy technique such as TEM, SEM, or AFM.

These and many other polyplex characteristics are variable and dependent on the final polyplex composition, which is known to the skilled person who is capable of determining them by any of the suitable techniques available in the art, if needed.

Most of the known saponins of the 12,13-dehydrooleanane-type that naturally comprise the aldehyde group in position C-23 in their native or unconjugated form are saponins for which the aglycone core structure is either quillaic acid or gypsogenin. An exemplary such saponin is depicted as SAPONIN A and illustrated by the following structure:

(SAPONIN A)

In line with this, it was observed that saponins comprising a quillaic acid aglycone or a gypsogenin aglycone core structure are particularly suitable for the purposes of the present disclosure. Hence, in a further embodiment, a

pharmaceutical composition is provided, wherein the saponin comprises an aglycone core structure selected from quillaic acid and gypsogenin, more preferably wherein the aglycone core structure is quillaic acid.

Saponins can comprise one or more saccharide chains attached to the aglycone core structure. Preferred saponins of the (pharmaceutical) compositions of the disclosure comprise a single chain (i.e. are monodesmosidic) or two chains (i.e. are bidesmosidic) attached to the triterpene 12,13-dehydrooleanane aglycone core structure that comprises an aldehyde group in position C-23.

It was postulated that the sugar chains also play a role in the endosomal-escape-enhancing properties. In line with the above, in a further embodiment, a pharmaceutical composition is provided, wherein the saponin is at least a monodesmosidic saponin comprising at least a first saccharide chain comprising at least three sugar residues in a branched configuration, or is at least a bidesmosidic saponin further comprising a second saccharide chain comprising a glucuronic acid residue, preferably being a terminal glucuronic acid residue.

In a preferred embodiment, the first branched saccharide chain comprises a terminal fucose residue and/or a terminal rhamnose residue and preferably comprises at least four sugar residues, and/or wherein the second saccharide chain is Gal-(1→2)-[Xyl-(1→3)]-GlcA.

Advantageously, the saponin may comprise the first saccharide chain at position C-28 of the saponin's aglycone core structure, and/or the second saccharide chain at position C-3 of the saponin's aglycone core structure.

In a related advantageous embodiment, the first saccharide chain is a carbohydrate substituent at the C-28-OH group of the saponin's aglycone core structure and/or wherein the second saccharide chain is a carbohydrate substituent at the C-3beta-OH group of the saponin's aglycone core structure.

In a related specific embodiment, a composition is provided, wherein the saponin comprises the first saccharide chain at position C-28 of the saponin's aglycone core structure and the second saccharide chain at position C-3 of the saponin's aglycone core structure; preferably wherein the first saccharide chain is a carbohydrate substituent at the C-28-OH group of the saponin's aglycone core structure and/or wherein the second saccharide chain is a carbohydrate substituent at the C-3beta-OH group of the saponin's aglycone core structure.

Particularly preferred saponins include SO1861 and/or GE1741, which are purified from plant roots of either *Saponaria officinalis L.* or *Gypsophila elegans M. BIEB,* respectively. In an embodiment, a pharmaceutical composition is provided, wherein the saponin is any one or more of:

a) saponin selected from any one or more of list A:

- *Quillaja saponaria* saponin mixture, or a saponin isolated from *Quillaja saponaria,* for example Quil-A, QS-17-api, QS-17-xyl, QS-21, QS-21A, QS-21B, QS-7-xyl;
- *Saponinum album* saponin mixture, or a saponin isolated from *Saponinum album;*
- *Saponaria officinalis* saponin mixture, or a saponin isolated from *Saponaria officinalis;* and
- Quillaja bark saponin mixture, or a saponin isolated from Quillaja bark, for example Quil-A, QS-17-api, QS-17-xyl, QS-21, QS-21A, QS-21B, QS-7-xyl; or

b) a saponin comprising a gypsogenin aglycone core structure, selected from list B:
SA1641, gypsoside A, NP-017772, NP-017774, NP-017777, NP-017778, NP-018109, NP-017888, NP-017889, NP-018108, SO1658 and Phytolaccagenin; or

c) a saponin comprising a quillaic acid aglycone core structure, selected from list C:
AG1856, AG1, AG2, Agrostemmoside E, GE1741, Gypsophila saponin 1 (Gyp1), NP-017674, NP-017810, NP-003881, NP-017676, NP-017677, NP-017705, NP-017706, NP-017773, NP-017775, SA1657, Saponarioside B, SO1542, SO1584, SO1674, SO1700, SO1730, SO1772, SO1832, SO1861, SO1862, SO1904, QS-7, QS-7 api, QS-17, QS-18, QS-21 A-apio, QS-21 A-xylo, QS-21 B-apio and QS-21 B-xylo; or

preferably, wherein the saponin is any one or more of a saponin selected from list B or C, more preferably, a saponin selected from list C.

In a particular embodiment, a pharmaceutical composition is provided, wherein the saponin is any one or more of AG1856, GE1741, a saponin isolated from *Quillaja saponaria,* Quil-A, QS-17, QS-21, QS-7, SA1641, a saponin isolated from *Saponaria officinalis,* SO1542, SO1584, SO1658, SO1674, SO1700, SO1730, SO1772, Saponarioside B, SO1832, SO1861, SO1862 and SO1904; preferably wherein the saponin is any one or more of QS-21, SO1832, SO1861, SA1641 and GE1741; more preferably wherein the saponin is QS-21, SO1832 or SO1861; most preferably being SO1861.

In a more particular embodiment, a pharmaceutical composition is provided, wherein the saponin is a saponin isolated from *Saponaria officinalis,* preferably wherein the saponin is any one or more of SO1542, SO1584, SO1658, SO1674, SO1700, SO1730, SO1772, Saponarioside B, SO1832, SO1861, SO1862 and SO1904; more preferably wherein the saponin is any one or more of SO1832, SO1861 and SO1862;even more preferably wherein the saponin is SO1832 and SO1861; most preferably being SO1861.

In further particular embodiments, (pharmaceutical) compositions as disclosed herein can be prepared with the suitable triterpenoid 12,13-dehydrooleanane-type saponins comprising an aldehyde group at position C-23 of the saponin's aglycone core structure, wherein one or more, preferably one of:

i. an aldehyde group in the aglycone core structure of the at least one saponin has been derivatised when present,
ii. a carboxyl group of a glucuronic acid moiety in a second saccharide chain of the at least one saponin has been derivatised when present in the at least one saponin, and
iii. at least one acetoxy (Me(CO)O-) group in a first saccharide chain of the at least one saponin has been derivatised if present.

In more particular embodiments, pharmaceutical compositions can be provided wherein the at least one of the triterpenoid 12,13-dehydrooleanane-type saponins comprising an aldehyde group at position C-23 of the saponin's aglycone core structure further comprises:

i. an aglycone core structure comprising an aldehyde group which has been derivatised by:

- reduction to an alcohol;
- transformation into a hydrazone bond through reaction with N-ε-mateimidocaproic acid hydrazide (EMCH) wherein the maleimide group of the EMCH is optionally derivatised by formation of a thioether bond with mercaptoethanol;
- transformation into a hydrazone bond through reaction with N-[β-maleimidopropionic acid] hydrazide (BMPH) wherein the maleimide group of the BMPH is optionally derivatised by formation of a thioether bond with mercaptoethanol; or
- transformation into a hydrazone bond through reaction with N-[κ-maleimidoundecanoic acid] hydrazide (KMUH) wherein the maleimide group of the KMUH is optionally derivatised by formation of a thioether bond with mercaptoethanol; or

ii. a second saccharide chain comprising a carboxyl group, preferably a carboxyl group of a glucuronic acid moiety, which has been derivatised by transformation into an amide bond through reaction with 2-amino-2-methyl-1,3-propanediol (AMPD) or *N-(2*-aminoethyl)maleimide (AEM); or
iii. a first saccharide chain comprising an acetoxy group (Me(CO)O-) which has been derivatised by transformation into a hydroxyl group (HO-) by deacetylation; or
iv. any combination of two or three derivatisations i., ii. and/or iii., preferably any combination of two derivatisations of i., ii. and iii.

In a specific embodiment, provided are (pharmaceutical) compositions, wherein the at least one saponin comprises a first saccharide chain and a second saccharide chain, wherein the first saccharide chain comprises more than one saccharide moiety and the second saccharide chain comprises more than one saccharide moiety, and wherein the aglycone core structure is quillaic acid or gypsogenin, more preferably is quillaic acid, wherein one, two or three, preferably one or two, of:

i. an aldehyde group in the aglycone core structure has been derivatised,
ii. a carboxyl group of a glucuronic acid moiety in the second saccharide chain has been derivatised, and
iii. at least one acetoxy (Me(CO)O-) group in the first saccharide chain has been derivatised.

An embodiment is the conjugate of the invention, wherein one, two or three, preferably one or two, more preferably one, of:

iv. an aldehyde group in the aglycone core structure of the at least one saponin has been derivatised when present,
v. a carboxyl group of a glucuronic acid moiety in a second saccharide chain of the at least one saponin has been derivatised when present in the at least one saponin, and
at least one acetoxy (Me(CO)O-) group in a first saccharide chain of the at least one saponin has been derivatised if present.

One of the advantages of the compositions as disclosed herein, is the ability to use low concentrations of saponins. Hence, in a further embodiment, a pharmaceutical composition is provided, comprising 0.05 μM - 1 μM of the targeted saponin conjugate, preferably being 0.1 μM - 0.5 μM, more preferably being about 0.3 μM.

In another embodiment, a pharmaceutical composition is provided wherein the nucleic acid is selected from a plasmid, a minicircle, cDNA, mRNA, siRNA, oligonucleotide, and any modified equivalent thereof comprising one or more nucleotide analogues, preferably wherein the nucleic acid is a plasmid, more preferably wherein the nucleic acid comprises at least 0.25 kbp, or at least 0.5 kbp; or at least 0.75 kbp, more preferably at least 1 kbp, or at least 1.5 kbp; or at least 2 kbp, even more preferably at least 2.5 kbp, or at least 3 kbp, or at least 4, kbp, most preferably at least 5 kbp.

In a next embodiment, a pharmaceutical composition is provided, wherein the endocytic receptor is selected from any of the following: epidermal growth factor receptor (EGFR) and receptor tyrosine-protein kinase erbB-2 (Her-2).

In a further embodiment, a pharmaceutical composition is provided, wherein the polyplex further comprises a second targeting ligand recognised by a second endocytic receptor, wherein the first endocytic receptor and the second endocytic receptor are either the same receptor, or are different endocytic receptors present at the same cell.

In another possible embodiment, the presented herein scaffold can further be conjugated with other type of molecules such as dyes, e.g. fluorescent dyes, or quenchers, which can be useful for performing e.g. subcellular localisation studies of polyplexes labelled with scaffolds and/or studies of how such localisation is affected by e.g. the size and nature of the polyplexed nucleic acid (e.g. plasmid of about 2kb versus a plasmid > 5kb vs PMO or siRNA or microRNA) and/or by the presence of different ligands. There exist many fluorescent dyes that can be attached to the scaffold according to any coupling strategy known in the art, so that the scaffold becomes labelled similarly as it is typically done with other molecular biology, for example fluorescently-labelled antibodies. Suitable fluorescent dyes can be dyes belonging to any of the known families like coumarin, rhodamine, cyanine, or xanthene like fluorescein family dyes, or modifications thereof like Alexa Flour dyes. Good results were obtained with cyanine dyes, which can be e.g. of non-sulfonated type including Cy3, Cy3.5, Cy5, Cy5.5, Cy7, and Cy7.5, as well as of sulfonated type such as sulfo-Cy3, sulfo-Cy5, and sulfo-Cy7 etc.

In another embodiment, a pharmaceutical composition is provided, wherein the first targeting ligand, and optionally the second targeting ligand, is an antibody or a binding fragment thereof, preferably is a monoclonal antibody such as Cetuximab or Panitumumab, or is or comprises a peptide or a protein that is recognised by the first endocytic receptor, or optionally the second endocytic receptor.

In an embodiment, a pharmaceutical composition is provided, wherein the first targeting ligand, and optionally the second targeting ligand, is an antibody or a binding fragment thereof, preferably is a monoclonal antibody such as Cetuximab or Panitumumab, or is or comprises a peptide or a protein that is recognised by the first endocytic receptor, or optionally the second endocytic receptor.

In a further preferred embodiment, a pharmaceutical composition is provided comprising a pharmaceutically acceptable excipient and/or pharmaceutically acceptable diluent.

In a possible embodiment, a pharmaceutical composition is provided wherein the polyplex and the targeted saponin conjugate are provided in a mixture or are provided in separate compartments.

In another possible embodiment of the provided herein pharmaceutical composition, the targeted saponin conjugate comprises two or more molecules of the saponin, preferably being between 2-32 molecules of the saponin, even more preferably 4-16 molecules of the saponin, most preferably 4-8 molecules of the saponin.

In a further advantageous embodiment, a pharmaceutical composition is provided, wherein the linker is subject to cleavage under conditions present in endosomes or lysosomes, preferably acidic or enzymatic conditions present in endosomes or lysosomes, possibly wherein the linker comprises a cleavable bond selected from:

- a bond subject to cleavage under acidic conditions such as a hydrazone bond, acetal bond, a dioxalan, and/or an imine bond,
- a bond susceptible to proteolysis, for example amide or peptide bond, preferably subject to proteolysis by Cathepsin B;
- red/ox-cleavable bond such as disulfide bond, or thiol-exchange reaction-susceptible bond such as thio-ether bond.

Advantageously, a pharmaceutical composition is provided, wherein the linker is an acid-sensitive linker that comprises a covalent bond selected from any one or more of: a hydrazone bond, an imine bond, an ester bond, a dioxalane bond, and an oxime bond, preferably wherein the acid-sensitive linker comprises a hydrazone bond.

Preferably, a pharmaceutical composition is provided, wherein the aldehyde group at position C-23 of the saponin's aglycone core structure has been engaged in forming the covalent bond with the linker.

It is preferred that such a cleavable bond is not susceptible, or only to a minor extent, to cleavage when the ligand-saponin conjugate or, if provided, the polyplex comprising the nucleic acid and the second targeting ligand is present outside the endosome and lysosome of the cell, such as outside the cell or in the endocytosed vesicle after the conjugate engaged with an endocytic receptor by binding of the ligand to its target endocytic receptor. For example, the cleavable bond is preferably less susceptible to cleavage when the conjugate is present in the circulation of a human

subject and/or is present extracellularly in an organ of the human subject, compared to the susceptibility for cleavage of the bond when the conjugate is in the endosome or in the lysosome of a target cell.

In a particular embodiment, a (pharmaceutical) composition is provided, wherein the linker is directly or indirectly covalently linked to the ligand.

In a further particular embodiment, a (pharmaceutical) composition is provided, wherein the saponin is or comprises at least one molecule of any of SO1861 or SO1861-EMCH.

In a specific embodiment, a pharmaceutical composition is provided, wherein the nucleic acid is encoding for factor IX or a functional fragment thereof for use in the treatment of haemophilia B; or wherein the first targeting ligand is Cetuximab, and wherein the nucleic acid preferably encodes for a toxin, more preferably dianthin or saporin, for use in the treatment of EGFR positive colon cancer or breast cancer.

In another aspect, a method of preparation of the disclosed pharmaceutical composition is provided, the method comprising the steps of:

- mixing an at least one nucleic acid with a polymeric scaffold to obtain a polyplex;
- combining the polyplex with the targeted saponin conjugate, wherein the conjugate comprises:
    - a saponin being a triterpenoid 12,13-dehydrooleanane-type saponin that in an unreacted state comprises an aldehyde group at position C-23 of the saponin's aglycone core structure, and
    - a first targeting ligand recognised by an endocytic receptor, wherein the saponin is covalently bound to the first targeting ligand by a linker adapted to cleave and release the saponin from said ligand under conditions present in an endosome or a lysosome, preferably being an acid-sensitive linker adapted to cleave and release the saponin from the polymeric scaffold under acidic conditions present in an endosome or a lysosome,

and preferably wherein the polymeric scaffold is or comprises

a. PAMAM, preferably being at least generation 3 PAMAM or higher, and/or preferably being a PAMAM of ethylenediamine core, more preferably wherein the PAMAM comprises at least one pegylated group comprising at least one PEG adapter preferably comprising 5-15 PEG units, and/or preferably wherein the pegylation degree of the PAMAM is comprised between on average 1.5-20 equivalents of PEG adapter per PAMAM core;

or wherein the polymeric scaffold is or comprises

b. a polypeptide comprising between 5 to 25 lysines and at least one cysteine, preferably being exactly one cysteine, more preferably wherein the polypeptide comprises exactly one cysteine, preferably wherein the exactly one cysteine is flanked by a string of 1-7 glycines from each side, preferably 1-5 glycines from each side, more preferably 2-4 glycines, most preferably is flanked by 3-7 glycines from N-terminal side, preferably being 4 glycines; and by 1-4 glycines from C-terminal side, preferably being 4 glycines, and wherein optionally the polypeptide comprises a C-terminal tyrosine, possibly followed by a residue comprising an azide group, preferably being azidolysine, more preferably 6-azido-L-lysine, most preferably wherein the polypeptide is represented by a general formula of: 2-25 Lys ; 3-7 Gly; 1 Cys; 2-4 Gly, and possibly 1 Tyr; preferably wherein the polypeptide is represented by an amino acid sequence given by SEQ ID NO. 1: KKKKKKKKKKKKKKKKGGGGCGGY or SEQ ID NO. 2: KKKKKKKKKKKKKKKKGGGGCGGYK* wherein K* is azidolysine, preferably 6-azido-L-lysine.

In a related aspect use of a polymeric scaffold is provided, wherein the polymeric scaffold comprises or consists of:

a. PAMAM, preferably being at least generation 3 PAMAM or higher, and/or preferably being a PAMAM of ethylenediamine core, more preferably wherein the PAMAM comprises at least one pegylated group comprising at least one PEG adapter preferably comprising 5-15 PEG units, and/or preferably wherein the pegylation degree of the PAMAM is comprised between on average 1.5-20 equivalents of PEG adapter per PAMAM core;
or
b. a polypeptide comprising between 5 to 25 lysines and at least one cysteine, preferably being exactly one cysteine, more preferably wherein the polypeptide comprises exactly one cysteine, preferably wherein the exactly one cysteine is flanked by a string of 1-7 glycines from each side, preferably 1-5 glycines from each side, more preferably 2-4 glycines, most preferably is flanked by 3-7 glycines from N-terminal side, preferably being 4 glycines; and by 1-4 glycines from C-terminal side, preferably being 4 glycines, and wherein optionally the polypeptide comprises a C-terminal tyrosine, most preferably wherein the polypeptide is represented by a general formula of: 2-25 Lys ; 3-7 Gly; 1 Cys; 2-4 Gly, and possibly 1 Tyr, possibly followed by a residue comprising azide group, preferably being azidolysine, more preferably 6-azido-L-lysine; preferably wherein the polypeptide is represented by an amino acid

sequence given by SEQ ID NO. 1: KKKKKKKKKKKKKKKKGGGGCGGY or SEQ ID NO. 2: KKKKKKKKKKKKKKKKGGGGCGGYK* wherein K* is azidolysine, preferably 6-azido-L-lysine,

for preparation of a polyplex, preferably for preparation of a polyplex (by mixing with at least one nucleic acid) to be used with a targeted saponin conjugate wherein the saponin is a triterpenoid 12,13-dehydrooleanane-type saponin that in an unreacted state comprises an aldehyde group at position C-23 of the saponin's aglycone core structure, for transfection of a cell, preferably for preparation of a pharmaceutical composition as disclosed herein.

In a yet further aspect, provided is a kit of parts for delivering a nucleic acid into a cell *in vitro,* the kit comprising:

(a) at least a first container comprising the polymeric scaffold as disclosed here above; and

(b) a targeted saponin conjugate, possibly provided in the first container or in a second container, the targeted saponin conjugate comprising a saponin and a first targeting ligand recognised by an endocytic receptor, wherein the saponin is covalently bound to the first targeting ligand by an acid-sensitive linker adapted to cleave and release the saponin from the first targeting ligand under acidic conditions; and wherein the saponin is a triterpenoid 12,13-dehydrooleanane-type saponin that in an unreacted state comprises an aldehyde group at position C-23 of the saponin's aglycone core structure,
and, optionally

(c) instructions for use, preferably containing instructions for combining of the polymeric scaffold with a nucleic acid for preparation of a polyplex for delivering with the targeted saponin conjugate into a cell.

## EXAMPLES

***Materials:***

SO1861 was isolated and purified by Analyticon Discovery GmbH from raw plant extract obtained from *Saponaria officinalis L.*

Cetuximab was purchased from the pharmacy (Charité, Berlin),
PAMAM (Dendritech, Midland, Michigan),
Peptide scaffolds K16, K16C and K16CPEG were purchased as custom synthesis from GeneCust (Boynes, France),
Saporin-Nanoplasmid (NTC9385R-Sap-BGH pA, 2585 bp) was produced by Nature Technology Corporation, Lincoln, USA,
pEGFP-N3 plasmid (Gene Bank Accession: U57609) was amplificated in Dh5a-cells and isolated using Qiagen® Plasmid Mega Kit (Qiagen, Hilden, Germany),
6-Maleimidocaproic Acid Hydrazide, Trifluoroacetic Acid Salt (EMCH*TFA, >95%, TCI),
Tris(2-carboxyethyl)phosphine hydrochloride (TCEP, 98%, Sigma-Aldrich),
5,5-Dithiobis(2-nitrobenzoic acid) (DTNB, Ellman's reagent, 99%, Sigma-Aldrich),
N-Ethylmaleimide (NEM, 98%, Sigma-Aldrich),
1,4-Dithiothreitol (DTT, 98%, Sigma-Aldrich),
Isopropyl alcohol (IPA, 99.6%, VWR),
Tris(hydroxymethyl)aminomethane (Tris, 99%, Sigma-Aldrich),
Tris(hydroxymethyl)aminomethane hydrochloride (Tris.HCL, Sigma-Aldrich),
Polyethylene glycol sorbitan monolaurate (TWEEN 20, Sigma-Aldrich),
Dulbecco's Phosphate-Buffered Saline (DPBS, Thermo-Fisher),
Ethylenediaminetetraacetic acid disodium salt dihydrate (EDTA-$Na_2$, 99%, Sigma-Aldrich),
sterile filters 0.2 μm and 0.45 μm (Sartorius),
Dimethyl sulfoxide (DMSO, 99%, Sigma-Aldrich),
N-(2-Aminoethyl)maleimide trifluoroacetate salt (AEM, 98%, Sigma-Aldrich),
L-Cysteine (98.5%, Sigma-Aldrich),
deionized water (DI) was freshly taken from Ultrapure Lab Water Systems (MilliQ, Merck).

### Abbreviations

| | |
|---|---|
| DIPEA | N,N-diisopropylethylamine |

| | |
|---|---|
| DLS | Dynamic light scattering |
| DMEM | Dulbecco's modified Eagles medium |
| DMF | N,N-dimethylformamide |
| DTME | Dithiobismaleimidoethane |
| DTT | Dithiothreitol |
| EDCI.HCI | 3-((Ethylimino)methyleneamino)-N,N-dimethylpropan-1-aminium hydrochloride |
| EDTA | Ethylenediaminetetraacetic acid |
| EMCH.TFA | N-(ε-maleimidocaproic acid) hydrazide, trifluoroacetic acid salt |
| FBS | Fetal bovine serum |
| GPC | Gel Permeation Chromatography |
| HATU | 1-[Bis(dimethylamino)methylene]-1H-1,2,3-triazolo[4,5-b]pyridinium 3-oxid hexafluorophosphate |
| IR | Infrared |
| min | Minutes |
| MWCO | Molecular weight cut-off |
| NHS | N-hydroxysuccinimid |
| NMM | 4-Methylmorpholine |
| NMR | Nuclear magnetic resonance |
| PAMAM | Poly(amidoamine) |
| PCR | polymerase chain reaction |
| PDI | Poly dispersity index |
| PEG | Polyethylene glycol |
| PEG4-SPDP | 2-Pyridyldithiol-tetraoxatetradecane-N-hydroxysuccinimide |
| PDT | Pyridine 2-thione |
| SDS | Sodium dodecyl sulfate |
| SEC | Size exclusion chromatography |
| SMCC | Succinimidyl 4-(N-maleimidomethyl)cyclohexane-1-carboxylate |
| TBEU | (Tris-(hydroxymethyl)-aminomethan)-Borat-EDTA-Urea |
| TCEP | Tris(2-carboxyethyl)phosphine hydrochloride |
| Temp | Temperature |
| TFA | Trifluoroacetic acid |
| THPP | Tris(hydroxypropyl)phosphine |
| TNBS | 2,4,6-trinitrobenzene sulfonic acid |
| Tris | Tris(hydroxymethyl)aminomethane |

*Methods:*

**S01861-EMCH**

SO1861 was obtained from *Saponaria officinalis L* (Analyticon Discovery GmbH), and was coupled to respective handles according to methods known in the art, e.g. as described in patent EP3773737 of SAPREME TECHNOLOGIES. Figure 1A schematically shows derivatization of the aldehyde group at position C-23 of the saponin SO1861 aglycone core structure with a maleimide-and-hydrazide (EMCH) crosslinker via an acid-sensitive hydrazone for conjugating e.g. thiols such as cysteines. Figure 1B shows a skeletal structural formula of derivatized SO1861-EMCH with the acid-sensitive hydrazone bond at C-23 of the saponin SO1861 aglycone core structure, which bond cleaves under acidic conditions resulting in recreation of the aldehyde group at position C-23, which participates in endosomal escape enhancing properties of SO1861 and other similar saponins as listed in Table 1. Figure 1B further shows two different schematic representations of the skeletal structural formula as interchangeably used throughout the present disclosure.

**Conjugation of S01861 to antibodies and protein/peptide ligands**

SO1861-EMCH was conjugated to a TCEP-treated monoclonal antibody (mAb), such as Cetuximab or Panitumumab (Figure 2A, the mAb being represented by a schematic IgG structure, via cysteine residues, to produce monoclonal antibody-targeted SO1861 conjugates (further mAb-SO1861) with an average DAR value of about 4 (Figure 2B, marked as IgG-SO1861).

Similarly, peptide or protein ligands (for example, EGF or transferrin) can be conjugated to SO1861-hydrazone-NHS via lysines to produce other ligand-SO1861 constructs (Figure 2C).

**Synthesis of pegylated PAMAM scaffolds including PAMAM-PEG-$N_3$**

As a proof-of-principle model system, generation 5 of PAMAM dendrimers with ethylenediamine core were chosen for preparation of dendrimer-based scaffolds and for investigation of the impact of the scaffold modifications on crucial properties like polyplex solubility and DNA binding. PAMAM generation 5 (Dendritech, Midland, Michigan) was purchased lyophilized. After establishing the modification synthesis strategy (shown in Figure 3A and in more detail in 3B), libraries of PAMAM scaffolds were prepared and included modified scaffolds with varying modifications such as dye conjugation (not shown) and different numbers of PEG-based click adapters for conjugation of various targeting ligands like antibodies. Forthe introduction of the mentioned modifications, the amines of PAMAM were utilized. PAMAM G5 contains 128 amine groups. However, as these positively charged groups provide the cationic surface charge for polyplex formation through their ionic interactions with the negatively charged phosphate backbones of the nucleic acids, it was decided to modify only a proportion of them. Consequently, only a limited number of the amines were subjected to pegylation in order to guarantee that a sufficient positive charge remains for effective polyplexation of the modified PAMAM with nucleic acid such as a plasmid. To do so, the modifications of amine groups were controlled stoichiometrically. As the first modification of PAMAM it was decided to introduce binding sites for conjugation of a targeting ligand. To do so, a heterobifunctional polyethylene glycol (PEG)-spacer was employed that contains an active N-hydroxysuccinimide (NHS) ester on one side and an azide functionality on the other side. Then, optimization testing was performed to investigate PEG-spacers varying in length of 6 or 12 PEG units, and it was decided also to investigate the properties of scaffolds containing different numbers of spacers. The length of the PEG-spacer was selected to ensure the accessibility for the ligand in order to ensure efficient conjugation thereof to the modified PEG scaffold. In a typical reaction, PAMAM G5 (20 mg, 1.0 equiv.) was dissolved in DMSO (1.0 mL) and NHS-$PEG_{12}$-$N_3$ (2.57 mg, 5.0 equiv.) dissolved in DMSO (0.26 mL) was added. The reaction was shaken (800 rpm) at room temperature for 2 h. Subsequently, the reaction was diluted with water (1:8 v/v) and placed in a dialysis membrane (12 kDa MWCO, Roth). The diluted reaction mixture was dialyzed against water for 72 h to remove DMSO, N-hydroxysuccinimide (NHS) and unreacted PEG linker. During this time, dialysis medium was changed 6 times. The dialysis fraction was lyophilized, and the product was obtained as a colorless solid (19.9 mg, 90% yield). The product was characterized using infrared (IR) spectroscopy and $^1$H NMR. Different dendrimer-based scaffolds were characterised by standard laboratory methods for the pegylation degree, size, stability, purity, and polydispersity.

**Polyplex formation between PAMAM-PEG-$N_3$ and plasmid DNA**

PAMAM conjugates (non-pegylated PAMAM G5, and PAMAM-(PEG-$N_3$)$_x$ including different degrees of pegylation) were dissolved in HEPES (10 mM, pH 7.1) at 5 mg/mL one day before polyplex formation. The plasmid DNA was diluted to 0.2 $\mu$g/$\mu$L with HEPES buffer and PAMAM solutions were diluted in HEPES (10 mM HEPES, pH 7.1) to the calculated concentration which depended on the desired N/P ratio. For the N/P ratio, primary amines of PAMAM and the positive ion introduced by 2-iminothiolane were considered. For 20 $\mu$L of polyplexes, 12.5 $\mu$L PAMAM solutions in HEPES buffer were added to 7.5 $\mu$L (1.5 $\mu$g) DNA solution and mixed thoroughly by pipetting for 5 seconds, followed by brief vortexing and subsequent centrifugation. The polyplexes were allowed to incubate for at least 30 min at room temperature before being used in subsequent experiments. Polyplexes were characterized by gel retardation (DNA binding, e.g. Figure 6F), dynamic light scattering (hydrodynamic diameter), laser Doppler electrophoresis (LDE, for zeta potential), and transmission electron microscopy (TEM) (data no shown). When testing the polyplexes, it was observed that the pegylation of PAMAM improved their stability. Interestingly, we also observed that the pegylation of the PAMAM mitigated toxicity of the formulated therewith polyplexes.

**Conjugation of PAMAM scaffolds with ligands**

As the next modification, the pegylated and equipped-with-a-click-adapter PAMAM scaffold was conjugated with a ligand conjugated with a second click adapter, compatible with the one equipped as part of the modified PAMAM. Many of such compatible click adapters are known in the art and suitable for performing click-chemistry reactions, such as, to name a few, azide-alkyne Huisgen cycloaddition, strain-promoted azide-alkyne cycloaddition (SPAAC), or the inverse electron-demand Diels-Alder (IEDDA) reaction. The conjugation with a second click adapter equipped targeting ligand (for example being an antibody, a peptide, a protein, a carbohydrate, a vitamin) can be done before the polyplex formation, or because of the high thermodynamic driving force and specificities of click-chemical syntheses, it can even be performed on an already formed polyplex as shown in Figure 4. The latter mode of conjugation has the additional advantage of ensuring that the targeting ligand is presented on the surface of the polyplex. According to this procedure Cetuximab-targeted polyplex was successfully prepared as follows. First, the PAMAM conjugate (PAMAM G5, or PAMAM-(PEG-$N_3$)$_x$) was polyplexed with the desired plasmid as described in the "Polyplex formation" section above. For a polyplex of PAMAM-(PEG-$N_3$)$_6$ at N/P 8 and volume of 100 $\mu$L (1.1 nmol, 42.2 $\mu$g PAMAM conjugate), a solution

of Cy5-cetuximab-PEG-DBCO in 10 mM PBS pH 7.4 (54.1 μL, 0.3 mg/mL, 0.011 nmol, equivalent to 10 mol% of PAMAM conjugate i.e., 0.1 equivalents relative to PAMAM conjugate) was added to the polyplex solution and SPAAC bio-conjugation was allowed to proceed for 48 h at room temperature under shaking (800 rpm). The progress of the reaction was followed by quantifying the fraction of unreacted Cy5-cetuximab-DBCO using SDS-PAGE electrophoresis (10%, 200 V, 90 min) by comparison with a standard calibration curve made with solutions of Cy5-cetuximab-DBCO (100, 50, 25, 12.5%) of known concentration prepared under identical conditions. Gel quantification was done by using a Versadoc imaging system equipped with a Cy5 filter (data not shown). The functionalized polyplex was used without further purification.

**Generation of peptide scaffolds and pegylated peptide scaffolds**

As a second proof-of-principle model system, following a triblock copolymer strategy, several peptide scaffolds containing a poly-lysine strip were designed and tested, with or without modifications. In the initial designs, the peptides included several cysteines for the introduction of free thiol groups into the peptide scaffold for conjugation purposes, for example by means of thiol-maleimide coupling chemistry. However, due to aggregate formation, possibly due to interpeptide disulfide formation, the number of cysteines was reduced to only one. Further modifications of the initial peptide sequence included the introduction of several glycines directly preceding and directly following the cysteine to avoid or reduce steric hindrances, and the incorporation of tyrosine to facilitate the detection and quantitation using ultraviolet-visible (UV/Vis) spectrophotometry. Lastly, a terminal azide group was introduced in a form of an azidolysine (6-azido-L-lysine) for conjugation purposes with potential targeting ligands. For certain types of ligands that could suffer of steric hindrance, a PEG-spacer was introduced just before the azidolysine. Satisfactory results were observed with a PEG8-spacer. The two optimized designs of peptide scaffolds ($K_{16}G_4CG_2Y\text{-}PEG_8\text{-}K(N_3)\text{-}NH_2$ and $H\text{-}K_{16}G_4CG_2YK(N_3)\text{-}NH_2$) are schematically shown in Figure 5 (Sequences are given from N-terminus to C-terminus with the latter being amidated to increase the stability of the peptide scaffold).

***Analytical and Preparative methods***

**Spectrophotometry:** Concentrations were determined using either a Thermo Nanodrop 2000 spectrometer or Perkin Elmer Lambda 365 Spectrophotometer.

**DLS measurements:** DLS measurements were performed on a Malvern Nano ZS (Malvern Instruments, U.K.), operating at 633 nm with a 173° scattering angle, at 25 or 37 °C. DLS mean diameters were obtained from the intensity particle size distribution provided by Malvern Zetasizer Software. DLS histograms were obtained from the intensity particle size distributions.

**Infrared Spectroscopy:** FT-IR spectra were recorded on a Bruker Vertex 70v, equipped with a RockSolid interferometer, after preparing the samples (1-2 mg) as KBr pellets. Spectra were processed using OPUS 7.8 software (Bruker).

**NMR Spectroscopy:** $^1H$ NMR spectra were recorded on a 11.7 T Bruker DRX-500 spectrometer. Chemical shifts are reported in ppm ($\delta$ units) downfield from the HOD residual solvent peak ($CD_3OD$). Experiments were recorded acquiring 64-512 scans, with a pre-scan delay (d1) of 12 s, and an acquisition time (aq) of 4 s at 300 K. $^{13}C$ NMR spectrum was recorder on a 9.39 T Varian Innova 400 spectrometer. MestReNova 14.2 software (Mestrelab Research) was used for spectra processing.

**Cell culture (cancer cell lines) conditions and treatments:** Human cell lines (HEK293FT, A431, and A2058) were maintained in 10% FBS-supplemented DMEM medium (PAN-Biotech GmbH) + Pen/Strep at 37°C with 5% $CO_2$. Targeted Cetuximab-SO1861 was co-administrated onto different cell lines *in vitro,* at a concentration of 73 nM conjugate (300 nM SO1861), and different concentrations of polyplex (as indicated); made with a polymeric scaffold (comprising of PAMAM or K16-polypeptide) and a DNA plasmid. SO1861-EMCH was co-administrated onto different cell lines *in vitro,* at a concentration of 2000 or 4000 nM (as indicated), and different concentrations of polyplex (as indicated); made with a polymeric scaffold (comprising of PAMAM or K16-polypeptide) and a DNA plasmid.

**Cell viability assay:** Cells were seeded in a 96 well plate at 5.000-10.000 c/w in 100 μL/well and incubated overnight at 37°C. Before treatment, the mAb-SO1861 and polyplexes were diluted separately in DPBS (PAN-Biotech GmbH) to 10x the required concentration. The cells received 80 μL/well fresh media, followed by 20 μL/well polyplex solution and 20 μL/well saponin solution. Control transfections were performed with Lipofectamine™ 2000 Transfection Reagent (Thermo Fisher Scientific) according to the manufacturer's instruction, using 160 pM plasmid and with a media change 4-5 hrs after transfection. After treatment, the cells were incubated for 72 hr at 37°C before the cell viability was determined by a MTS-assay performed according to the manufacturer's instruction (CellTiter 96® AQueous One Solution Cell Proliferation Assay, Promega). Briefly, the MTS solution was diluted 20x in DMEM without phenol red (PAN-Biotech GmbH) supplemented with 10% FBS (PAN-Biotech GmbH). Media was removed, after which 100 μL diluted MTS solution was added per well. The plate was incubated for approximately 20-30 minutes at 37°C. Subsequently, the optical density

at 492 nm was measured according to instructions, using a SpectraMax ID5 plate reader (Molecular Devices). For quantification, the background signal of 'medium only' wells was subtracted from all other wells before the ratio of untreated/treated cells was calculated by dividing the background corrected signal of untreated wells over the background corrected signal of the treated wells.

***Results and Discussion***

**Example 1. Characterisation of PAMAM-based scaffolds**

The dendrimer-based scaffolds were characterised with respect to the degrees of their substitution, size, stability, purity, polydispersity, and structural analysis. Pegylation and thiolation were determined by $^1$H nuclear magnetic resonance (NMR). As an example, analysis of a PAMAM G5-(PEG-$N_3$)$_5$ by $^1$H NMR revealed the incorporation of the $PEG_{12}$ linkers associated to a new multiplet at 3.80-3.64 ppm, characteristic of the ethylene glycol monomers, not present in the spectrum of commercial PAMAM (Figure 6A and B). A pegylation degree of 5 for this sample was determined by integration of these characteristic multiplet signals at 3.80-3.64 ppm, accounting for 48 of the 52 protons in the $PEG_{12}$ linker, relative to that of the multiplet at 2.71-2.59 ppm due to the 252 internal protons present in G5 PAMAM. The presence of the $PEG_{12}$ linker was also confirmed by $^{13}$C NMR that showed the expected characteristic signals of the $PEG_{12}$-$N_3$ groups (Figure 6C).

Due to the characteristic, intense signal of the azide functional group by IR spectroscopy, IR spectra for PAMAM G5 dendrimers after functionalization with PEG-$N_3$ linkers were recorded. Figure 6D shows the IR spectra of a commercial PAMAM and three pegylated derivatives with pegylation degrees of 3.2, 6.2, and 12.4 (as indicated). The presence of a new band at ca. 2100 $cm^{-1}$ reveals the azide incorporation in the pegylated dendrimers. In addition, a new band is observed at ca. 1100 $cm^{-1}$ corresponding to the ether functional group indicative of the presence of PEG. Notably, both bands increase their intensity as the degree of pegylation increases, confirming the efficient incorporation of PEG-$N_3$.

The hydrodynamic diameter of PAMAM G5-(PEG-$N_3$) with pegylation degrees between 3.2 and 12.4 was determined by DLS as shown in Figure 6E. Namely, sizes calculated in these histograms using volume distributions revealed a range of 8.1-8.8 nm, confirming no substantial difference in size within the range of pegylation degree. Table 2 shows sizes and PDI by DLS of polyplexes prepared from equipped PAMAM G5 with increasing degree of pegylation.

***Table 2.***

| dissolved in PBS (5 mg/mL) | + HEPES to N/P 8 | | + NaCl | | |
|---|---|---|---|---|---|
| $PEG_{12}$-$N_3$ per PAMAM by $^1$H NMR | $D_H$ [nm] | PDI | $D_H$ [nm] | PDI | delta with NaCl |
| 4.6 | 152.3 | 0.201 | 204.0 | 0.190 | 51.7 |
| 9.2 | 170.7 | 0.218 | 264.0 | 0.210 | 93.3 |
| 20.6 | 218.0 | 0.242 | 243.3 | 0.229 | 25.3 |
| 35.2 | 170.0 | 0.311 | 189.0 | 0.188 | 19.0 |

For the untargeted dendrimer system, it was found that all pegylated PAMAMs were able to efficiently bind DNA at amine to phosphate ratios (N/P) equal or higher to four. To determine the N/P ratios, the PAMAM scaffold libraries equipped with increasing amount of click adapter were tested for their capacity to complex the effector gene and form stable polyplexes. The capability to complex plasmid DNA was studied using pUC19 plasmid via gel retardation assays with SYBR™ sage DNA gel stain to visualize double-stranded DNA. All PAMAM conjugates were able to efficiently complex the effector plasmid at N/P ratios of four or higher. Even, highly PEGylated PAMAMs with 80 eq. of $PEG_{12}$-linker per PAMAM core were able to complex the DNA completely (Figure 6F).

Regarding polyplex stability, it was confirmed that polyplexes of unmodified PAMAM tend to aggregate at higher ionic strength. However, $PEG_{12}$ linkers (5-40 equivalents per PAMAM) seemed to stabilize the polyplexes at a physiological salt concentration, whereas $PEG_6$ linkers were only able to stabilize the polyplex at the highest degree of pegylation (40 equivalents per PAMAM). Therefore, PAMAM scaffolds modified with $PEG_{12}$-linkers were used for transfection experiments. Here, unmodified PAMAM and pegylated PAMAM showed significant increase in transfection efficiency when cell medium was supplemented with SO1861 (up to 20-fold increase). With increasing degree of pegylation, this effect was reduced indicating that higher pegylation advantageously impaired non-specific internalization of polyplexes. Importantly, lower toxicity was also observed for systems with higher degree of pegylation.

However, PAMAM scaffolds with degrees of pegylation higher than 20 exhibited poorer transfection efficiencies and the enhancer effect of endosomal escape-enhancing saponin like SO1861 was weak or absent. Consequently, pegylation degrees being on average about 3-13 equivalents of PEG adapter per PAMAM core were evaluated as

advantageous.

**Example 2. Characterisation of peptide-based scaffolds**

Based on *in vitro* polyplex formation experiments, it is believed that the sequence of the peptide scaffold for polyplexation and ligand conjugation was optimized. In summary, the core structure of the peptide scaffold is a linear polypeptide containing a polylysine tail, a single cysteine surrounded by several glycines followed by a tyrosine, an optional PEGs-spacer, and, optionally, a terminal azido-lysine (i.e. a lysine carrying an azide group) for conjugation purposes using click-chemistry, for example of targeting ligands.

The two peptide scaffolds (differing in presence of a PEGs-spacer) were tested for their capability to complex pEGFP-$N_3$ as model plasmid, as compared to unmodified $K_{16}$. Different N/P ratios were used for the complexation of a constant amount of DNA in 10 mM HEPES, pH 7.1. The proportion of free DNA was quantified using QuantiFluor® dsDNA Dye System (Promega) with a fluorescent DNA-binding dye that allows sensitive quantification of small amounts of double-stranded DNA in solution. The results are shown in Figure 7 demonstrating that the modified scaffolds have a DNA complexation efficiency comparable to $K_{16}$ and complexed ≥ 98% of the DNA at an N/P ratio of 10.

**Example 3. Transfection of polyplexes made with pegylated PAMAM with either Cetuximab-targeted S01861 or non-targeted S01861**

To focus on target selectivity and the potential of delivering DNA effector plasmids efficiently into the cytosol of only the target cells, a comparison was made between polyplex transfection efficiencies achieved in the presence of either a targeted-saponin conjugate or a non-targeted saponin.

In the first step, a saporin-encoding nanoplasmid (npSaporin) polyplexed PAMAM-$(PEG\text{-}N_3)_{6.2}$ was co-administrated with either 73 nM Cetuximab-SO1861 (4 saponin SO1861 moieties linked per antibody, thus with DAR4 and hence corresponding to ~300 nM = 0.3 μM of SO1861) or 4 μM SO1861-EMCH, to A431 and A2058 cell lines differing with respect to EGFR expression. The results are shown in Figure 8. In line with the initial expectation, it was observed that co-administration of PAMAM G5$(PEG\text{-}N_3)_{6.2}$ npSaporin polyplexes with 4 μM SO1861-EMCH strongly enhanced the cytoplasmic delivery of npSaporin and, consequently, also saporin protein expression that results in cell killing in both A431 and A2058 cells (Figure 8A and B). Importantly, however, when PAMAM G5$(PEG\text{-}N_3)_{6.2}$ npSaporin polyplexes were co-administrated with Cetuximab-SO1861, a very strong enhancement in cytoplasmic delivery of the npSaporin was observed only in the EGFR+ cell line A431 (Figure 8A), and not in the EGFR- cell line A2058 (Figure 8B), leading to saporin protein expression and increased cell killing only in the cells expressing the endocytic receptor specifically recognized by Cetuximab. No significant differences in cell viability were observed with control transfections using polyplexed pEGFP-N3 in both cell lines (Figure 8C and D).

This data provides experimental evidence for efficient, enhanced and EGFR selective targeted delivery of the npSaporin plasmid when using Cetuximab-SO1861 conjugates, thus showing that an efficient targeted non-viral delivery of a plasmid DNA into cancer cells is possible to achieve with a 2-component system. Consequently, the data demonstrates that a targeted two-component system constitutes a very promising new way of delivering large nucleic acids in a target-cell-selective manner.

**Example 4. Transfection of polyplexes made with either pegylated or un-pegylated PAMAM with Cetuximab-targeted S01861**

Having confirmed target-selectivity and suitability of the 2-component system made of a PAMAM-based polyplex and a Cetuximab-targeted-saponin conjugate, another set of transfection experiments was performed in the same two cell lines (EGFR+ A431 cells and EGFR- A2058 cells) to investigate if the degree of PAMAM G5 pegylation can affect the transfection efficiency.

Similar to Example 3, this set-up allowed to study the targeted delivery of the DNA plasmid on EGFR positive and negative cell lines. The transfections were performed with non-pegylated PAMAM G5 scaffold versus pegylated PAMAM G5$(PEG\text{-}N_3)_6$ scaffold, whereby both of the PAMAM scaffolds were polyplexed with either npSaporin nanoplasmid or with pEGFP as a control DNA, +/- Cetuximab-SO1861 (DAR4). The results are shown in Figure 9.

The results show that only in the presence of Cetuximab-targeted-saponin conjugates, saporin-encoding polyplexes made with non-pegylated PAMAM G5 scaffolds and the pegylated PAMAM G5$(PEG\text{-}N_3)_6$ scaffold alike, can be delivered to EGFR+ cells (Figure 9A) but not to EGFR- cells (Figure 9B), indicating target specific delivery of these polyplexes. In the absence of Cetuximab-targeted saponin, npSaporin nanoplasmid polyplexed with PAMAM G5 or PAMAM G5$(PEG\text{-}N_3)_6$ did not cause any substantial effects on cell viability regardless of EGFR expression status (Figure 9A-B), which shows that the polyplexed npSaporin nanoplasmid by itself cannot be delivered efficiently to the cytoplasm. As expected, no substantial effects on viability were observed for polyplexes made with the pEGFP control plasmid

(Figure 9C-D). Only at 1000 pM of pEGFP, some cytotoxicity was observed in A2058 cells when treated with the non-pegylated PAMAM G5 polyplexed with pEGFP nanoplasmid +/- Cetuximab-SO1861 (DAR4) (Figure 9D). This indicates that non-pegylated PAMAM G5 causes a degree of transfection-mediated toxicity at 1000 pM, while pegylated PAMAM G5 is still tolerated.

In sum, the results show that efficient cytoplasmic delivery of the polyplexed npSaporin plasmid was only possible in EGFR expressing cells with co-administration of Cetuximab-SO1861 (DAR4). This effect appears to be dependent on the EGFR-targeted co-delivery of SO1861 that enhances the endosomal escape of the saporin-encoding plasmid, leading to cell killing from 10 pM of plasmid concentration on (Figure 9A). In the absence of EGFR expression (A2058 cells Figure 9B), necessary for Cetuximab-SO1861 binding, this cell killing was not observed. Neither was it observed for the negative control pEGFP plasmid at similar concentrations (10-100 pM polyplexed plasmid), regardless of the EGFR-expression status.

**Example 5. Transfection of polyplexes made with peptide poly-lysine scaffold (K16C) with either Cetuximab-targeted S01861 or non-targeted S01861**

Next, transfection experiments in EGFR+ A431 cells were performed with the peptide poly-lysine scaffold (K16C) polyplexed with saporin-encoding nanoplasmid npSaporin. The results of this treatment on cell viability are shown in Figure 10, which indicate that K16C peptide scaffold polyplexed with saporin nanoplasmid DNA shows practically no enhanced cell killing activity up to about 1000 pM of plasmid in the absence of free saponin or targeted saponin. Notably, due to the target cell specificity of Cetuximab conjugates saponin, the amount of saporin nanoplasmid necessary per treatment is greatly reduced.

**Example 6. Stability of PAMAM and peptide polyplexes in blood**

Sufficient stability in blood is required for the polyplexes to sustain prolonged circulation in the blood following systemic administration into the patient's body. To measure the stability of the described herein polyplexes when exposed to human blood, an assay was designed as shown in Figure 11A. The assay starts with incubation of the polyplexes in blood serum for 1 h at 37 °C. Afterwards, intermediate steps are performed for the sample purification and polyplex isolation. A heat shock procedure was used for blood protein precipitation, followed by a centrifugation to separate the solid and the liquid part of the sample. The supernatant was then mixed with heparin to release the plasmid from the polyplex. In a final step, PCR and gel electrophoresis were used for qualitative assessments and product size control (Figure 11B). The results show that both the PAMAM- or peptide- based polyplexes were stable in human blood serum at body temperature for at least 1hr.

**Example 7. Toxicity tests of PAMAM and peptide scaffolds or polyplexes in blood**

To get a first estimation of the haemolytic activity of the scaffolds and its potential to induce aggregation of red blood cells, possible disruption of red blood cells was investigated by measuring the release of haemoglobin into the supernatant according to the scheme shown in Figure 12A.

First, the red blood cells (RBCs) were treated with solutions containing PAMAM- or peptide-based scaffolds or polyplexes obtained therewith at different concentrations (2.5-80 μg/mL). The incubation was performed for 3 h at 37°C. RBCs treated with PBS under same conditions were used as a negative control while samples treated with Triton X-100 (10%) were used as a positive control corresponding to conditions of full haemolysis. After centrifugation, the absorbance at 540 nm ($OD_{540}$) of the supernatants was recorded with a spectrometer. The following equation was used for the calculation of the percentages of haemolysis:

$$Hemolysis\ (\%) = \left(\frac{OD(sample) - OD(0)}{OD(100) - OD(0)}\right) \times\ 100\%$$

The results of haemolytic activity of two different scaffolds (PAMAM G5 and peptide $K_{16}C$), and polyplexes obtained therewith are shown in Figure 12B and indicate a surprisingly low haemolysis degree for all cases.

As a next step, the erythrocyte aggregation propensity of the scaffolds was examined under an optical microscope to visualize possible interactions of the scaffolds and polyplexes with RBCs and their surface (Figure 12C).

The results show that polyplexes of K16C or PAMAM and the scaffold molecule K16C did not cause any RBC aggregation. Only a mild degree of irregular transformation of the cell surfaces was observed and only at the highest concentrations tested. In contrast, under this experiment set-up, the scaffold molecule PAMAM caused massive blood clotting reaction at higher concentrations, likely due to the interaction of its positively charged surface with the negatively

charged cell membranes.

Next, the impact on haemostasis was investigated. It is known that to stop bleeding from blood vessels, three major steps are involved in haemostasis. These are vascular spasm, the platelet plug formation, and coagulation. It is widely accepted that two pathways are involved in clot formation in mammals; being the intrinsic and the extrinsic pathway. The activated partial thromboplastin time (aPTT) is a parameter used to detect disruptions in the intrinsic pathway and the prothrombin time (PT) reveals problems in the extrinsic part of the coagulation cascade. Consequently, to investigate the impact of the PAMAM- and peptide-based scaffolds on the patient's blood coagulation, these two parameters were measured in fresh whole blood after incubation of nanoparticles at different concentrations (0 nM-5 μM). The results for PAMAM or peptide K16C scaffolds or polyplexes made with them (as indicated) are shown in Table 3 containing partial thromboplastin time (PTT) values (the reference is 26-40 s), and in Table 4 containing prothrombin time (PT) values (the reference for PT is 70-130%). In both of the Tables 3 and 4, the values within the reference range are highlighted in grey, while the values in the remaining non-highlighted boxes are prolonged or not detectable. The extended coagulation times in Table 4 can likely be explained by electrostatic interactions between the positively charged scaffold molecules and the negatively charged coagulation factor proteins, which are responsible for the clot formation at the end of the common pathway of the cascade. Pegylated PAMAM still remains to be investigated, but the initial results appear promising and indicate that systemic delivery of polyplexes using the presented herein scaffolds will likely not only be sufficiently stable in the blood but also well tolerated by the patient.

*Table 3.*

| Nanomaterials \ Concentration | 0 nM | 0.1 μM | 0.5 μM | 1 μM | 5 μM |
|---|---|---|---|---|---|
| PAMAM G5 | 32.48 s | 34.15 s | 60.85 s | - | - |
| PAMAM G5-polyplexes | 31.5 s | 38 s | - | - | - |
| K16C | 31 s | 38.5 | - | - | - |
| K16C-polyplexes | 31.6 s | 30.15 | - | - | - |

*Table 4.*

| Nanomaterials \ Concentration | 0 nM | 0.1 μM | 0.5 μM | 1 μM | 5 μM |
|---|---|---|---|---|---|
| PAMAM G5 | 100 % | 62 % | 29 % | 29 % | 0 % |
| PAMAM G5-polyplexes | 100 % | 71 % | 31.5 % | 27 % | 0 % |
| K16C | 100 % | 100 % | 82 % | 61 % | 33.5 % |
| K16C-polyplexes | 100 % | 62 % | 44 % | 41.5 % | 27 % |

| | |
|---|---|
| 100 % | - 13.9 s |
| 50 % | - 18.5 s |
| 25 % | - 29.5 s |
| 10% | - 65.8 s |

**Example 8 The in vivo toxicity assessment**

The *in vivo* toxicity of the prototypes was investigated in mice by determining the non-observed adverse effect level (NOAEL) for intravenous administration (data not shown). Severe side effects were not observed, neither for the highest dose (45 μg of plasmid) of the dendrimer-based prototype nor for the peptide-based prototype (15 μg). Only a locally limited inflammation was observed at the injection site. For performing histological analyses of mouse organs

after treatments, mice were treated eight times with 5 μg nanoplasmid DNA encoding for saporin, which was either polyplexed by a peptide-based scaffold or a dendrimer-based scaffold. Spleens and livers were observed. The while pulpa of the spleens was activated in both cases. The liver of the animal that was treated with the peptide-based prototype showed single cell necrosis, while in the liver of the animal treated with the dendrimer-based scaffold, necrotic cell groups were observed. The results indicate only very low systemic intoxication after intravenous administration.

**Conclusions**

The co-administration experiments show marked increase in selectivity of the treatment and also indicate that the targeted saponin conjugates are less toxic than free saponin which needs to be used at about 10-fold higher amounts as compared to its targeted form to achieve transfection efficiency. This particular advantage suggests that co-administration of polyplexes with targeted saponin conjugates could be a suitable candidate for conducting gene therapy ex *vivo* or *in vivo* using systemic administration. The fact the efficient delivery and expression was achieved with plasmids also indicates that the presented herein technology can likely be translated to other effector genes such as genome editing cassettes, for which a transient expression in the specific cells is highly desirable. The latter is further supported by promising stability results and low haemolytic activity of the polyplexes as investigated in human blood.

**LITERATURE**

Agarwal, S., et al., 2012. PDMAEMA based gene delivery materials. Mater. Today 15 (9), 388-393.

Berkhout B, Liu YP. Towards improved shRNA and miRNA reagents as inhibitors of HIV-1 replication. Future Microbiol 9:561-571 (2014)

Böttger S - PhD thesis (2013): Untersuchungen zur synergistischen Zytotoxizität zwischen Saponinen und Ribosomen inaktivierenden Proteinen Typ I,

Clifford, A., et al., 2019. Biomimetic modification of poly-L-lysine and electrodeposition of nanocomposite coatings for orthopaedic applications. Colloids Surf. B 176, 115-121.

Clochard J et al, A new acetylated triterpene saponin from Agrostemma githago L. modulates gene delivery efficiently and shows a high cellular tolerance, International Journal of Pharmaceutics, Volume 589, 15 November 2020, 119822.

Démoulins, T., et al., 2016. Polyethylenimine-based polyplex delivery of self-replicating RNA vaccines. Nanomedicine 12 (3), 711-722.

Dufes C, et al. (2005) Adv Drug Deliv Rev 57:2177. DOI: 10.1016/j.addr.2005.09.017.

Fleck et al. (2019) in Table 1, row no. 28 [Juliane Deise Fleck, Andresa Heemann Betti, Francini Pereira da Silva, Eduardo Artur Troian, Cristina Olivaro, Fernando Ferreira and Simone Gasparin Verza, Saponins from Quillaja saponaria and Quillaja brasiliensis: Particular Chemical Characteristics and Biological Activities, Molecules 2019, 24, 171; doi:10.3390/molecules24010171]

Fuchs H, et al. Glycosylated Triterpenoids as Endosomal Escape Enhancers in Targeted Tumor Therapies. Biomedicines 5(2017)

George LA, et al. Hemophilia B Gene Therapy with a High-Specific-Activity Factor IX Variant. N Engl J Med 377:2215-2227 (2017)

Gilleron J, et al. Image based analysis of lipid nanoparticle-mediated siRNA delivery, intracellular trafficking and endosomal escape. Nat Biotechnol 31:638-646 (2013)

He, Y., et al., 2013. Polyethyleneimine/DNA polyplexes with reduction-sensitive hyaluronic acid derivatives shielding for targeted gene delivery. Biomaterials 34 (4), 1235-1245.

Hess, K.L., et al., 2017. Polyplexes assembled from self-peptides and regulatory nucleic acids blunt toll-like receptor signaling to combat autoimmunity. Biomaterials 118, 51-62.

Holliger, P, et al. (1993) Proc. Natl. Acad. Sci. USA 90:6444-6448;

Jia et al., Major Triterpenoid Saponins from Saponaria officinalis, J. Nat. Prod. 1998, 61, 11, 1368-1373, Publication Date: September 19, 1998, https://doi.org/10.1021/np980167u

Kabanov, A.V., Kabanov, V.A., 1995. DNA complexes with polycations for the delivery of genetic material into cells. Bioconjug Chem 6 (1), 7-20.

Kabat, E.A., Wu, T.T., Perry, H.M., Gottesman, K.S. and Foeller, C. (1991) Sequences of Proteins of Immunological Interest. NIH publication No. 91-3242, Bethesda.

Kipriyanov, S. M., et al. (1994) Mol. Immunol. 31:1047-1058

Kipriyanov, S. M., et al. (1995) Human Antibodies and Hybridomas 6:93-101

Kochetkov N.K.; Khorlin A.J.; Ovodov J.S (1963). The structure of gypsoside - triterpenic saponin from gypsophila pacifica kom.. , 4(8), 477-482. doi:10.1016/s0040-4039(01)90658-6

Kodama, Y., et al., 2014. Biodegradable nanoparticles composed of dendrigraft poly-Llysine for gene delivery. Eur. J. Pharm. Biopharmaceutics 87 (3), 472-479.

Koping-Hoggard, M., et al., 2004. Improved chitosan-mediated gene delivery based on easily dissociated chitosan polyplexes of highly defined chitosan oligomers. Gene Ther. 11 (19), 1441-1452.

Kozielski, K.L., Rui, Y., Green, J.J., 2016. Non-viral nucleic acid containing nanoparticles as cancer therapeutics. Expert Opin Drug Deliv 13 (10), 1475-1487.

Kwoh, D.Y., et al., 1999. Stabilization of poly-L-lysine/DNA polyplexes for in vivo gene delivery to the liver. Biochim. Biophys. Acta 1444 (2), 171-190.

Lee CS, et al. Adenovirus-Mediated Gene Delivery: Potential Applications for Gene and Cell based Therapies in the New Era of Personalized Medicine. Genes Dis 4:43-63 (2017).

Mali P, et al. RNA-guided human genome engineering via Cas9. Science 339:823-826 (2013)

Moniuszko-Szajwaj et al., Highly Polar Triterpenoid Saponins from the Roots of Saponaria officinalis L., Helv Chim. Acta, V99, pp. 347-354, 2016 (doi.org/10.1002/hlca.201500224).

Poljak, R. J., et al. (1994) Structure 2:1121-1123

Puras, G., et al., 2013. Oligochitosan polyplexes as carriers for retinal gene delivery. Eur. J. Pharm.Sci. 48 (1), 323-331.

Rumschöttel, J., et al., 2017. DNA polyplexes with dendritic glycopolymer-entrapped gold nanoparticles. Colloids and Surf. B 154, 74-81.

Sama S et al., Sapofectosid - Ensuring non-toxic and effective DNA and RNA delivery, International Journal of Pharmaceutics, Volume 534, Issues 1-2, 20 December 2017, Pages 195-205 (dx.doi.org/10.1016/j.ijpharm.2017.10.016)

Sama S et al., Structure-Activity Relationship of Transfection-Modulating Saponins - A Pursuit for the Optimal Gene Trafficker, Planta Med. Volume 85, pp. 513-518, 2019 (doi:10.1055/a-0863-4795)

Sama S, et al. Targeted suicide gene transfections reveal promising results in nu/nu mice with aggressive neuroblastoma. J Control Release 275:208-216 (2018)

Tros de Ilarduya, C., Sun, Y., Düzgünes, N., 2010. Gene delivery by lipoplexes and polyplexes. Eur. J. Pharm. Sci. 40 (3), 159-170.

van Setten (1995), Table 2 [Dirk C. van Setten, Gerrit van de Werken, Gijsbert Zomer and Gideon F. A. Kersten, Glycosyl Compositions and Structural Characteristics of the Potential Immuno-adjuvant Active Saponins in the Quillaja saponaria Molina Extract Quil A, RAPID COMMUNICATIONS IN MASS SPECTROMETRY, VOL. 9,660-666 (1995)].

Vasiliu, T., et al., 2017. Optimization of polyplex formation between DNA oligonucleotide and Poly(l-Lysine): experimental study and modeling approach. Int. J. Mol. Sci. 18 (6), 1291.

Wu, G.Y., Wu, C.H., 1987. Receptor-mediated in vitro gene transformation by a soluble DNA carrier system. J. Biol. Chem. 262 (10), 4429-4432.

Zhang, X., et al., 2010. Poly(L-lysine) nanostructured particles for gene delivery and hormone stimulation. Biomaterials 31 (7), 1699-1706.

Ziady, A.-.G., et al., 2003. Transfection of airway epithelium by stable PEGylated poly-Llysine DNA nanoparticles in vivo. Mol. Ther. 8 (6), 936-947.

## Claims

1. A pharmaceutical composition comprising

   a polyplex comprising at least one nucleic acid and a polymeric scaffold; and
   a targeted saponin conjugate comprising a saponin and a first targeting ligand recognised by a first endocytic receptor, wherein the saponin is covalently bound to the first targeting ligand by a linker adapted to cleave and release the saponin from the first targeting ligand under conditions present in an endosome or a lysosome; and

   wherein the saponin is a triterpenoid 12,13-dehydrooleanane-type saponin that in an unreacted state comprises an aldehyde group at position C-23 of the saponin's aglycone core structure.

2. The pharmaceutical composition according to claim 1, wherein the linker is an acid-sensitive linker or is a linker configured to be enzymatically cleaved by an enzyme present in the endosome or the lysosome, preferably wherein the linker is an acid-sensitive linker adapted to cleave and release the saponin from the first targeting ligand under acidic conditions present in the endosome or the lysosome.

3. The pharmaceutical composition according to claim 1 or 2, wherein the polymeric scaffold is or comprises polyamidoamine dendrimer, further referred to as PAMAM, preferably wherein the PAMAM comprises ethylenediamine core and/or wherein the PAMAM is at least generation 3 PAMAM or higher, preferably is generation 5 PAMAM, and/or wherein preferably the PAMAM comprises at least one pegylated group comprising at least one PEG adapter, preferably wherein the PEG adapter comprises 5-15 PEG units, more preferably being 6-12 PEG units; even more preferably wherein the pegylation degree of the PAMAM is comprised between on average 1.5-20 equivalents of PEG adapter per PAMAM core, preferably being on average 2-16 equivalents of PEG adapter per PAMAM core, even more preferably being on average 3-13 equivalents of PEG adapter per PAMAM core, most preferably being on average selected from any one of 3.2, 6.2, and 12.4 PEG adapter equivalents per PAMAM core.

4. The pharmaceutical composition according to claim 1 or 2, wherein the polymeric scaffold is or comprises a polypeptide comprising between 5 to 25 lysines and at least one cysteine, preferably being exactly one cysteine, more preferably wherein the polypeptide comprises exactly one cysteine flanked by a string of 1-7 glycines from each side, preferably 1-5 glycines from each side, more preferably 2-4 glycines, even more preferably wherein the exactly one cysteine is flanked by 3-7 glycines from N-terminal side, preferably being 4 glycines; and by 1-4 glycines from C-terminal side, preferably being 2 glycines, and wherein optionally the polypeptide comprises at least one C-terminal tyrosine, possibly followed by a residue comprising an azide group, preferably being azidolysine, and/or
   wherein the polypeptide is represented by a general formula of, from N-terminus to C-terminus: 2-25 Lys; 3-7 Gly; 1 Cys; 2-4 Gly, and possibly 1 or more Tyr; preferably wherein the polypeptide is represented by an amino acid sequence given by SEQ ID NO. 1: KKKKKKKKKKKKKKKKGGGGCGGY or SEQ ID NO. 2:

KKKKKKKKKKKKKKKKGGGGCGGYK* wherein K* is azidolysine, preferably 6-azido-L-lysine.

**5.** Pharmaceutical composition according to any one of the preceding claims, wherein the saponin comprises an aglycone core structure selected from quillaic acid and gypsogenin, more preferably wherein the aglycone core structure is quillaic acid.

**6.** Pharmaceutical composition according to any one of the preceding claims, wherein the saponin is at least a monodesmosidic saponin comprising at least a first saccharide chain comprising at least three sugar residues in a branched configuration, or is at least a bidesmosidic saponin further comprising a second saccharide chain comprising a glucuronic acid residue, preferably being a terminal glucuronic acid residue;

preferably wherein the first branched saccharide chain comprises a terminal fucose residue and/or a terminal rhamnose residue and preferably comprises at least four sugar residues, and/or wherein the second saccharide chain is Gal-(1→2)-[Xyl-(1→3)]-GlcA;
more preferably wherein the saponin comprises the first saccharide chain at position C-28 of the saponin's aglycone core structure, and/or the second saccharide chain at position C-3 of the saponin's aglycone core structure;
most preferably wherein the first saccharide chain is a carbohydrate substituent at the C-28-OH group of the saponin's aglycone core structure and/or wherein the second saccharide chain is a carbohydrate substituent at the C-3beta-OH group of the saponin's aglycone core structure.

**7.** Pharmaceutical composition according to any one of the preceding claims, wherein the saponin is any one or more of:

a) saponin selected from any one or more of list A:

- *Quillaja saponaria* saponin mixture, or a saponin isolated from *Quillaja saponaria,* for example Quil-A, QS-17-api, QS-17-xyl, QS-21, QS-21A, QS-21B, QS-7-xyl;
- *Saponinum album* saponin mixture, or a saponin isolated from *Saponinum album;*
- *Saponaria officinalis* saponin mixture, or a saponin isolated from *Saponaria officinalis;* and
- Quillaja bark saponin mixture, or a saponin isolated from Quillaja bark, for example Quil-A, QS-17-api, QS-17-xyl, QS-21, QS-21A, QS-21B, QS-7-xyl; or

b) a saponin comprising a gypsogenin aglycone core structure, selected from list B:
SA1641, gypsoside A, NP-017772, NP-017774, NP-017777, NP-017778, NP-018109, NP-017888, NP-017889, NP-018108, SO1658 and Phytolaccagenin; or
c) a saponin comprising a quillaic acid aglycone core structure, selected from list C:
AG1856, AG1, AG2, Agrostemmoside E, GE1741, Gypsophila saponin 1 (Gyp1), NP-017674, NP-017810, NP-003881, NP-017676, NP-017677, NP-017705, NP-017706, NP-017773, NP-017775, SA1657, Saponarioside B, SO1542, SO1584, SO1674, SO1700, SO1730, SO1772, SO1832, SO1861, SO1862, SO1904, QS-7, QS-7 api, QS-17, QS-18, QS-21 A-apio, QS-21 A-xylo, QS-21 B-apio and QS-21 B-xylo; or

preferably, the saponin is any one or more of a saponin selected from list B or C, more preferably, a saponin selected from list C.

**8.** Pharmaceutical composition according to any one of the preceding claims, wherein the saponin is any one or more of AG1856, GE1741, a saponin isolated from *Quillaja saponaria,* Quil-A, QS-17, QS-21, QS-7, SA1641, a saponin isolated from *Saponaria officinalis,* SO1542, SO1584, SO1658, SO1674, SO1700, SO1730, SO1772, Saponarioside B, SO1832, SO1861, SO1862 and SO1904; preferably wherein the saponin is any one or more of QS-21, SO1832, SO1861, SA1641 and GE1741; more preferably wherein the saponin is QS-21, SO1832 or SO1861;
most preferably being SO1861.

**9.** Pharmaceutical composition according to any one of the preceding claims, wherein the saponin is a saponin isolated from *Saponaria officinalis,* preferably wherein the saponin is any one or more of SO1542, SO1584, SO1658, SO1674, SO1700, SO1730, SO1772, Saponarioside B, SO1832, SO1861, SO1862 and SO1904;

more preferably wherein the saponin is any one or more of SO1832, SO1861 and SO1862;
even more preferably wherein the saponin is SO1832 and SO1861;
most preferably being SO1861.

**10.** Pharmaceutical composition according to any one of the preceding claims, wherein the endocytic receptor is selected from any of the following: epidermal growth factor receptor (EGFR) and receptor tyrosine-protein kinase erbB-2 (Her-2).

**11.** Pharmaceutical composition according to any one of the preceding claims, wherein the polyplex further comprises a second targeting ligand recognised by a second endocytic receptor, wherein the first endocytic receptor and the second endocytic receptor are either the same receptor, or are different endocytic receptors present at the same cell.

**12.** Pharmaceutical composition according to any one of the preceding claims, wherein the first targeting ligand, and optionally the second targeting ligand, is an antibody or a binding fragment thereof, preferably is a monoclonal antibody such as Cetuximab or Panitumumab, or is or comprises a peptide or a protein that is recognised by the first endocytic receptor, or optionally the second endocytic receptor.

**13.** Pharmaceutical composition according to any one of the preceding claims, wherein the nucleic acid is selected from a plasmid, a minicircle DNA, cDNA, mRNA, siRNA, oligonucleotide, and any modified equivalent thereof comprising one or more nucleotide analogues, preferably wherein the nucleic acid is a plasmid, more preferably wherein the nucleic acid comprises at least 0.25 kbp, more preferably at least 1 kbp, even more preferably at least 2.5 kbp, most preferably at least 5 kbp.

**14.** Pharmaceutical composition according to any one of the preceding claims, wherein the linker is an acid-sensitive linker that comprises a covalent bond selected from any one or more of a hydrazone bond, an imine bond, an ester bond, a dioxalane bond, and an oxime bond, preferably wherein the acid-sensitive linker comprises a hydrazone bond.

**15.** Pharmaceutical composition according to any one of the preceding claims wherein the aldehyde group at position C-23 of the saponin's aglycone core structure has been engaged in forming the covalent bond with the linker.

**16.** Pharmaceutical composition according to any one of the preceding claims, comprising 0.05 μM - 1 μM of the targeted saponin conjugate, preferably being 0.1 μM - 0.5 μM, more preferably being about 0.3 μM.

**17.** Pharmaceutical composition according to any one of the preceding claims, wherein the nucleic acid is encoding for factor IX or a functional fragment thereof for use in the treatment of haemophilia B; or wherein the first targeting ligand is Cetuximab, and wherein the nucleic acid preferably encodes for a toxin, more preferably dianthin, for use in the treatment of EGFR positive colon cancer or breast cancer.

**18.** A method of preparation of the pharmaceutical composition according to any one of the claims 1-17, the method comprising the steps of:

- mixing an at least one nucleic acid with a polymeric scaffold to obtain a polyplex;
- combining the polyplex with the targeted saponin conjugate, wherein the conjugate comprises:

  - a saponin being a triterpenoid 12,13-dehydrooleanane-type saponin that in an unreacted state comprises an aldehyde group at position C-23 of the saponin's aglycone core structure, and
  - a first targeting ligand recognised by an endocytic receptor, wherein the saponin is covalently bound to the first targeting ligand by a linker adapted to cleave and release the saponin from said ligand under conditions present in an endosome or a lysosome, preferably being an acid-sensitive linker adapted to cleave and release the saponin from the polymeric scaffold under acidic conditions present in an endosome or a lysosome,

and preferably wherein the polymeric scaffold is or comprises

a. PAMAM, preferably being at least generation 3 PAMAM or higher, and/or preferably being a PAMAM of ethylenediamine core, more preferably wherein the PAMAM comprises at least one pegylated group comprising at least one PEG adapter preferably comprising 5-15 PEG units, and/or preferably wherein the pegylation degree of the PAMAM is comprised between on average 1.5-20 equivalents of PEG adapter per PAMAM core;

or wherein the polymeric scaffold is or comprises
b. a polypeptide comprising between 5 to 25 lysines and at least one cysteine, preferably being exactly one

cysteine, more preferably wherein the polypeptide comprises exactly one cysteine, preferably wherein the exactly one cysteine is flanked by a string of 1-7 glycines from each side, preferably 1-5 glycines from each side, more preferably 2-4 glycines, most preferably is flanked by 3-7 glycines from N-terminal side, preferably being 4 glycines; and by 1-4 glycines from C-terminal side, preferably being 2 glycines, and wherein optionally the polypeptide comprises a C-terminal tyrosine, possibly followed by a residue comprising an azide group, preferably being azidolysine, more preferably 6-azido-L-lysine, most preferably wherein the polypeptide is represented by a general formula of: 2-25 Lys ; 3-7 Gly; 1 Cys; 2-4 Gly, and possibly 1 Tyr; preferably wherein the polypeptide is represented by an amino acid sequence given by SEQ ID NO. 1: KKKKKKKKKKKKKKKKGGGGCGGY or SEQ ID NO. 2: KKKKKKKKKKKKKKKKGGGGCGGYK* wherein K* is azidolysine, preferably 6-azido-L-lysine.

**19.** Use of a polymeric scaffold comprising or being either:

a. PAMAM, preferably being at least generation 3 PAMAM or higher, and/or preferably being a PAMAM of ethylenediamine core, more preferably wherein the PAMAM comprises at least one pegylated group comprising at least one PEG adapter preferably comprising 5-15 PEG units, and/or preferably wherein the pegylation degree of the PAMAM is comprised between on average 1.5-20 equivalents of PEG adapter per PAMAM core;
or
b. a polypeptide comprising between 5 to 25 lysines and at least one cysteine, preferably being exactly one cysteine, more preferably wherein the polypeptide comprises exactly one cysteine, preferably wherein the exactly one cysteine is flanked by a string of 1-7 glycines from each side, preferably 1-5 glycines from each side, more preferably 2-4 glycines, most preferably is flanked by 3-7 glycines from N-terminal side, preferably being 4 glycines; and by 1-4 glycines from C-terminal side, preferably being 2 glycines, and wherein optionally the polypeptide comprises a C-terminal tyrosine, most preferably wherein the polypeptide is represented by a general formula of: 2-25 Lys ; 3-7 Gly; 1 Cys; 2-4 Gly, and possibly 1 Tyr, possibly followed by a residue comprising azide group, preferably being azidolysine, more preferably 6-azido-L-lysine; preferably wherein the polypeptide is represented by an amino acid sequence given by SEQ ID NO. 1: KKKKKKKKKKKKKKKKGGGGCGGY or SEQ ID NO. 2: KKKKKKKKKKKKKKKKGGGGCGGYK* wherein K* is azidolysine, preferably 6-azido-L-lysine, for preparation of a polyplex, preferably for preparation of a polyplex to be used with a targeted saponin conjugate wherein the saponin is a triterpenoid 12,13-dehydrooleanane-type saponin that in an unreacted state comprises an aldehyde group at position C-23 of the saponin's aglycone core structure, for transfection of a cell, preferably for preparation of a pharmaceutical composition according to any one of claims 1-17.

**20.** Kit of parts for delivering a nucleic acid into a cell *in vitro* comprising

(a) at least a first container comprising the polymeric scaffold as defined in claim 19; and
(b) a targeted saponin conjugate, possibly provided in the first container or in a second container, the targeted saponin conjugate comprising a saponin and a first targeting ligand recognised by an endocytic receptor, wherein the saponin is covalently bound to the first targeting ligand by an acid-sensitive linker adapted to cleave and release the saponin from the first targeting ligand under acidic conditions; and wherein the saponin is a triterpenoid 12,13-dehydrooleanane-type saponin that in an unreacted state comprises an aldehyde group at position C-23 of the saponin's aglycone core structure,
and, optionally
(c) instructions for use, preferably containing instructions for combining of the polymeric scaffold with a nucleic acid for preparation of a polyplex for delivering with the targeted saponin conjugate into a cell.

# FIG. 1

## A


SO1861
H2N
N
H
TFA
SO1861
N
N
H
SO1861
EMCH
SO1861-EMCH


## B


=
SO1861
N
N
H
SO1861-EMCH

# FIG. 2

## A

TCEP
IgG
IgG-SH
Cys
SH
HS
NH
H
N
O
=
Interchain cysteine residue

## B

SO1861
SO1861-EMCH
IgG-SH
IgG-SO1861
Cys
SH
HS
S
N
O

**FIG. 2**
**C**

ligand
Lys
$NH_2$
SO1861
SO1861-hydrazone-NHS
ligand
Lys
SO1861
ligand - SO1861
SO1861
SO1861-hydrazone-NHS
Lys
$NH_2$
Peripheral lysine residue

**FIG. 3**

**A**

NHS-PEG12-N3
= Click adapter
Polyamidoamine
(PAMAM)
PAMAM with click
adapter


**B**

NH2
n
O
N
O
O
12
N3
NHS-PEG12-N3
Click adapter
NH2
n-m
H
N
O
12
N3
m
PAMAM
PAMAM with click adapter

FIG. 4

Equipped PAMAM
with click adapter
Effector gene
Polyplex
Targeting ligand
with click adapter
Targeted Polyplex

**FIG. 5**

Lys
Gly
Cys
Tyr
PEG8
Lys-N3
4
4

FIG. 6

A
Residual solvent
C,c
A,a,D
760
d
252
504
B,b
B
x,w,v
241
y
C,c
u,C,D
A,a,D
750
d
252
z
514
B,b
5.0
4.5
4.0
3.5
(ppm)
3.0
2.5

C
y, x, w, v
F
z
a
b, d
u
e
DE
c, t
180
160
140
120
100
80
60
40
20
(ppm)

FIG. 6
D

A
B
C
D
-N3
-O-C2H4-O-
4000 3500 3000 2500 2000 1500 1000 500
Wavenumber (cm-1)

E

Volume (%)
25
20
15
10
5
0
1
10
100
1000
10000
Diameter (nm)
PAMAM G5-(PEG-N3)3.2
PAMAM G5-(PEG-N3)6.2
PAMAM G5-(PEG-N3)12.4

FIG. 6

F

N/P ratio
0.5
1
2
4
8
16
N/P ratio
0.5
1
2
4
8
16

G

a
M
0.5
1
2
4
8
16
b
M
0.5
1
2
4
8
16
c
M
0.5
1
2
4
8
16

**FIG. 7**

Proportion of free DNA [%]
0
25
50
75
100
0.0
2.5
5.0
7.5
10.0
N/P ratio
K16
K16C
K16CPEG

# FIG. 8

## A

A431 (EGFR high)
Relative cell viability (Untr. = 100%)
125
100
75
50
25
0
1
10
100
1000
npSaporin (pM)
PAMAM G5 (PEG-N3)6.2
PAMAM G5 (PEG-N3)6.2
+ 4uM SO1861-EMCH
PAMAM G5 (PEG-N3)6.2
+ 73 nM Cetuximab-SO1861
(corresponding to 0.3 uM SO1861)

## B

A2058 (EGFR negative)
Relative cell viability (Untr. = 100%)
125
100
75
50
25
0
1
10
100
1000
npSaporin (pM)
PAMAM G5 (PEG-N3)6.2
PAMAM G5 (PEG-N3)6.2
+ 4uM SO1861-EMCH
PAMAM G5 (PEG-N3)6.2
+ 73 nM Cetuximab-SO1861
(corresponding to 0.3 uM SO1861)

# FIG. 8

C

A431 (EGFR high)
Relative cell viability (Untr. = 100%)
125
100
75
50
25
0
1
10
100
1000
pEGFP (pM)
PAMAM G5 (PEG-N3)6.2
PAMAM G5 (PEG-N3)6.2
+ 4uM SO1861-EMCH
PAMAM G5 (PEG-N3)6.2
+ 73 nM Cetuximab-SO1861
(corresponding to 0.3 uM SO1861)

D

A2058 (EGFR negative)
Relative cell viability (Untr. = 100%)
125
100
75
50
25
0
1
10
100
1000
pEGFP (pM)
PAMAM G5 (PEG-N3)6.2
PAMAM G5 (PEG-N3)6.2
+ 4uM SO1861-EMCH
PAMAM G5 (PEG-N3)6.2
+ 73 nM Cetuximab-SO1861
(corresponding to 0.3 uM SO1861)

**FIG. 9**

**A**

A431 (EGFR high)

Relative cell viability (Untr. = 100%)
120
95
70
45
20
-5
1
10
100
1000
npSaporin (pM)
PAMAM G5
PAMAM G5 (PEG)6
PAMAM G5 +73 nM Cetuximab SO1861 (300 nM SO1861)
PAMAM G5 (PEG)6 +73 nM Cetuximab-SO1861 (300 nM SO1861)

**B**

A2058 (EGFR negative)
Relative cell viability (Untr. = 100%)
120
95
70
45
20
-5
1
10
100
1000
npSaporin (pM)
PAMAM G5
PAMAM G5 (PEG)6
PAMAM G5 +73 nM Cetuximab SO1861 (300 nM SO1861)
PAMAM G5 (PEG)6 +73 nM Cetuximab-SO1861 (300 nM SO1861)

A431 (EGFR high)
Relative cell viability (Untr. = 100%)
120
95
70
45
20
-5
1
10
100
1000
pEGFP (pM)
PAMAM G5
PAMAM G5 (PEG)6
PAMAM G5 +73 nM Cetuximab SO1861 (300 nM SO1861)
PAMAM G5 (PEG)6 +73 nM Cetuximab-SO1861 (300 nM SO1861)

A2058 (EGFR negative)
Relative cell viability (Untr. = 100%)
120
95
70
45
20
-5
1
10
100
1000
pEGFP (pM)
PAMAM G5
PAMAM G5 (PEG)6
PAMAM G5 +73 nM Cetuximab SO1861 (300 nM SO1861)
PAMAM G5 (PEG)6 +73 nM Cetuximab-SO1861 (300 nM SO1861)

# FIG. 10

A431 (EGFR+), npSaporin (72 hr)

Relative cell viability (Untr. = 100%)
100
75
50
25
0
1
10
100
1000
npSaporin (pM)
K16C
K16C + 4000 nM SO1861-EMCH
K16C + 73 nM Cetuximab-SO1861

FIG. 11
A

Incubation of the Polyplex in blood serum
Sample purification Polyplex isolation
Replacement
–
Heparin addition
PCR
Plasmid
PAMAM
Polyplex
Heparin

B

1
2
3
4
5
6
5000
2686
1500
711
500

FIG. 12

A

Blood cleaning
Blood
Cleaned RBCs
Colloidal nanomaterials
Nanomaterials incubation with RBCs
RBCs + nanomaterials
Possible results
Hemolysis
No hemolysis

B

3.0%
2.5%
2.0%
1.5%
1.0%
0.5%
0.0%
Hemolysis [%]
2.5
5
10
20
40
80
Concentration [µg/mL]
K16C
PAMAM
PAMAM polyplexes
K16C polyplexes

# FIG. 12
# C

1.
50 µm
PAMAM
80 µg/mL
2.
50 µm
K16C
80 µg/mL
3.
50 µm
PAMAM polyplexes (NP 8) 80 µg/mL
4.
50 µm
K16C polyplexes (NP 8) 80 µg/mL

# EUROPEAN SEARCH REPORT

Application Number
EP 22 19 3399

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | QI RONG ET AL: "PEG-conjugated PAMAM Dendrimers Mediate Efficient Intramuscular Gene Expression", THE AAPS JOURNAL, SPRINGER US, BOSTON, vol. 11, no. 3, 29 May 2009 (2009-05-29), pages 395-405, XP035719034, DOI: 10.1208/S12248-009-9116-1 [retrieved on 2009-05-29] | 19 | INV. A61K47/59 A61K47/64 A61K47/68 A61P7/00 A61P35/00 |
| Y | * the whole document * | 1-20 | |
| X | KWOH D Y ET AL: "Stabilization of poly-l-lysine/DNA polyplexes for in vivo gene delivery to the liver", BIOCHIMICA ET BIOPHYSICA ACTA . GENE STRUCTURE AND EXPRESSION, ELSEVIER, AMSTERDAM, NL, vol. 1444, no. 2, 16 February 1999 (1999-02-16), pages 171-190, XP004275308, ISSN: 0167-4781, DOI: 10.1016/S0167-4781(98)00274-7 | 19 | |
| Y | * the whole document and in particular section "3.4 Size and shape of polyplexes" * | 1-20 | TECHNICAL FIELDS SEARCHED (IPC) A61K |
| Y | WO 2020/126064 A1 (SAPREME TECH BV [NL]; UNIV BERLIN CHARITE [DE]) 25 June 2020 (2020-06-25) * page 10, line 26 - page 12, line 10 * * claims 1-13, 42 * * figure 50 * | 1-20 | |

-/--

The present search report has been drawn up for all claims

1

| Place of search | Date of completion of the search | Examiner |
|---|---|---|
| The Hague | 9 February 2023 | Birikaki, Lemonia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

# EUROPEAN SEARCH REPORT

Application Number
EP 22 19 3399

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | SAMA SIMKO ET AL: "Sapofectosid - Ensuring non-toxic and effective DNA and RNA delivery", INTERNATIONAL JOURNAL OF PHARMACEUTICS, vol. 534, no. 1, 17 October 2017 (2017-10-17), pages 195-205, XP085280601, ISSN: 0378-5173, DOI: 10.1016/J.IJPHARM.2017.10.016<br>* the whole document and in particular the abstract and Figure 1 *<br>----- | 1-20 | |
| | | | TECHNICAL FIELDS SEARCHED (IPC) |

The present search report has been drawn up for all claims

| Place of search | Date of completion of the search | Examiner |
|---|---|---|
| The Hague | 9 February 2023 | Birikaki, Lemonia |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

1

page 2 of 2

# ANNEX TO THE EUROPEAN SEARCH REPORT ON EUROPEAN PATENT APPLICATION NO.

EP 22 19 3399

This annex lists the patent family members relating to the patent documents cited in the above-mentioned European search report. The members are as contained in the European Patent Office EDP file on
The European Patent Office is in no way liable for these particulars which are merely given for the purpose of information.

09-02-2023

| Patent document cited in search report | Publication date | Patent family member(s) | Publication date |
|---|---|---|---|
| WO 2020126064 A1 | 25-06-2020 | AU 2019400630 A1 | 12-08-2021 |
| | | AU 2019407234 A1 | 19-08-2021 |
| | | AU 2019408811 A1 | 12-08-2021 |
| | | AU 2019411276 A1 | 12-08-2021 |
| | | AU 2019411278 A1 | 12-08-2021 |
| | | AU 2019411289 A1 | 12-08-2021 |
| | | CA 3123978 A1 | 25-06-2020 |
| | | CA 3124064 A1 | 25-06-2020 |
| | | CA 3124065 A1 | 25-06-2020 |
| | | CA 3124129 A1 | 25-06-2020 |
| | | CA 3124151 A1 | 25-06-2020 |
| | | CA 3124406 A1 | 25-06-2020 |
| | | CN 113453722 A | 28-09-2021 |
| | | CN 113474009 A | 01-10-2021 |
| | | CN 113474010 A | 01-10-2021 |
| | | CN 113498350 A | 12-10-2021 |
| | | CN 113507941 A | 15-10-2021 |
| | | CN 113747923 A | 03-12-2021 |
| | | DK 3773737 T3 | 04-10-2021 |
| | | EP 3773737 A1 | 17-02-2021 |
| | | EP 3808379 A1 | 21-04-2021 |
| | | EP 3856254 A2 | 04-08-2021 |
| | | EP 3897735 A1 | 27-10-2021 |
| | | EP 3897738 A1 | 27-10-2021 |
| | | EP 3897739 A1 | 27-10-2021 |
| | | EP 3897741 A1 | 27-10-2021 |
| | | EP 3897742 A2 | 27-10-2021 |
| | | EP 3897743 A1 | 27-10-2021 |
| | | EP 3915587 A1 | 01-12-2021 |
| | | EP 4015003 A1 | 22-06-2022 |
| | | ES 2899612 T3 | 14-03-2022 |
| | | IL 284265 A | 31-08-2021 |
| | | IL 284272 A | 31-08-2021 |
| | | IL 284273 A | 31-08-2021 |
| | | IL 284274 A | 31-08-2021 |
| | | IL 284276 A | 31-08-2021 |
| | | IL 284278 A | 31-08-2021 |
| | | IL 284279 A | 31-08-2021 |
| | | IL 284280 A | 31-08-2021 |
| | | JP 2022515250 A | 17-02-2022 |
| | | JP 2022515251 A | 17-02-2022 |
| | | JP 2022515797 A | 22-02-2022 |
| | | JP 2022516043 A | 24-02-2022 |
| | | JP 2022516044 A | 24-02-2022 |
| | | JP 2022516045 A | 24-02-2022 |
| | | KR 20210107073 A | 31-08-2021 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO.

EP 22 19 3399

This annex lists the patent family members relating to the patent documents cited in the above-mentioned European search report. The members are as contained in the European Patent Office EDP file on
The European Patent Office is in no way liable for these particulars which are merely given for the purpose of information.

09-02-2023

| Patent document cited in search report | Publication date | Patent family member(s) | Publication date |
|---|---|---|---|
| | | KR 20210110323 A | 07-09-2021 |
| | | KR 20210117274 A | 28-09-2021 |
| | | KR 20210117275 A | 28-09-2021 |
| | | KR 20210117276 A | 28-09-2021 |
| | | KR 20210119976 A | 06-10-2021 |
| | | KR 20210119978 A | 06-10-2021 |
| | | KR 20210119979 A | 06-10-2021 |
| | | SG 11202106572Q A | 29-07-2021 |
| | | SG 11202106574U A | 29-07-2021 |
| | | SG 11202106575Y A | 29-07-2021 |
| | | SG 11202106603U A | 29-07-2021 |
| | | SG 11202106606R A | 29-07-2021 |
| | | SG 11202106612Q A | 29-07-2021 |
| | | SG 11202106664P A | 29-07-2021 |
| | | SG 11202106672S A | 29-07-2021 |
| | | US 2022023433 A1 | 27-01-2022 |
| | | US 2022054643 A1 | 24-02-2022 |
| | | US 2022072149 A1 | 10-03-2022 |
| | | US 2022111066 A1 | 14-04-2022 |
| | | US 2022218837 A1 | 14-07-2022 |
| | | US 2022313834 A1 | 06-10-2022 |
| | | WO 2020126064 A1 | 25-06-2020 |
| | | WO 2020126600 A1 | 25-06-2020 |
| | | WO 2020126604 A1 | 25-06-2020 |
| | | WO 2020126609 A2 | 25-06-2020 |
| | | WO 2020126610 A1 | 25-06-2020 |
| | | WO 2020126620 A2 | 25-06-2020 |
| | | WO 2020126626 A1 | 25-06-2020 |
| | | WO 2020126627 A1 | 25-06-2020 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5693780 A
- EP 3773737 A


### Non-patent literature cited in the description


- **HOLLIGER, P et al.** *Proc. Natl. Acad. Sci. USA,* 1993, vol. 90, 6444-6448
- **POLJAK, R. J. et al.** *Structure,* 1994, vol. 2, 1121-1123
- **KIPRIYANOV, S. M. et al.** *Human Antibodies and Hybridomas,* 1995, vol. 6, 93-101
- **KIPRIYANOV, S. M. et al.** *Mol. Immunol.,* 1994, vol. 31, 1047-1058
- **DIRK C. VAN SETTEN ; GERRIT VAN DE WERKEN ; GIJSBERT ZOMER ; GIDEON F. A. KERSTEN.** Glycosyl Compositions and Structural Characteristics of the Potential Immuno-adjuvant Active Saponins in the Quillaja saponaria Molina Extract Quil A. *RAPID COMMUNICATIONS IN MASS SPECTROMETRY,* 1995, vol. 9, 660-666
- **JULIANE DEISE FLECK ; ANDRESA HEEMANN BETTI ; FRANCINI PEREIRA DA SILVA ; EDUARDO ARTUR TROIAN ; CRISTINA OLIVARO ; FERNANDO FERREIRA ; SIMONE GASPARIN VERZA.** Saponins from Quillaja saponaria and Quillaja brasiliensis: Particular Chemical Characteristics and Biological Activities. *Molecules,* 2019, vol. 24, 171
- **JIA et al.** Major Triterpenoid Saponins from Saponaria officinalis. *J. Nat. Prod.,* 19 September 1998, vol. 61 (11), 1368-1373, https://doi.org/10.1021/np980167u
- **J. CLOCHARD et al.** A new acetylated triterpene saponin from Agrostemma githago L. modulates gene delivery efficiently and shows a high cellular tolerance. *International Journal of Pharmaceutics,* 15 November 2020, vol. 589, 119822
- **MONIUSZKO-SZAJWAJ et al.** Highly Polar Triterpenoid Saponins from the Roots of Saponaria officinalis L. *Helv. Chim. Acta,* 2016, vol. 99, 347-354
- **SAMA et al.** Structure-Activity Relationship of Transfection-Modulating Saponins - A Pursuit for the Optimal Gene Trafficker. *Planta Med.,* 2019, vol. 85, 513-518
- **FUCHS et al.** Glycosylated Triterpenoids as Endosomal Escape Enhancers in Targeted Tumor Therapies. *Biomedicine,* 2017, vol. 5 (14
- **SAMA et al.** Sapofectosid - Ensuring non-toxic and effective DNA and RNA delivery. *International Journal of Pharmaceutics,* 20 December 2017, vol. 534 (1-2), 195-205
- *Tetrahedron Letters,* 1963, vol. 8, 477-482
- **AGARWAL, S et al.** PDMAEMA based gene delivery materials. *Mater. Today,* 2012, vol. 15 (9), 388-393
- **BERKHOUT B ; LIU YP.** Towards improved shRNA and miRNA reagents as inhibitors of HIV-1 replication. *Future Microbiol,* 2014, vol. 9, 561-571
- **BÖTTGER S.** Untersuchungen zur synergistischen Zytotoxizität zwischen Saponinen und Ribosomen inaktivierenden Proteinen Typ I. *PhD thesis,* 2013
- **CLIFFORD, A. et al.** Biomimetic modification of poly-L-lysine and electrodeposition of nanocomposite coatings for orthopaedic applications. *Colloids Surf. B,* 2019, vol. 176, 115-121
- **CLOCHARD J et al.** A new acetylated triterpene saponin from Agrostemma githago L. modulates gene delivery efficiently and shows a high cellular tolerance. *International Journal of Pharmaceutics,* 15 November 2020, vol. 589, 119822
- **DÉMOULINS, T. et al.** Polyethylenimine-based polyplex delivery of self-replicating RNA vaccines. *Nanomedicine,* 2016, vol. 12 (3), 711-722
- **DUFES C et al.** *Adv Drug Deliv Rev,* 2005, vol. 57, 2177
- **FUCHS H et al.** Glycosylated Triterpenoids as Endosomal Escape Enhancers in Targeted Tumor Therapies. *Biomedicines,* 2017, 5
- **GEORGE LA et al.** Hemophilia B Gene Therapy with a High-Specific-Activity Factor IX Variant. *N Engl J Med,* 2017, vol. 377, 2215-2227
- **GILLERON J et al.** Image based analysis of lipid nanoparticle-mediated siRNA delivery, intracellular trafficking and endosomal escape. *Nat Biotechnol,* 2013, vol. 31, 638-646
- **HE, Y. et al.** Polyethyleneimine/DNA polyplexes with reduction-sensitive hyaluronic acid derivatives shielding for targeted gene delivery. *Biomaterials,* 2013, vol. 34 (4), 1235-1245

- **HESS, K.L. et al.** Polyplexes assembled from self-peptides and regulatory nucleic acids blunt toll-like receptor signaling to combat autoimmunity. *Biomaterials,* 2017, vol. 118, 51-62
- **JIA et al.** Major Triterpenoid Saponins from Saponaria officinalis. *J. Nat. Prod.,* 1998, vol. 61 (11), 1368-1373
- **KABANOV, A.V. ; KABANOV, V.A.** DNA complexes with polycations for the delivery of genetic material into cells. *Bioconjug Chem,* 1995, vol. 6 (1), 7-20
- **KABAT, E.A. ; WU, T.T. ; PERRY, H.M. ; GOTTESMAN, K.S. ; FOELLER, C.** Sequences of Proteins of Immunological Interest. NIH publication, 1991
- **KOCHETKOV N.K. ; KHORLIN A.J. ; OVODOV J.S.** *The structure of gypsoside - triterpenic saponin from gypsophila pacifica kom.,* 1963, vol. 4 (8), 477-482
- **KODAMA, Y. et al.** Biodegradable nanoparticles composed of dendrigraft poly-Llysine for gene delivery. *Eur. J. Pharm. Biopharmaceutics,* 2014, vol. 87 (3), 472-479
- **KOPING-HOGGARD, M. et al.** Improved chitosan-mediated gene delivery based on easily dissociated chitosan polyplexes of highly defined chitosan oligomers. *Gene Ther.,* 2004, vol. 11 (19), 1441-1452
- **KOZIELSKI, K.L. ; RUI, Y. ; GREEN, J.J.** Non-viral nucleic acid containing nanoparticles as cancer therapeutics. *Expert Opin Drug Deliv,* 2016, vol. 13 (10), 1475-1487
- **KWOH, D.Y. et al.** Stabilization of poly-L-lysine/DNA polyplexes for in vivo gene delivery to the liver. *Biochim. Biophys. Acta,* 1999, vol. 1444 (2), 171-190
- **LEE CS et al.** Adenovirus-Mediated Gene Delivery: Potential Applications for Gene and Cell based Therapies in the New Era of Personalized Medicine. *Genes Dis,* 2017, vol. 4, 43-63
- **MALI P et al.** RNA-guided human genome engineering via Cas9. *Science,* 2013, vol. 339, 823-826
- **MONIUSZKO-SZAJWAJ et al.** Highly Polar Triterpenoid Saponins from the Roots of Saponaria officinalis L. *Helv Chim. Acta,* 2016, vol. 99, 347-354
- **PURAS, G. et al.** Oligochitosan polyplexes as carriers for retinal gene delivery. *Eur. J. Pharm.Sci.,* 2013, vol. 48 (1), 323-331
- **RUMSCHÖTTEL, J. et al.** DNA polyplexes with dendritic glycopolymer-entrapped gold nanoparticles. *Colloids and Surf. B,* 2017, vol. 154, 74-81
- **SAMA S et al.** Sapofectosid - Ensuring non-toxic and effective DNA and RNA delivery. *International Journal of Pharmaceutics,* 20 December 2017, vol. 534 (1-2), 195-205
- **SAMA S et al.** Structure-Activity Relationship of Transfection-Modulating Saponins - A Pursuit for the Optimal Gene Trafficker. *Planta Med.,* 2019, vol. 85, 513-518
- **SAMA S et al.** Targeted suicide gene transfections reveal promising results in nu/nu mice with aggressive neuroblastoma. *J Control Release,* 2018, vol. 275, 208-216
- **TROS DE ILARDUYA, C. ; SUN, Y. ; DÜZGÜNES, N.** Gene delivery by lipoplexes and polyplexes. *Eur. J. Pharm. Sci.,* 2010, vol. 40 (3), 159-170
- **VASILIU, T. et al.** Optimization of polyplex formation between DNA oligonucleotide and Poly(l-Lysine): experimental study and modeling approach. *Int. J. Mol. Sci.,* 2017, vol. 18 (6), 1291
- **WU, G.Y. ; WU, C.H.** Receptor-mediated in vitro gene transformation by a soluble DNA carrier system. *J. Biol. Chem.,* 1987, vol. 262 (10), 4429-4432
- **ZHANG, X. et al.** Poly(L-lysine) nanostructured particles for gene delivery and hormone stimulation. *Biomaterials,* 2010, vol. 31 (7), 1699-1706
- **ZIADY, A.-.G. et al.** Transfection of airway epithelium by stable PEGylated poly-Llysine DNA nanoparticles in vivo. *Mol. Ther.,* 2003, vol. 8 (6), 936-947